(19) Europäisches Patentamt
European Patent Office
Office européen des brevets

(11) Publication number: 0 129 408 A2

(12) # EUROPEAN PATENT APPLICATION

(21) Application number: 84304006.4

(22) Date of filing: 14.06.84

(51) Int. Cl.³: C 07 D 275/02
A 01 N 47/36

(30) Priority: 20.06.83 US 505625

(43) Date of publication of application: 27.12.84 Bulletin 84/52

(84) Designated Contracting States: BE CH DE FR GB IT LI LU NL SE

(71) Applicant: ELI LILLY AND COMPANY
307, East McCarty Street
Indianapolis Indiana 46285(US)

(72) Inventor: Burow, Kenneth Wayne, Jr.
6467 Johnson Road
Indianapolis Indiana 46220(US)

(74) Representative: Tapping, Kenneth George et al,
Lilly Industries Limited Patent Department Erl Wood Manor
Windlesham Surrey, GU20 6PH(GB)

(54) (3-alkyl-5-isothiazolyl)ureas.

(57) Novel (3-alkyl-5-isothiazolyl) ureas which are useful as aquatic algicides, terrestrial herbicides, and aquatic herbicides, are described herein. The products are prepared from the corresponding 5-amino-3-substituted-isothiazoles with a ureido forming reagent. Further provided are methods of use for these products and formulations comprising these products with an acceptable carrier.

EP 0 129 408 A2

Croydon Printing Company Ltd

# (3-ALKYL-5-ISOTHIAZOLYL)UREAS

This invention concerns a class of novel N'-(3-alkyl-5-isothiazolyl)-N-substituted-ureas, which are useful as aquatic algicides, terrestrial herbicides, and aquatic herbicides.

The use of herbicides, both terrestrial and aquatic, and aquatic algicides to control selectively the growth of unwanted vegetation and algae is well established. However, a number of problems are associated with the continued use of chemical herbicides and algicides, including environmental pollution, crop tolerance, weed resistance, as well as economic considerations. Accrodingly, the search continues for new herbicides and algicides which are economical and which are selectively toxic to unwanted vegetation.

A wide variety of isothiazolyl-ureas are known in the art. For example, U.S. Patent 3,563,985 describes a process for making certain (3-substituted-5-isothiazolyl)ureas, including in the 3-position hydrogen or methyl. U.S. Patents 4,196,126 and 4,227,916 disclose N'-(3-methyl-5-isothiazolyl)-N-alkoxy-N-methylureas. U.S. Patent 3,454,591 teaches (3-methyl-5-isothiazolyl)ureas.

This invention concerns isothiazolylureas of the formula

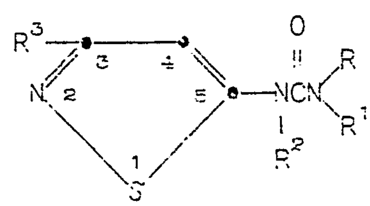

IX

wherein

R and $R^1$ are hydrogen, $C_1$-$C_3$ alkyl, n-butyl, sec-butyl, $C_2$-$C_6$ alkenyl, or $C_1$-$C_2$ alkoxy;

provided that only one of R or $R^1$ is hydrogen;

provided that only one of R or $R^1$ is $C_1$-$C_2$ alkoxy;

provided that one of R or $R^1$ is less than three carbon atoms; or

R and $R^1$ together with the nitrogen atom form a pyrrolidyl, piperidyl, morpholinyl, or piperazinyl ring;

$R^2$ is hydrogen or $C_1$-$C_2$ alkyl; and

$R^3$ is $C_2$-$C_{10}$ alkyl, benzyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, or ($C_3$-$C_6$ cycloalkyl)$C_1$-$C_4$ alkyl;

or an agriculturally- or aquatically-acceptable acid addition salt thereof.

The compounds of formula IX are prepared by reacting a 5-aminoisothiazole of the formula

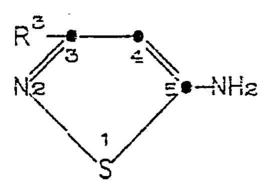

V

wherein $R^3$ is defined as before with a ureido forming reagent, to provide the compounds of formula IX wherein $R^2$ is hydrogen;

followed by alkylation with

$R^{2'}Y$

wherein $R^{2'}Y$ is $C_1$-$C_2$ alkyl and Y is bromo, chloro, fluoro, or iodo, to provide the compounds of formula IX wherein $R^2$ is $C_1$-$C_2$ alkyl; and

optionally followed by salification to form the corresponding acid addition salt.

Examples of suitable ureido forming reagents are

a) RNCO,

b) $RR^1N\overset{\overset{\displaystyle O}{\|}}{C}V$,

c) $COV_2$ and $HNRR^1$,

d) V-phenylformate and $HNRR^1$, and

e) V-phenylformate and hydroxylamine,

followed by alkylating with dimethyl sulfate, wherein R and $R^1$ are defined as before, and V is chloro or bromo.

The (3-alkyl-5-isothiazolyl)-ureas of formula IX, VII or X, or an agriculturally- or aquatically-acceptable acid addition salt thereof, will normally be sold as a herbicidal or algicidal formulation together with one or more agriculturally- or aquatically-acceptable carriers or diluents therefor. Formulations comprising one or more other compatible herbicides or algicides are also contemplated.

Additionally provided by the invention is a method for controlling the growth of unwanted terrestrial vegetation which comprises contacting the vegeta-

tation to be controlled or the soil in which the vegetation is growing with a herbicidally-effective amount of a (3-alkyl-5-isothiazolyl)urea of formula IX.

Also provided by the invention is a method of controlling the growth of unwanted aquatic vegetation which comprises contacting the vegetation to be controlled or the water in which the vegetation is growing with an aquatically herbicidally-effective amount of a compound of formula X, defined hereinafter.

The invention also provides a method of controlling the growth of aquatic algae which comprises contacting the algae to be controlled or the water in which the algae is growing with an algicidally-effective amount of a compound of formula VII, defined hereinafter.

In addition to the isothiazolylurea compounds of formula IX, there is also provided a preferred group of compounds of the formula IX wherein:

$R^3$ is $C_3$-$C_{10}$ alkyl.

Other preferred compounds of formula IX are those wherein:

R and $R^1$ are $C_1$-$C_3$ alkyl or $C_1$-$C_2$ alkoxy, provided that only one of R or $R^1$ is $C_1$-$C_2$ alkoxy and that one of R and $R^1$ is less than three carbon atoms;

$R^2$ is hydrogen; and

$R^3$ is $C_3$-$C_6$ alkyl

Even more preferred compounds of formula IX are those wherein:

R is methyl; $R^1$ is methoxy or methyl; and

$R^3$ is $C_3$-$C_4$ alkyl.

Most preferred compounds of formula IX include:

N'-[3-(1,1-dimethylethyl)-5-isothiazolyl]-N-methoxy-N-methylurea;

N'-[3-(1,1-dimethylethyl)-5-isothiazolyl]-N,N-dimethylurea;

N'-[3-(1-methylethyl)-5-isothiazolyl]-N,N-dimethylurea;

N'-[3-(1-methylethyl)-5-isothiazolyl]-N-methoxy-N-methylurea; and

N'-[3-(2-methylpropyl)-5-isothiazolyl]-N-methoxy-N-methylurea.

Other compounds, which are preferred for use in the various methods, include:

N'-(3-cyclohexyl-5-isothiazolyl)-N-methoxy-N-methylurea;

N'-(3-cyclohexyl-5-isothiazolyl)-N,N-dimethylurea;

N'-[3-(1-methylpropyl)-5-isothiazolyl]-N-methyl-N-(1-methylpropyl)urea;

N'-[3-(1-ethylpropyl)-5-isothiazolyl]-N-ethyl-N-methylurea;

N'-[3-(2-methylpropyl)-5-isothiazolyl]-N,N-dimethylurea;

N'-(3-butyl-5-isothiazolyl)-N,N-dimethylurea;

N'-[3-(2-methylpropyl)-5-isothiazolyl]-N-methyl-N-(1-methylethyl)urea;

N'-[3-(1-methylethyl)-5-isothiazolyl]-N-methyl-N-(1-methylethyl)urea;

N'-[3-(1-methylethyl)-5-isothiazolyl]-N-ethyl-N-methylurea;

N'-(3-ethyl-5-isothiazolyl)-N-methoxy-N-methylurea;

N'-[3-(1-methylpropyl)-5-isothiazolyl]-N-methoxy-N-methylurea;

N'-[3-(1,1-dimethylethyl)-5-isothiazolyl]-N-methoxy-N-methylurea monohydrochloride;

N'-[3-(1-methylpropyl)-5-isothiazolyl]-N-methyl-N-(1-methylethyl)urea;

N'-[3-(1-ethyl-1-methylpropyl)-5-isothiazolyl]-N-ethyl-N-methylurea; and

N'-(3-cyclohexylmethyl-5-isothiazolyl)-N,N-dimethylurea.

The following defines the various terms used in this application.

$C_1$-$C_{10}$ alkyl refers to straight and branched aliphatic radicals of one to ten carbon atoms including methyl, propyl, isopropyl (1-methylethyl), butyl, ethyl, isobutyl (2-methylpropyl), sec-butyl (1-methylpropyl), tert-butyl (1,1-dimethylethyl), pentyl, isopentyl (3-methylbutyl), sec-pentyl (1-methylbutyl), 1,1-dimethylpropyl, 1,2-dimethylpropyl, neopentyl (2,2-dimethylpropyl), hexyl, isohexyl (4-methylpentyl), sec-hexyl (1-methylpentyl), 2-methylpentyl, 3-methylpentyl, 1,1-dimethylbutyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, heptyl, isoheptyl (5-methylhexyl), sec-heptyl (1-methylhexyl), 1-ethylpentyl, 2,2-dimethylpentyl, 3,3-dimethylpentyl, 4,4-dimethylpentyl, 1,2-dimethylpentyl, 1,4-dimethylpentyl, 1,2,3-trimethylbutyl, 1,1,2-trimethylbutyl, 1,1,3-trimethylbutyl, octyl, 1-methylheptyl, 2-methylheptyl, 3-methylheptyl, 4-methylheptyl, 1,1-dimethylhexyl, 2,2-dimethylhexyl, 3,3-dimethylhexyl, 4,4-dimethylhexyl, 1,2-dimethylhexyl, 1,3-dimethylhexyl, 1,4-dimethylhexyl, 1,2,3-trimethylpentyl, 1,1,2-trimethylpentyl, nonyl, 1-methyloctyl, 2,2-dimethylheptyl, 1,2,3-trimethylhexyl, 1,2,3,4-tetramethylpentyl, decyl, and the like. The terms "$C_2$-$C_{10}$ alkyl", "$C_1$-$C_4$ alkyl", "$C_1$-$C_3$ alkyl", and "$C_1$-$C_2$ alkyl" are included in this definition.

The term "$C_2$-$C_6$ alkenyl" refers to unsaturated aliphatic radicals of two to six carbon atoms, wherein one carbon to carbon double bond exists in the radical, and includes ethenyl, propenyl, 1-butenyl, 2-butenyl, 2-methylpropenyl, 1-pentenyl, 2-pentenyl, 3-pentenyl, 3-methyl-1-butenyl, 2-methyl-2-butenyl, 1-hexenyl, 2-hexenyl, 3-hexenyl, 2,3-dimethyl-2-butenyl, and the like. This definition also includes the term "$C_2$-$C_4$ alkenyl".

The term "$C_1$-$C_2$ alkoxy" refers to the aliphatic radicals of one to two carbon atoms attached to the remainder of the molecule by an oxygen atom, such as methoxy, and ethoxy.

The term "$C_3$-$C_6$ cycloalkyl" refers to saturated aliphatic rings of three to six carbon atoms, such as cyclopropyl, cyclobutyl, cyclopentyl, cyclohexyl, and the like.

The term "$C_1$-$C_4$ haloalkyl" refers to the straight and branched aliphatic radicals of one to four carbon atoms which have a halogen atom attached to the alkyl. The halogens include bromide, chloride, fluoride, and iodide.

The term "pyrrolidyl" refers to a pyrrolidine radical attached at the nitrogen. The term "piperidyl" refers to a piperidine radical attached at the nitrogen, while the term "piperazinyl" refers to a piperazine radical attached at one of the nitrogens. The term "morpholinyl" refers to a morpholine radical attached at the nitrogen.

## Preparation of Ureas of Formula IX

The process for making the (3-alkyl-5-isothiazolyl)ureas of formula IX is outlined in the following schematic:

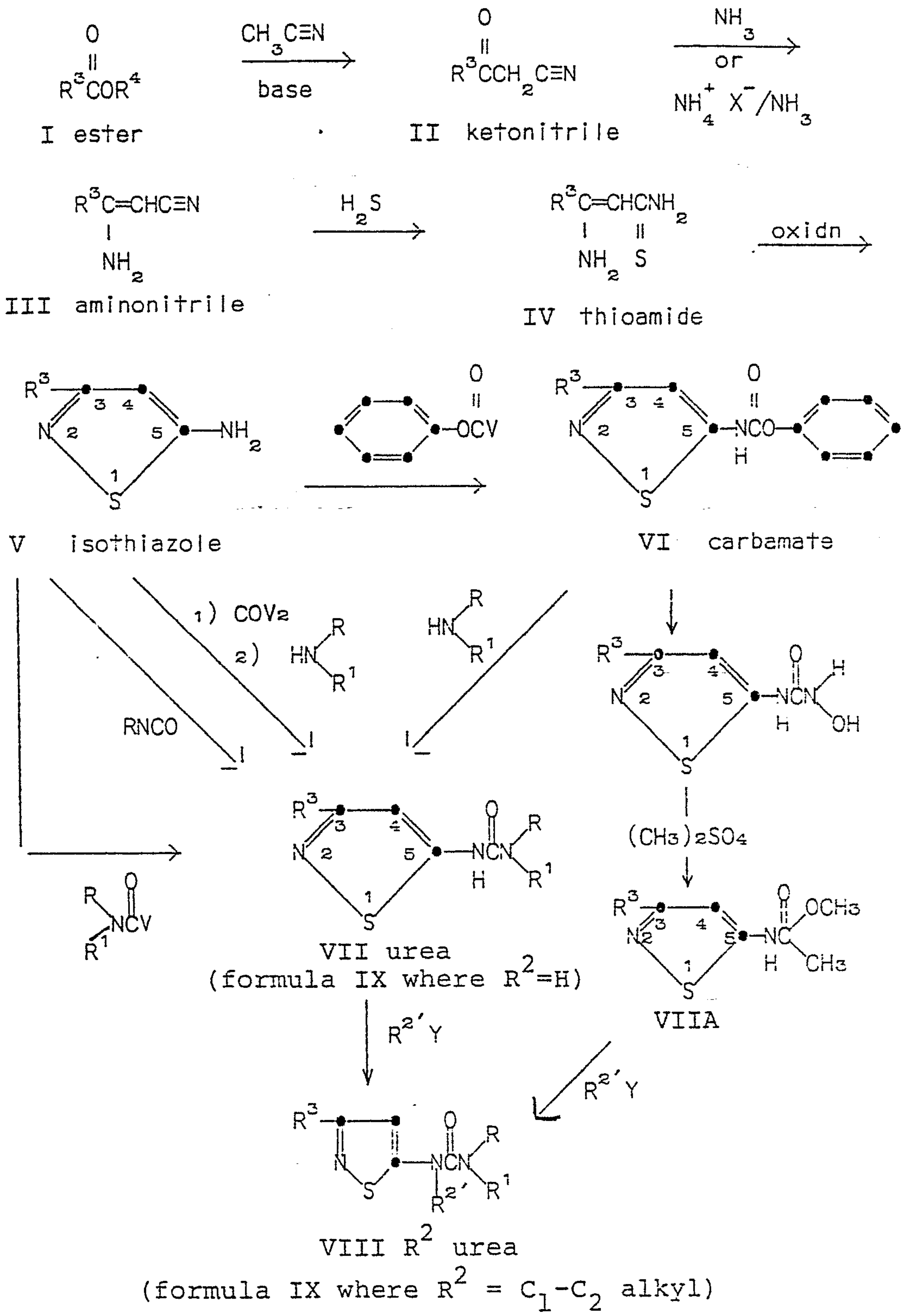


I ester

II ketonitrile

III aminonitrile

IV thioamide

V isothiazole

VI carbamate

VII urea
(formula IX where $R^2$=H)

VIIA

VIII $R^2$ urea
(formula IX where $R^2$ = $C_1$-$C_2$ alkyl)

wherein R, $R^1$, and $R^3$ are defined as above, and $R^{2'}$ is $C_1$-$C_2$ alkyl;

$R^4$ is $C_1$-$C_6$ alkyl or phenyl;

V is chloro or bromo;

X is nitrate, acetate, formate, bromide, chloride, or fluoride; and

Y is bromo, chloro, fluoro, or iodo.

A substituted ester of formula I is converted to the ketonitrile of formula II using acetonitrile and a base. The ketonitrile of formula II is then treated with ammonia or an ammonium salt-ammonia mixture to form the aminonitrile of formula III. Subsequently, thiation of this aminonitrile of formula III is completed using hydrogen sulfide. Upon thiation a thioamide of formula IV is formed, which is then oxidized to facilitate ring closure, resulting in the 3-substituted-5-aminoisothiazole of formula V.

Once the isothiazole of formula V is formed, it is reacted with a halogenated phenylformate, usually chloro, to generate a carbamate intermediate of formula VI. This intermediate of formula VI is subsequently aminated to produce the desired urea compound of formula VII.

Alternatively, the urea compound of formula VII can be made from (a) the reaction of a 5-aminoisothiazole of formula V and the appropriate alkylisocyanate in an inert organic solvent or from (b) the reaction of the isothiazole of formula V with phosgene to form an isocyanate and then reacting the isocyanate

with the appropriate amine or from (c) the reaction of a 5-aminoisothiazole of formula V with a carbamoyl halide.

A certain group of urea compounds of formula VII, such as the N-methyl-N-methoxy derivatives of formula VIIA, can be prepared by the alkylation of the corresponding hydroxyl urea.

Other, more-substituted ureas can also be prepared from already-made ureas of formulae VII and VIIA by alkylation.

## Compound I - Ester

Compound I, the substituted ester, can be obtained by methods well-known in the art. These include the preparation of the ester from the corresponding carboxylic acid, $R^3COOH$, wherein $R^3$ is defined above, and the appropriate alcohol, $R^4OH$, wherein $R^4$ is defined above, under acidic conditions. Typical acids used include concentrated sulfuric acid, dry hydrogen chloride, and boron trifluoride etherate, with boron trifluoride etherate preferred. Preferred alcohols are methanol and ethanol, wherein $R^4$ is methyl or ethyl, with the most preferred being methanol. The resulting preferred ester will be the methyl carboxylate. Preferred carboxylic acid groups, $R^3$, are listed above. Usually, to shift the equilibrium toward the ester, a large excess of alcohol is used with elevated temperatures, but the temperature can be from about ambient temperature to about the reflux temperature of the alcohol. Alternatively, the ester can be removed upon formation.

A more preferred preparation of the ester involves using the corresponding acid chloride, $R^3COCl$ and the appropriate alcohol, $R^4OH$. Esterification of the acid chloride is often carried out in the presence of a base, such as pyridine, aqueous sodium hydroxide, or others, under conditions described as the Schotten-Baumann technique. However, sometimes the use of the above-listed bases is unnecessary. (Typically, the acid chloride is prepared from its carboxylic acid using thionyl chloride, phosphorus oxychloride, oxalyl chloride, or phosphorus pentachloride.) Acid anhydrides can also undergo alcoholysis to form an ester, as do the acid chlorides.

The process called transesterification allows conversion of one ester to another, catalyzed by acidic or basic conditions. The acid can be concentrated sulfuric acid, dry hydrogen chloride, and the like, while the base can be an alkoxide ion, and the like. To shift the equilibrium to form the new ester, a large excess of the appropriate alcohol is used or the desired ester is removed upon formation.

The intermediates of formula I can be isolated, if desired, by typical techniques, such as extraction or recrystallization. However, isolation is not necessary when an intermediate is going to be used in a following reaction. Compound I is typically isolated by being washed with water and then extracted with ether and dried over magnesium sulfate.

Compound II - Ketonitrile

The ketonitrile, compound II, is formed from the substituted ester of formula I using a base condensation reaction with acetonitrile. The base present can be sodium metal, sodium ethoxide, sodium methoxide, sodium hydride, and the like. Sodium hydride is the base of choice and is usually dispersed in mineral oil. The substituted ester of formula I is allowed to react with from about one to about two equivalents of acetonitrile in the presence of the base and a solvent at the reflux temperature of the reaction mixture. The reaction continues for a time sufficient to bring about substantial completion, usually from about 4 hours to about 24 hours. Suitable solvents include tetrahydrofuran (THF), ether, toluene, benzene, ethanol, methanol, and the like, with THF the preferred solvent.

A preferred method for preparing Compound II is slurrying sodium hydride in THF and then adding the appropriate ester and acetonitrile, while the slurry is maintained at a temperature from about 60 to about 65°C. After the addition is complete, the mixture is heated to reflux for about 16 to about 24 hours.

The intermediates of formula II can be isolated, if desired, by typical techniques. The ketonitrile, Compound II, is formed in the reaction mixture as the sodium salt. Then the solvent is removed *in vacuo* before the salt is dissolved in water. Any residual mineral oil is washed out with hexane, and the free ketonitrile is obtained by acidifying with a mineral acid, such as hydrochloric acid and the like,

and extracting into ether. Any necessary crystallization can be done with ethanol, methylene chloride, cyclohexane, hexane, toluene, or appropriate mixtures thereof.

Compound III - Aminonitrile

Once the ketonitrile of formula II is formed, it can be converted to the aminonitrile, Compound III, by using a source of ammonia. Using ammonia under pressure provides a source of ammonia, with the pressure being generated by the reaction itself run in a sealed vessel. The reaction is heated at temperatures from about 100 to about 150°C for anywhere from about 4 to about 24 hours. Preferably, the reaction is conducted at about 150°C for about 16 hours. Although an organic solvent can be used in the reaction, such as ethanol, benzene, toluene, methanol, THF, and the like, with ethanol being preferred, such a solvent is not essential to the reaction. It is, however, necessary to use at least one equivalent of ammonia, but an excess of ammonia can be used if desired.

The preferred source of ammonia utilizes an ammonium salt and ammonia under atmospheric pressure at temperatures from about ambient temperature to about the reflux temperature of the solvent used. The ketonitrile, Compound II, is mixed in an inert organic solvent that already contains the ammonium salt. Typical solvents include ethanol, methanol, THF, and the like, with ethanol being preferred. Typical ammonium salts include ammonium chloride, ammonium

bromide, ammonium acetate, ammonium formate, ammonium nitrate, and the like, with ammonium nitrate being preferred, but, ammonium sulfate cannot be used. The amount of ammonium salt can be varied and an excess can be used; however, it is preferable to use less than or equal to one equivalent of the salt in the reaction. The resulting mixture of solvent and ammonium salt is then warmed to the desired temperature, which is usually the reflux temperature of the solvent, and ammonia is bubbled in. The reaction runs from about 4 to about 24 hours, with the preferred time being from about 16 to 24 hours.

Alternatively, Compound II may be reacted with one to two equivalents of ammonia with an ammonium salt added, all under pressure, at a temperature of about 25 to about 80°C for about 4 to about 24 hours.

A more preferred method of preparing Compound III uses ammonium nitrate as the desired ammonium salt, uses ethanol as the solvent, and is run for about 16 hours at the reflux temperature of ethanol, which is about 80°C.

Compound III, the aminonitrile, can be isolated, if desired, by removing the excess ammonia and solvent *in vacuo*, and then extracting with hexane, methylene chloride, ethyl acetate, ether, and the like. The mixture containing the product can also be dried over magnesium sulfate and then recrystallized. Typical solvents used for crystallization are toluene, hexane, cyclohexane, and the like.

Compound IV - Thioamide

Compound IV, a thioamide, can be made under pressure by following a procedure similar to that outlined in J. Goerdeler and H.W. Pohland, Chem. Ber., 94, 2950 (1961) and British Patent 1,153,186 (Netherlands Patent 6,608,094).

The aminonitrile, Compound III, is reacted with hydrogen sulfide under pressure; the pressure being generated by the reaction itself. This reaction is run in an organic solvent, such as methylene chloride, chloroform, and the like, with methylene chloride being preferred. A small amount of a base-catalyst (0.5-2%) is used to facilitate reaction and includes such bases as: triethylamine and potassium hydroxide, with the latter preferred. The temperature of the reaction can be from about 25 to about 100°C, with about 60°C being preferred, while the reaction can be allowed to continue from about 16 hours to about 72 hours, with about 48 hours being preferred.

A more preferred method of making the thioamide of formula IV with hydrogen sulfide utilizes a temperature at about 60°C for about 48 hours, with methylene chloride as the solvent, and potassium hydroxide as the catalyst.

Alternatively, the thioamide of formula IV can be prepared at atmospheric pressure by reacting the aminonitrile of formula III in an inert organic solvent such as pyridine, ethanol, toluene, benzene, and the like, pyridine being preferred, with hydrogen sulfide in the presence of a base-catalyst, such as triethylamine. Typically this type of reaction is run at a temperature from about 25 to about 100°C.

Another route, even more preferred, involves using a phase-transfer catalyst (PTC) at about one to two atmospheres of pressure (atm), following a procedure similar to that outlined in L. Cassar, S. Panossian, and C. Giordano, Synthesis, 917 (1978). The aminonitrile of formula III is dissolved in an inert organic solvent, such as benzene, toluene, 1,2-dichlorobenzene, diphenyl ether, and the like. (The use of a solvent can be avoided if a liquid aminonitrile is utilized.) Then an aqueous solution of sulfide ion (usually sodium sulfide nonahydrate is used to supply the sulfide ion) and the PTC are added. The use of dilute aqueous sulfide solutions are preferred, allowing the reaction to proceed more rapidly. The mixture is then heated to about 35-80°, preferably 70°C, under an atmosphere of hydrogen sulfide at a pressure of about 1-2 atm for about 4 to about 48 hours. Typical PTCs include tetrabutylammonium chloride, tetrabutylammonium bromide, tricaprylmethylammonium chloride, dibenzo-18-crown-6, and the like. The preferred PTC is tetrabutylammonium chloride (TBAC).

The thioamide of formula IV, prepared by the first route, can be isolated, if desired, by removing the excess hydrogen sulfide and solvent and then crystallizing it from other solvents, such as ethanol, toluene, benzene, ethyl acetate, chloroform, hexane, and the like. Once the preparation of the thioamide is completed using the PTC or second route, the water

layer is removed, and the organic layer is isolated. The product contained in the organic layer is precipitated, and can be recrystallized from solvents, such as methylene chloride, ether, benzene, hexane, ethyl acetate, and the like. Any excess solvent can be removed and/or dried before the desired product is obtained.

Compound V - Isothiazole

Ring closure of the thioamide of formula IV to form the isothiazole of formula V is facilitated by using an oxidizing agent, such as iodine, bromine, hydrogen peroxide, sodium hypochlorite, chloramine, chloramine T, chlorine, persalts such as potassium or ammonium persulfate, and the like. Preferred oxidizing agents are iodine and hydrogen peroxide. First, the thioamide is dissolved in an inert organic solvent, such as ether, benzene, ethanol water, and the like, and then the oxidizing agent is added.

After cyclization of the thioamide of formula IV, the isothiazole of formula V can be isolated if desired as follows. The organic layer containing the product can be dried and then the solvent removed. Crystallization can be done using toluene, ethanol, cyclohexane, acetone, hexane, methylene chloride, chloroform, ether, and mixtures thereof, and the like.

When impure thioamide of formula IV is used in the cyclization step, the isothiazole product of formula V is best purified by extracting it from the organic solvent with a dilute mineral acid, washing the

aqueous solution with an organic solvent, such as methylene chloride, and the like. The isothiazole of formula V is then freed with sodium or potassium hydroxide, and can be collected by filtration if it is a solid or it can be extracted into an inert organic solvent, such as methylene chloride and the like. Then the solution is dried and the solvent removed.

Compound VI - Carbamate

The carbamate of formula VI is typically prepared from the 5-aminoisothiazole of formula V and a chloro phenylformate, in an inert organic solvent, such as toluene. In addition, an acid receptor, such as an inorganic or organic base, should be present to react with the hydrogen chloride formed as a by-product. The preferred base to be used is pyridine.

The carbamate, Compound VI, can be isolated, if desired, by recrystallization from typical solvents such as ethanol, acetone, and the like, with an ethanol-water mixture being preferred.

Compound VII - Urea

The 3-alkyl-5-isothiazolylureas of formula VII can be prepared from the carbamates of formula VI or directly from the 5-aminoisothiazoles of formula V. The preferred method of preparation is using the carbamate, Compound VI, which is dissolved in an inert organic solvent, such as dimethylformamide (DMF), THF, benzene, and the like, with benzene being the preferred

solvent. Then the appropriately substituted amine of the formula $HNRR^1$ is added and the entire reaction is run for about 1/2 to about 24 hours at a temperature from about 25 to about 100°C. The preferred reaction conditions are about 80°C for about 1 hour.

A tertiary amine, such as triethylamine, is added when the substituted amine is an amine salt or free methoxymethylamine.

Certain ureas of formula VII, the N-methyl-N-methoxy derivatives of formula VIIA, can be prepared by the alkylation of the N'-(3-substituted-5-isothiazolyl)-N-hydroxyurea, using dimethyl sulfate. The hydroxyurea is generated from the appropriate carbamate of formula VI and hydroxylamine in a process similar to the process of forming ureas of formula VII from the carbamate of formula VI and substituted amine. Upon formation of the hydroxyurea, the dimethyl sulfate is added to form the compounds of formula VIIA.

Another preparation of Compound VII involves the reaction of a 5-aminoisothiazole of formula V with an alkylisocyanate of the formula RNCO. The reaction is run in an inert organic solvent, such as THF, benzene, ethyl acetate, and the like. Typically dibutyltin diacetate is added to the 5-aminoisothiazole of formula V and then the alkylisocyanate in solvent is added. The reaction is run at a temperature of from about 25 to about 100°C for about 1/2 to about 24 hours.

The 5-aminoisothiazole of formula V can also be reacted with a compound of the formula $COV_2$ wherein V is chloro or bromo, and then reacted with the substituted amine. Typically phosgene is used as the com-

pound of formula $COV_2$ and the isothiazole-phosgene reaction is run in the presence of an acid of the formula HV (with V being chloro or bromo.) The reaction is carried out in an inert organic solvent, such as toluene, and the like, at elevated temperatures of from about 60 to about 110°C. The substituted amine is then added (optionally in an inert organic solvent, such as toluene, chloroform, and the like) and the reaction run at a temperature from about 0 to about 100°C.

Another method of preparing the urea of formula VII is from the reaction of the 5-aminoisothiazole of formula V and a substituted carbamoyl halide of the formula $RR^1NCOV$. The halide is placed in pyridine or in an inert organic solvent, such as DMF, THF, benzene, and the like, with an acid acceptor, such as pyridine, present. Then the isothiazole of formula V is added and the reaction is run for about 1 to about 16 hours at a temperature from about 20 to about 60°C. Preferably, the reaction is at about 25°C for about 2 hours.

The desired urea of formula VII is isolated, if desired, by removing the solvent under vacuum and extracting with dilute acids, such as hydrochloric acid, sulfuric acid, and the like. After the extract is washed, the solution is made basic with sodium bicarbonate, sodium carbonate, sodium hydroxide, and the like. The urea can be recrystallized from typical solvents, such as ethanol-water, hexane, acetone, and the like, with ethanol-water being preferred.

## Compound VIII - $R^2$ Alkyl-substituted Ureas

Additionally-substituted ureas of formula VIII are prepared by an alkylation reaction between the unsubstituted urea and an alkyl halide of the formula $R^{2'}Y$, when $R^{2'}$ is $C_1$-$C_2$ alkyl and wherein Y is bromo, chloro, fluoro, or iodo. The unsubstituted urea of formula VII or VIIA and alkyl halide are usually reacted in an inert organic solvent, such as DMF and the like, in the presence of a base such as sodium hydride.

The acid addition salts are formed from the reaction of the urea of formula VII, VIIA or VIII in an inert organic solvent, such as ether and the appropriate acid.

The following examples are illustrative of the compounds of formula IX and the intermediates used in their preparation. All temperatures are in degrees Celsius and the melting points are uncorrected.

## Examples of preparation of Compound I

### Intermediate 1

### Ethyl 1-methylcyclohexylcarboxylate

One hundred grams (0.704 mole) of 1-methylcyclohexylcarboxylic acid and 97.02 g (0.77 mole) of oxalyl chloride (ethandioyl chloride) were mixed in 750 ml of carbon tetrachloride and stirred at room temperature until all hydrogen chloride gas evolution stopped. The carbon tetrachloride and excess oxalyl chloride were removed, and the crude 1-methylcyclo-

hexylcarboxyl chloride formed was added to 750 ml of ethanol. After stirring at room temperature for about 16 hours, the excess ethanol was removed and the product, ethyl 1-methylcyclohexylcarboxylate, was collected. Yield = 45%.

It was then vacuum distilled at .15 mm and the fraction coming off at 40-43° was collected.

NMR ($CDCl_3$): δ4.1 (q, 2H); δ2.3-1.7 (m, 2H); δ1.7-1.0 (m, 15H).

Intermediate 2

Methyl cyclopentylcarboxylate

A mixture of 100 g of cyclopentylcarboxylic acid and 10 ml of boron trifluoride etherate was mixed in 500 ml of methanol and heated at reflux temperature for about 16 hours. After cooling, the mixture was poured into water and extracted with ether. Then the ether was removed, and the mixture was dried and distilled at atmospheric pressure.

The fraction distilling at about 150-155° was collected, yielding 75.2 g (67%).

The following elemental analysis was obtained:

Calculated for $C_7H_{12}O_2$:

Theory: C, 65.60; H, 9.44

Found: C, 65.32; H, 9.17.

The following examples were prepared using the general procedure of Intermediate 2.

Intermediate 3

Methyl 2-ethylbutanoate

BP (boiling point) = 134-140°
Yield = 72 g (55%)
NMR ($CDCl_3$): δ3.6 (s, 3H); δ2.4-1.8 (m, 1H); δ1.8-1.1 (m, 4H); δ0.8 (t, 6H).

Intermediate 4

Methyl 2-methylbutanoate

Yield = 30%
BP = 118-122°
Calculated for $C_6H_{12}O_2$
Theory: C, 62.04; H, 10.41
Found: C, 61.80; H, 10.12.

Intermediate 5

Methyl 1-methylcyclopropylcarboxylate

Yield = 88 g (51%)
BP = 122-126°

## Examples of preparation of Compound II

### Intermediate 6

### 3-Ketoheptanenitrile

A dispersion of 76.8 g (1.6 mole) of 50% sodium hydride in mineral oil was added to 800 ml of tetrahydrofuran (THF). This solution was stirred under reflux as a solution of 104 g (0.8 mole) ethyl valerate and 65.6 g (1.6 mole) of acetonitrile in 250 ml of THF was added dropwise. After about one hour the foaming became excessive, so the heat was removed and the solution was stirred for about 16 hours.

Then the solution was refluxed briefly and cooled, followed by the dropwise addition of 40 ml of isopropyl alcohol. The solvent was then removed under vacuum. Six hundred ml of water was added and then extracted with 500 ml hexane and 500 ml of a hexane-ether mixture. A black material was filtered off and the solution acidified with concentrated hydrochloric acid and then extracted with two-300 ml portions of ether.

The combined extracts were washed with 150 ml of water and dried over magnesium sulfate before the solvent was removed under vacuum. The yield was 90 g of an oil (88%).

NMR ($CDCl_3$): δ3.60 (s, 2H); δ2.60 (t, 2H); δ1.8-0.8 (m, 7H).

The following examples were prepared using the general procedure of Intermediate 6.

Intermediate 7

3-Ketohexanenitrile

Yield = 89 g (96%)

NMR ($CDCl_3$): δ3.63 (s, 2H); δ2.60 (t, 2H); δ1.65 (m, 2H); δ0.97 (t, 3H).

Intermediate 8

4-Methyl-3-ketopentanenitrile

Yield = 76.5 g 86%)

BP = 95-100°/8 mm; 72-76°/1 mm

NMR ($CDCl_3$): δ3.65 (s, 2H); δ2.8 (m, 1H); δ1.08 (d, 6H).

Intermediate 9

3-Cyclohexyl-3-ketopropionitrile

Yield 94 g (89%)

BP = 95-100°/1 mm

Calculated for $C_9H_{13}NO$:

Theory: C, 71.49; H, 8.67; N, 9.26

Found: C, 71.52; H, 8.55; N, 9.42.

NMR ($CDCl_3$): δ3.55 (s, 2H); δ2.7-1.2 (m, 11H).

Intermediate 10

3-Ketopentanenitrile

Yield = 74 g (95%)

NMR ($CDCl_3$): δ3.63 (s, 2H); δ2.65 (q, 2H); δ1.1 (t, 3H).

Intermediate 11

4-Cyclohexyl-3-ketobutanenitrile

Yield = 61 g (89%)

NMR ($CDCl_3$): δ3.5 (s, 2H); δ2.5-0.6 (m, 13H).

Intermediate 12

3-(1-Methylcyclohexyl)-3-ketopropionitrile

Yield = 66%

BP = 112-115°/.7 mm

NMR ($CDCl_3$): δ3.8 (s, 2H); δ2.2-1.0 (m, 14H).

Intermediate 13

4,4-Diethyl-3-ketopentanenitrile

Yield = 55.5 g (69%)

BP = 94-96°/1.5 mm

Calculated for $C_9H_{15}NO$:

Theory: C, 70.55; H, 9.87; N, 9.14

Found: C, 70.28; H, 9.95; N, 8.88.

Intermediate 14

4,4-Dimethyl-3-ketopentanenitrile

Yield = 82.9 g (83%)
MP (melting point) = 60-62°

Intermediate 15

3-Cyclopentyl-3-ketopropionitrile

Yield = 31.8 g (23%)
NMR ($CDCl_3$): δ3.7 (s, 2H); δ3.4-2.5 (m, 1H); δ2.2-1.5 (m, 8H).

Intermediate 16

3-(1-Methylcyclopropyl)-3-ketopropionitrile

NMR ($CDCl_3$): δ3.7 (s, 2H); δ1.4 (m, 4H); δ0.9 (m, 3H).

Intermediate 17

4-Methyl-3-ketohexanenitrile

Yield = 57.77 g (77%)
BP = 49-52°/.1 mm

Intermediate 18

4-Ethyl-3-ketohexanenitrile

Yield = 67 g (88%)

NMR ($CDCl_3$): δ3.65 (s, 2H); δ2.8 (m, 1H); δ1.7, m, 4H); δ1.0 (t, 6H).

Intermediate 19

5-Methyl-3-ketohexanenitrile

Yield = 99.93 g 89%)

NMR ($CDCl_3$): δ3.7 (s, 2H); δ2.7-1.7 (m, 3H); δ1.0 (d, 6H).

Examples of preparation of Compound III

Intermediate 20

3-Amino-3-cyclohexylpropenenitrile

Ninety-one grams (0.6 mole) of 3-cyclohexyl-3-ketopropionitrile was added to 300 ml of liquid ammonia and 350 ml of ethanol and the mixture was heated in a sealed vessel at about 150° for about 16 hours. The solvent was removed under vacuum to leave an oil. Hexane was added to the oil and then the solution was cooled, leaving beige crystals with a MP of 44-47° . The product was recrystallized from hexane, giving a MP of 60-63° . The overall yield was 65 g (72%).

The following elemental analysis was obtained.

Calculated for $C_9H_{14}N_2$:

Theory: C, 71.96; H, 9.39; N, 18.65

Found: C, 72.17; H, 9.54; N, 18.90.

NMR ($CDCl_3$): δ4.7 (broad, 2H); δ3.7 (s, 1H); δ2.0-1.0 (m, 1H).

The following examples were prepared using the general procedure of Intermediate 20.

Intermediate 21

3-Amino-4,4-dimethyl-2-pentenenitrile

Yield = 93%

MP = 48-49°

NMR ($CDCl_3$): δ4.9 (broad, 2H); δ3.95 (s, 1H); δ1.2 (s, 9H).

Intermediate 22

3-Amino-4-methyl-2-pentenenitrile

Yield = 52 g (70%)

NMR ($CDCl_3$): δ5.0 (broad, 2H); δ4.0 (s, 1H); δ3.75 (s, 1H); δ3.2-2.0 (m, 1H); δ1.15 (d, 6H).

Intermediate 23

3-Amino-4-cyclohexyl-2-butenenitrile

Yield = 24.5 g (55%)

NMR ($CDCl_3$): δ4.7 (m, 2H); δ3.7 (s, 1H); δ2.3-0.4 (m, 13H).

Intermediate 24

3-Amino-3-(1-methylcyclohexyl)propenenitrile

Yield = 36.02 g (73%)

MP = 82-84°

Calculated for $C_{10}H_{16}N_2$

Theory: C, 73.13; H, 9.82; N, 17.06

Found: C, 72.89; H, 9.67; N, 16.79.

Intermediate 25

3-Amino-4-ethyl-4-methyl-2-hexenenitrile

Yield = 16.85 g (34%)

NMR ($CDCl_3$): δ5.3-4.5 (m, 2H); δ3.8 (s, 1H); δ1.5 (m, 4H); δ0.9 (m, 9H).

Intermediate 26

3-Amino-2-pentenenitrile

Yield = 25 g (50%)

BP = 80-85°/1 mm

Intermediate 27

3-Amino-4,4-dimethyl-2-pentenenitrile

Twelve and one-half grams (0.1 mole) of 4,4-dimethyl-3-ketopentanenitrile, 8.5 g (0.10 mole) of ammonium nitrate, and 100 ml of ethanol were stirred at room temperature as ammonia was bubbled into the solution. After 2.5 hours, TLC (methylene chloride) showed that product was present. The solution was then heated to reflux and the ammonia addition was continued for about 16 hours.

One hundred-fifty ml of water was added and then the ethanol was removed under vacuum. The oil formed was extracted with 150 ml of ether, then was washed with 25 ml of water, and dried over magnesium sulfate. The ether was removed under vacuum, then hexane was added. Cooling the solution resulted in a pale yellow solid. The yield was 11.5 g (93%) with a MP of 48-49°.

The following elemental analysis was obtained:

Calculated for $C_7H_{12}N_2$

Theory: C, 67.70; H, 9.74; N, 22.56

Found: C, 67.49; H, 9.57; N, 22.83.

The following examples were prepared using the general procedure of Intermediate 27.

Intermediate 28

3-Amino-4-methyl-2-pentenenitrile

Yield = 66.2 g (97%)

NMR ($CDCl_3$): δ5.0 (broad, 2H); δ4.0 (s, 1H); δ3.75 (s, 1H); δ3.2-2.0 (m, 1H); δ1.15 (d, 6H).

Intermediate 29

3-Amino-2-hexenenitrile

Yield = 75 g oil (85%)

NMR ($CDCl_3$): δ5.2 (broad, 2H); δ4.1 (s, 1H); δ3.7 (s, 1H); δ2.8-0.9 (m, 7H).

Intermediate 30

3-Amino-2-heptenenitrile

Yield = 81 g (90%)

NMR ($CDCl_3$): δ5.2 (broad, 2H); δ4.0 (s, 1H); δ3.7 (s, 1H); δ2.5-0.8 (m, 9H).

## Intermediate 31

### 3-Amino-4-ethyl-4-methyl-2-hexenenitrile

Yield = 59 g (97%)
MP = 45-48°

Calculated for $C_9H_{16}N_2$:

Theory: C, 71.01; H, 10.59; N, 18.40
Found: C, 71.09; H, 10.74; N, 18.18.

## Intermediate 32

### 3-Amino-4-methyl-2-hexenenitrile

Yield = 42.2 g (56%)
BP = 97-102°/.25-.5 mm

Calculated for $C_7H_{12}N_2$:

Theory: C, 67.71; H, 9.74; N, 22.56
Found: C, 67.47; H, 9.69; N, 22.41.

## Intermediate 33

### 3-Amino-3-(1-methylcyclopropyl)-2-propenenitrile

Yield = 42.7 g (88%)
NMR ($CDCl_3$/DMSO): δ6.2 (s, 1H); δ5.8 (broad, 2H); δ1.4 (s, 3H); δ0.8 (m, 4H).

Intermediate 34

3-Amino-2-pentenenitrile

Yield = 51 g (70%)
BP = 80-85°/1 mm

Intermediate 35

3-Amino-4-ethyl-2-hexenenitrile

Yield = 36.61 g (64%)
BP = 95-98°/.01 mm

Intermediate 36

3-Amino-3-cyclopentylpropenenitrile

Yield = 85 g (100%)

Intermediate 37

3-Amino-5-methyl-2-hexenenitrile

Yield = 62.75 g (63%)
NMR ($CDCl_3$): δ5.2 (broad, 2H); δ4.1 (s, 1H); δ3.6 (s, 1H); δ2.8-1.7 (m, 3H); δ1.0 (m, 6H).

## Examples of preparation of Compound IV

### Intermediate 38

### 3-Amino-3-cyclohexylpropenethioamide

The following were added to 58.3 g of 3-amino-3-cyclohexylpropenenitrile: 500 ml of methylene chloride, 60 ml of liquid hydrogen sulfide, and 1.0 g of potassium hydroxide. The reaction mixture was kept in a sealed vessel at about 60° for about 24 hours.

A black solid was filtered, and the solvent was evaporated on a steam bath to leave a black oil. The oil was chromatographed on grade 62 silica gel in a toluene-ethyl acetate mixture. Much of the black oil came off with the toluene, but NMR did not indicate the desired product. Then a solid was eluted with about 25% ethyl acetate in toluene and recrystallized from a chloroform-hexane mixture. MP of 139-141°. NMR indicated the desired product. The solid was recrystallized from chloroform-hexane to give white crystals with a MP of 141-143°. The yield was 3.3 g (5%).

The following elemental analysis was obtained:

Calculated for $C_9H_{16}N_2S$:

Theory: C, 58.65; H, 8.75; N, 15.20; S, 17.40

Found: C, 58.75; H, 9.00; N, 15.17; S, 17.61.

The following examples were prepared using the general procedure of Intermediate 38.

Intermediate 39

3-Amino-4-cyclohexyl-2-butenethioamide

Yield = 12.25 g (69%)
MP = 89-91°
Calculated for $C_{10}H_{18}N_2S$:
Theory: C, 60.56; H, 9.15; N, 14.12; S, 16.17
Found: C, 60.48; H, 8.94; N, 13.85; S, 16.06.

Intermediate 40

3-Amino-3-(1-methylcyclohexyl)propenethioamide

Yield = 4 g (10%)
NMR ($CDCl_3$): δ8.0 (broad, 2H); δ6.3 (broad, 2H); δ2.3-0.7 (m, 13H).

Intermediate 41

3-Amino-2-pentenethioamide

Yield = 19 g (59%)
NMR ($CDCl_3$): δ8.1 (broad, 2H) δ6.6 (broad, 2H); δ5.2 (s, 1H); δ2.2 (q, 2H); δ1.3 (t, 3H).

Intermediate 42

3-Amino-4-ethyl-4-methyl-2-hexenethioamide

Yield = 12.8 g (68%)

NMR ($CDCl_3$): δ8.0 (broad, 2H); δ6.7 (broad, 2H); δ5.2 (s, 1H); δ2.5-0.8 (m, 11H).

Intermediate 43

3-Amino-4,4-dimethyl-2-pentenethioamide

Yield = 58 g (59%)

NMR ($CDCl_3$): δ8.6-8 (m, 2H); δ6.6-5.8 (m, 2H); δ5.3 (s, 1H); δ1.2 (s, 9H).

Intermediate 44

3-Amino-2-heptenethioamide

A solution of 81 g of 3-amino-2-heptenenitrile, 5 g of sodium sulfide nonahydrate ($Na_2S \cdot 9H_2O$), 3.8 g of tricaprylmethylammonium chloride, 100 ml of benzene, and 100 ml of water were stirred under an atmosphere of hydrogen sulfide at about 65-70° for about 16 hours.

The solution was cooled and extracted with 200 ml of ether. The extract was washed with 100 ml of water before drying over magnesium sulfate. The remaining solvent was removed under vacuum to leave 95 g

of a dark oil. NMR indicated that approximately 50% of the desired product was formed. NMR ($CDCl_3$): δ8.1 (broad, 2H); δ6.5 (broad, 2H); δ5.2 (s, 1H); δ2.4-1.7 (m, 9H).

Intermediate 45

3-Amino-2-hexenethioamide

Seventy-five grams of 3-amino-2-hexenenitrile, 6 g of sodium sulfide nonahydrate, 3.4 g of tetrabutylammonium chloride, 100 ml of benzene, and 100 ml of water were stirred at about 65-70° under an atmosphere of hydrogen sulfide for about 16 hours. The product was extracted with 400 ml of ether and the ether extract was washed with 100 ml of water. Then the extract was dried over magnesium sulfate and the ether was removed under vacuum, leaving a black oil. The yield was 84 g. NMR indicated the desired product was present to the extent of 25%. NMR ($CDCl_3$): δ8.0 (broad, 2H); δ6.6 (broad, 2H); δ5.1 (s, 1H); δ2.5-0.8 (broad, 7H).

The following examples were prepared using the general procedure of Intermediate 45.

Intermediate 46

3-Amino-3-cyclohexylpropenethioamide

Yield = 7.7 g (60%)

NMR ($CDCl_3$): δ8.0 (broad, 2H); δ6.2 (broad, 2H); δ2.3-0.6 (m, 11H).

Intermediate 47

3-Amino-4,4-dimethyl-2-pentenethioamide

Yield = 83.3 g
Second crop yield = 38.25 g (85%)
NMR ($CDCl_3$): δ8.6-8 (m, 2H); δ6.6-5.8 (m, 2H); δ5.3 (s, 1H); δ1.2 (s, 9H).

Intermediate 48

3-Amino-3-(1-methylcyclopropyl)propenethioamide

NMR (DMSO): δ8.8-8.2 (broad, 2H); δ8.0-7.2 (broad, 2H); δ5.2 (s, 1H); δ1.3 (s, 3H); δ0.7 (d, 4H).

Intermediate 49
3-Amino-4-methyl-2-pentenethioamide

Yield = 240 g (82%)
NMR ($CDCl_3$): δ8.6-7.8 (broad, 2H); δ6.9-6.4 (broad, 2H); δ5.3 (s, 1H); δ2.3 (m, 1H); δ1.2 (d, 6H).

Intermediate 50

3-Amino 2-pentenethioamide

Yield = 46.85 g (68%)
NMR ($CDCl_3$): δ8.5 (broad, 2H); δ6.2 (broad, 2H); δ5.1 (s, 1H); δ2.1 (q, 2H); δ1.0 (m, 3H).

Intermediate 51

3-Amino-3-cyclopentylpropenethioamide

Yield = 59.8 g (58%)

Intermediate 52

3-Amino-5-methyl-2-hexenethioamide

Yield = 62.5 g (80%)

NMR ($CDCl_3$): δ8.1 (broad, 2H); δ6.6 (broad, 2H); δ5.2 (s, 1H); δ2.7-1.8 (m, 3H); δ1.0 (m, 6H).

The following example was prepared using the general procedure of Intermediate 45, except toluene was used instead of benzene.

Intermediate 53

3-Amino-4,4-dimethyl-2-pentenethioamide

Yield = 100 g (14%)

NMR ($CDCl_3$): δ8.6-8 (m, 2H); δ6.6-5.8 (m, 2H); δ5.3 (s, 1H); δ1.2 (s, 9H).

Examples of preparation of Compound V

Intermediate 54

5-Amino-3-cyclohexylisothiazole

Four grams (0.024 mole) of potassium carbonate, 2.7 g (0.015 mole) of 3-amino-3-cyclohexylpropenethioamide, and 175 ml of ether were stirred under reflux. Then 3.7 g (0.015 mole) of iodine in 50 ml of ether was added dropwise. The reflux was continued for about 4 hours, then the solution was cooled and 150 ml of water was added, causing an initial exotherm. The ether layer was dried over magnesium sulfate and the ether was removed under vacuum, leaving a brown solid. The solid was recrystallized from a toluene-hexane mixture. The MP was 132-136°.

The following elemental analysis was obtained:
Calculated for $C_9H_{14}N_2S$:

Theory: C, 59.30; H, 7.74; N, 15.34
Found: C, 59.28; H, 7.91; N, 15.26.

NMR ($CDCl_3$): δ6.1 (s, 1H); δ4.5 (broad, 2H); δ2.6 (broad, 1H); δ2.2-1.2 (broad, 10H).

The following examples were prepared using the general procedure of Intermediate 54.

Intermediate 55

5-Amino-3-(1-methylethyl)isothiazole

Yield = 14.4 g
Second crop = 23.5 g (54%)
MP = 49-51°

Calculated for $C_6H_{10}N_2S$:
Theory: C, 50.64; H, 7.09; N, 19.70
Found: C, 50.44; H, 6.88; N, 19.50.

NMR ($CDCl_3$): δ6.1 (s, 1H); δ5.0 (broad, 2H); δ2.9 (m, 1H); δ1.25 (d, 6H).

Intermediate 56

5-Amino-3-propylisothiazole

Yield = 10.3 g (49%)
NMR ($CDCl_3$): δ6.0 (s, 1H); δ5.3 (broad, 2H); δ2.6 (t, 2H); δ1.6 (m, 2H); δ0.95 (t, 3H).

Intermediate 57

5-Amino-3-ethylisothiazole

Yield = 6.7 g (60%)
NMR ($CDCl_3$): δ6.1 (s, 1H); δ5.4 (broad, 2H); δ2.6 (q, 2H); δ1.2 (t, 3H).

## Intermediate 58

### 5-Amino-3-butylisothiazole

Yield = 16.6 g (36%)

NMR ($CDCl_3$): δ6.1 (s, 1H); δ5.0 (broad, 2H); δ2.6 (t, 2H); δ1.6 (m, 2H); δ1.4 (m, 2H); δ0.9 (t, 3H).

## Intermediate 59

### 5-Amino-3-benzylisothiazole

Yield = 4 g (77%)

MP = 115-120°

Calculated for $C_{10}H_{10}N_2S$:

Theory: C, 63.13; H, 5.30; N, 14.72

Found: C, 62.90; H, 5.36; N, 14.51.

## Intermediate 60

### 5-Amino-3-(1-methylcyclohexyl)isothiazole

Yield = 70%

MP = 103-105°

Calculated for $C_{10}H_{16}N_2S$:

Theory: C, 61.18; H, 8.22; N, 14.27; S, 16.33

Found: C, 60.99; H, 7.93; N, 13.97; S, 16.45.

Intermediate 61

5-Amino-3-(1-methylcyclopropyl)isothiazole

Yield = 13.7 g (88%)

Intermediate 62

5-Amino-3-(1-methylpropyl)isothiazole

Yield = 22.5 g (49%)

NMR ($CDCl_3$): δ6.0 (s, 1H); δ5.4-4.8 (broad, 2H); δ2.7 (m, 1H); δ1.6 (m, 2H); δ1.1 (d, 3H); δ0.9 (t, 3H).

Intermediate 63

5-Amino-3-(1,1-dimethylethyl)isothiazole

Yield = 61 g (96%)

MP = 91-93°

Calculated for $C_7H_{12}N_2S$:

Theory: C, 53.81; H, 7.74; N, 17.93; S, 20.52

Found: C, 53.60; H, 7.45; N, 17.63; S, 20.50.

Intermediate 64

5-Amino-3-(1-ethyl-1-methylpropyl)isothiazole monohydrochloride

Yield = 34 g (67%)
MP = 140-142°

Calculated for $C_9H_{16}N_2S \cdot HCl$:
Theory: C, 48.97; H, 7.76; N, 12.69; S, 14.52; Cl, 16.06
Found: C, 48.94; H, 7.51; N, 12.83; S, 14.25; Cl, 16.32.

Intermediate 65

5-Amino-3-(1-ethylpropyl)isothiazole

Yield = 6.1 g (35%)
NMR ($CDCl_3$): δ6.2 (s, 1H); δ5.4-4.7 (broad, 2H); δ2.6 (m, 1H); δ1.7 (m, 4H); δ0.9 (t, 6H).

Intermediate 66

5-Amino-3-(2-methylpropyl)isothiazole

Yield = 21 g (53%)

Calculated for $C_7H_{12}N_2S$:
Theory: C, 53.81; H, 7.74; N, 17.93; S, 20.52
Found: C, 53.82; H, 7.60; N, 17.69; S, 20.26.

NMR ($CDCl_3$): δ6.1 (s, 1H); δ5.0 (broad, 2H); δ2.5 (d, 2H); δ2.0 (broad, 1H); δ0.9 (t, 6H).

Intermediate 67

5-Amino-3-cyclopentylisothiazole

Yield = 11.7 g (14%)
MP = 71-73°
NMR ($CDCl_3$/DMSO): δ6.1 (s, 1H); δ4.5 (broad, 2H); δ3.1 (broad, 1H); δ2.0 (broad, 2H); δ1.8 (broad, 6H).

Examples of preparation of Compound VI

Intermediate 68

Phenyl N-(3-butyl-5-isothiazolyl)carbamate

To a solution of 8.6 g of (0.055 mole) of 3-(n-butyl)-5-aminoisothiazole in 65 ml of pyridine, was added dropwise 8.6 g (0.055 mole) of phenyl chloroformate with stirring. Stirring was continued for about two hours, then the solution was poured into ice-water. A yellow solid was collected and washed with water. Then it was recrystallized from an ethanol-water mixture, yielding 13.2 g (87%). The MP was 159-161°.

The following elemental analysis was obtained:
Calculated for $C_{14}H_{16}N_2SO_2$:
Theory: C, 60.85; H, 5.84; N, 10.14
Found: C, 60.80; H, 5.94; N, 10.06.

The following examples were prepared using the general procedure of Intermediate 68.

Intermediate 69

Phenyl N-(3-propyl-5-isothiazolyl)carbamate

Yield = 11.8 g (69%)
MP = 168-170°

Calculated for $C_{13}H_{14}N_2O_2S$:
Theory: C, 59.52; H, 5.38; N, 10.68
Found: C, 59.23; H, 5.43; N, 10.65.

Intermediate 70

Phenyl N-(3-cyclohexyl-5-isothiazolyl)carbamate

Yield = 2.5 g (92%)
MP = 296-298°
Calculated for $C_{16}H_{18}N_2O_2S$:
Theory: C, 63.55; H, 6.00; N, 9.26
Found: C, 63.32; H, 6.04; N, 9.02.

Intermediate 71

Phenyl N-[3-(1-methylethyl)-5-isothiazolyl]carbamate

Yield = 3.5 g (79%)
MP = 141-145°

Calculated for $C_{13}H_{14}N_2O_2S$:

Theory: C, 59.52; H, 5.38; N, 10.68

Found: C, 59.30; H, 5.34; N, 10.44.

Intermediate 72

Phenyl N-(3-benzyl-5-isothiazolyl)carbamate

Yield = 3.1 g (59%)

MP = 152-155°

Calculated for $C_{17}H_{14}N_2O_2S$:

Theory: C, 65.79; H, 4.55; N, 9.03

Found: C, 65.51; H, 4.53; N, 8.82.

Intermediate 73

Phenyl N-[3-(cyclohexylmethyl)-5-isothiazolyl]carbamate

Yield = 2.5 g (79%)

MP >240°

Intermediate 74

Phenyl N-[3-(1-methylcyclohexyl)-5-isothiazolyl]-carbamate

Yield = 2.3 g (72%)

MP = 168-170°

Calculated for $C_{17}H_{20}N_2O_2S$:

Theory: C, 64.53; H, 6.37; N, 8.85; S, 10.13

Found: C, 64.81; H, 6.27; N, 8.65; S, 9.89.

Intermediate 75

Phenyl N-[3-(1,1-dimethylethyl)-5-isothiazolyl]carbamate

Yield = 166 g (95%)
MP = 162-164°

Intermediate 76

Phenyl N-[3-(1-methylpropyl)-5-isothiazolyl]carbamate

Yield = 31.1 g (86.5%)
MP = 111-113°

Calculated for $C_{14}H_{16}N_2O_2S$:

Theory: C, 60.85; H, 5.84; N, 10.14; S, 11.60

Found: C, 60.62; H, 5.77; N, 10.33; S, 11.37.

Intermediate 77

Phenyl N-[3-(1-ethyl-1-methylpropyl)-5-isothiazolyl]-carbamate

Yield = 25 g (54%)
MP = 187-189°

Calculated for $C_{16}H_{20}N_2O_2S$:

Theory: C, 63.13; H, 6.62; N, 9.20; S, 10.53

Found: C, 63.37; H, 6.40; N, 9.26; S, 10.65.

Intermediate 78

Phenyl N-(3-ethyl-5-isothiazolyl)carbamate

Yield = 0.42 g (16%)

MP = 154-157°

Calculated for $C_{12}H_{12}N_2O_2S$:

Theory: C, 58.05; H, 4.87; N, 11.28

Found: C, 58.30; H, 4.98; N, 11.25.

Intermediate 79

Phenyl N-[3-(1-ethylpropyl)-5-isothiazolyl]carbamate

Yield = 10 g (98%)

MP = 124-127°

Calculated for $C_{15}H_{18}N_2O_2S$:

Theory: C, 62.04; H, 6.25; N, 9.65

Found: C, 61.80; H, 6.08; N, 9.50.

Intermediate 80

Phenyl N-[3-(2-methylpropyl)-5-isothiazolyl]carbamate

Yield = 20 g (72%)
MP = 148-150°
Calculated for $C_{14}H_{16}N_2O_2S$:
Theory: C, 60.85; H, 5.84; N, 10.14; S, 11.60
Found: C, 60.73; H, 5.69; N, 10.06; S, 11.49.

Intermediate 81

Phenyl N-(3-cyclopentyl-5-isothiazolyl)carbamate

Yield = 8 g (93%)
MP = 169-171°
NMR (DMSO): δ11.9 (s, 1H); δ7.4 (broad, 5H); δ6.7 (s, 1H); δ3.1 (broad, 1H); δ2.0 (broad, 2H); δ1.7 (broad, 6H).

Examples of preparation of Compounds of Formula IX

Example 1

N'-(3-Butyl-5-isothiazolyl)-N,N-dimethylurea

To 50 ml of benzene containing 6 g of dimethylamine was added 3.3 g (0.012 mole) of phenyl N-(3-butyl-5-isothiazolyl)carbamate. The solution was stirred under reflux for about three hours. After the solvent was removed under vacuum and 50 ml of ether was added, the product was extracted into 30 ml of 2N hydrochloric acid. The aqueous extract was washed with 30 ml of ether and then the solution was made basic with saturated aqueous sodium bicarbonate.

A solid was then collected and washed with water. The MP was 109-111°. The yield was 2.4 g (82%).

The following elemental analysis was obtained:

Calculated for $C_{10}H_{17}N_3SO$:

Theory: C, 52.84; H, 7.54; N, 18.48

Found: C, 52.79; H, 7.41; N, 18.23.

The following examples were prepared using the general procedure of Example 1, with the appropriate carbamate being reacted with the listed reactant.

## Example 2

### N'-(3-Propyl-5-isothiazolyl)-N-methoxy-N-methylurea

Reactant - O,N-dimethylhydroxylamine

Yield = 1.6 g (54%)

MP = 92-96°

## Example 3

### N'-(3-Propyl-5-isothiazolyl)-N,N-dimethylurea

Reactant - dimethylamine

Yield = 2.2 g (86%)

MP = 119-123°

Calculated for $C_9H_{15}N_3OS$:

Theory: C, 50.68; H, 7.09; N, 19.70

Found: C, 50.41; H, 6.91; N, 19.51.

Example 4

N'-[3-(n-Butyl)-5-isothiazolyl]-N-methoxy-N-methylurea

Reactant - O,N-dimethylhydroxylamine hydrochloride

Yield = 1.8 g (62%)

MP = 86-88°

Calculated for $C_{10}H_{17}N_3O_2S$:

Theory: C, 49.36; H, 7.04; N, 17.27

Found: C, 49.46; H, 6.76; N, 17.04.

Example 5

N'-(3-Cyclohexyl-5-isothiazolyl)-N,N-dimethylurea

Reactant - dimethylamine hydrochloride

Yield = 0.7 g (77%)

MP = 235-237°

Calculated for $C_{12}H_{19}N_3OS$:

Theory: C, 56.89; H, 7.56; N, 16.58

Found: C, 56.90; H, 7.82; N, 16.33.

## Example 6

N'-(3-Cyclohexyl-5-isothiazolyl)-N-methoxy-N-methylurea

Reactant - dimethylhydroxylamine hydrochloride
Yield = 0.5 g (56%)
MP = 135-137°

Calculated for $C_{12}H_{19}N_3O_2S$:
Theory: C, 53.51; H, 7.11; N, 15.60
Found: C, 53.77; H, 7.08; N, 15.31.

## Example 7

N'-[3-(1-Methylethyl)-5-isothiazolyl]-N,N-dimethylurea

Reactant - dimethylamine hydrochloride
Yield = 0.8 g (49%)
MP = 169-172°

Calculated for $C_9H_{15}N_3OS$:
Theory: C, 50.68; H, 7.09; N, 19.70
Found: C, 50.51; H, 7.02; N, 19.54.

## Example 8

N'-[3-(1-Methylethyl)-5-isothiazolyl]-N-methoxy-N-methylurea

Reactant - O,N-dimethylhydroxylamine hydrochloride
Yield = 1 g (46%)
MP = 91-92°

Calculated for $C_9H_{15}N_3O_2S$:

Theory: C, 47.16; H, 6.55; N, 18.34

Found: C, 47.15; H, 6.40; N, 18.01.

## Example 9

N'-[3-(Benzyl)-5-isothiazolyl]-N,N-dimethylurea

Reactant - dimethylamine
Solvent - tetrahydrofuran
Yield = 1.3 g (55%)
MP = 240-242°

Calculated for $C_{13}H_{15}N_3OS$:

Theory: C, 59.75; H, 5.79; N, 16.08

Found: C, 59.83; H, 5.75; N, 16.12.

## Example 10

N'-[3-(1,1-Dimethylethyl)-5-isothiazolyl]-N-ethyl-N-methylurea

Reactant - ethylmethylamine
Yield = 0.5 g (41%)
MP = 197-199°

Calculated for $C_{11}H_{19}N_3OS$:

Theory: C, 54.74; H, 7.94; N, 17.41; S, 13.28

Found: C, 55.01; H, 7.69; N, 17.21; S, 13.37.

Example 11

N'-[3-(1,1-Dimethylethyl)-5-isothiazolyl]-N,N-diethylurea

Reactant - diethylamine
Yield = 0.7 g (53%)
MP = 206-208°

Calculated for $C_{12}H_{21}N_3OS$:

Theory: C, 56.44; H, 8.29; N, 16.45; S, 12.56

Found: C, 56.72; H, 8.33; N, 16.23; S, 12.71.

Example 12

N-[3-(1,1-Dimethylethyl)-5-isothiazolyl]-1-piperidinecarboxamide

Reactant - piperidine
Yield = 2.2 g (84%)
MP = 218-220°

Calculated for $C_{13}H_{21}N_3OS$:

Theory: C, 58.39; H, 7.92; N, 15.71; S, 11.99

Found: C, 58.62; H, 8.17; N, 15.46; S, 12.25.

Example 13

N'-[3-(1,1-Dimethylethyl)-5-isothiazolyl]-N-methyl-N-(1-methylethyl)urea

Reactant - methylisopropylamine
Yield = 1.4 g (55%)
MP = 214-216°
Calculated for $C_{12}H_{21}N_3OS$:
Theory: C, 56.44; H, 8.29; N, 16.45; S, 12.56
Found: C, 56.66; H, 8.11; N, 16.16; S, 12.34.

Example 14

N-[3-(1,1-Dimethylethyl)-5 isothiazolyl]-1-pyrrolidinecarboxamide

Reactant - pyrrolidine
Yield = 0.9 g (71%)
MP = 127-129°
Calculated for $C_{12}H_{19}N_3OS$:
Theory: C, 56.89; H, 7.56; N, 16.58; S, 12.66
Found: C, 56.65; H, 7.70; N, 16.49; S, 12.47.

## Example 15

### N'-(3-Cyclohexylmethyl-5-isothiazolyl)-N,N-dimethylurea

Reactant - dimethylamine hydrochloride
Yield = 0.75 g (56%)
MP = 184-187°

Calculated for $C_{13}H_{21}N_3OS$:

Theory: C, 58.39; H, 7.92; N, 15.71; S, 11.99

Found: C, 58.60; H, 8.18; N, 15.45; S, 11.78.

## Example 16

### N'-[3-(1-Methylcyclohexyl)-5-isothiazolyl]-N,N-dimethylurea

Reactant - dimethylamine hydrochloride
Yield = 0.8 g (61%)
MP = 240-242°

Calculated for $C_{13}H_{21}N_3OS$:

Theory: C, 58.39; H, 7.82; N, 15.71; S, 11.92

Found: C, 58.49; H, 8.05; N, 15.43; S, 11.92.

## Example 17

N'-[3-(1,1-Dimethylethyl)-5-isothiazolyl]-N-methoxy-N-methylurea

Reactant - methylmethoxyamine hydrochloride
Yield = 2.2 g (59%)
MP = 104-106°

Calculated for $C_{10}H_{17}N_3O_2S$:

Theory: C, 49.36; H, 7.04; N, 17.27
Found: C, 49.59; H, 6.83; N, 17.07.

## Example 18

N'-[3-(1-Methylpropyl)-5-isothiazolyl]-N,N-dimethylurea

Reactant - dimethylamine hydrochloride
Yield = 1.65 g (72%)
MP = 127-129°

Calculated for $C_{10}H_{17}N_3OS$

Theory: C, 52.84; H, 7.54; N, 18.48; S, 14.10
Found: C, 52.64; H, 7.30; N, 18.25; S, 14.05.

Example 19

N'-[3-(1,1-Dimethylethyl)-5-isothiazolyl]-N-ethyl-N-(1-methylethyl)urea

Reactant - ethylisopropylamine
Yield = 0.9 g (33%)
MP = 161-163°
Calculated for $C_{13}H_{23}N_3OS$:
Theory: C, 57.96; H, 8.81; N, 15.60; S, 11.90
Found: C, 57.77; H, 8.39; N, 15.40; S, 12.18.

Example 20

N'-[3-(1-Methylethyl)-5-isothiazolyl]-N-methyl-N-(1-methylethyl)urea

Reactant - methylisopropylamine
Yield = 0.8 g (47%)
MP = 190-192°
Calculated for $C_{11}H_{19}N_3OS$:
Theory: C, 54.74; H, 7.94; N, 17.41; S, 13.28
Found: C, 54.78; H, 7.76; N, 17.28; S, 13.27.

Example 21

N'-[3-(1-Methylethyl)-5-isothiazolyl]-N,N-diethylurea

Reactant - diethylamine
Yield = 0.6 g (35%)
MP = 127-129°

Calculated for $C_{11}H_{19}N_3OS$:

Theory: C, 54.74; H, 7.94; N, 17.41

Found: C, 54.84; H, 7.99; N, 17.18.

Example 22

N'-[3-(1-Methylpropyl)-5-isothiazolyl]-N-methyl-N-(1-methylethyl)urea

Reactant - methylisopropylamine

Yield = 2 g (83%)

MP = 149-151°

Calculated for $C_{12}H_{21}N_3OS$:

Theory: C, 56.44; H, 8.29; N, 16.45; S, 12.56

Found: C, 56.62; H, 8.07; N, 16.41; S, 12.28.

Example 23

N'-[3-(1-Methylethyl)-5-isothiazolyl]-N-ethyl-N-methylurea

Reactant - methylethylamine

Yield = 0.5 g (55%)

MP = 120-122°

Calculated for $C_{10}H_{17}N_3OS$:

Theory: C, 52.84; H, 7.54; N, 18.48; S, 14.10

Found: C, 53.11; H, 7.76; N, 18.22; S, 13.83.

Example 24

N'-[3-(1-Methylpropyl)-5-isothiazolyl]-N-methoxy-N-methylurea

Reactant - methoxymethylamine hydrochloride
Yield = 1.5 g (31%)
MP = 91-94°
Calculated for $C_{10}H_{17}N_3O_2S$:
Theory: C, 49.36; H, 7.04; N, 17.27; S, 13.18
Found: C, 49.21; H, 7.24; N, 17.05; S, 13.38.

Example 25

N'-[3-(1-Methylpropyl)-5-isothiazolyl]-N-ethyl-N-(1-methylethyl)urea

Reactant - ethylisopropylamine
Yield = 1 g (74%)
MP = 127-129°

Example 26

N'-[3-(1-Methylpropyl)-5-isothiazolyl]-N-ethyl-N-methylurea

Reactant - methylethylamine
Yield = 0.55 g (46%)
MP = 122-123°

Calculated for $C_{11}H_{19}N_3OS$:

Theory: C, 54.74; H, 7.94; N, 17.41; S, 13.28
Found: C, 54.92; H, 7.70; N, 17.66; S, 13.48.

Example 27

N'-[3-(1-Methylpropyl)-5-isothiazolyl]-N,N-diethylurea

Reactant - diethylamine
Yield = 1.8 g (70%)
MP = 132-134°

Calculated for $C_{12}H_{21}N_3OS$:

Theory: C, 56.44; H, 8.29; N, 16.45; S, 12.56
Found: C, 56.66; H, 8.36; N, 16.58; S, 12.40.

Example 28

N'-[3-(1-Ethyl-1-methylpropyl)-5-isothiazolyl]-N,N-dimethylurea

Reactant - dimethylamine hydrochloride
Yield = 1.6 g
Second crop = 0.4 g (62%)
MP = 206-208°/204-206°

Calculated for $C_{12}H_{21}N_3OS$:

Theory: C, 56.44; H, 8.29; N, 16.45; S, 12.56
Found: C, 56.21; H, 8.01; N, 16.40; S, 12.32.

## Example 29

N'-[3-(1-Ethyl-1-methylpropyl)-5-isothiazolyl]-N-methoxy-N-methylurea

Reactant - methoxymethylamine hydrochloride
Yield = 0.56 g (20%)
MP = 91-93°
Calculated for $C_{12}H_{21}N_3O_2S$:
Theory: C, 53.11; H, 7.80; N, 15.48; S, 11.82
Found: C, 53.29; H, 7.61; N, 15.47; S, 11.62.

## Example 30

N'-[3-(1-Ethyl-1-methylpropyl)-5-isothiazolyl]-N-methyl-N-(1-methylethyl)urea

Reactant - methylisopropylamine
Yield = 2.6 g (91%)
MP = 224-226°
Calculated for $C_{14}H_{25}N_3OS$:
Theory: C, 59.33; H, 8.89; N, 14.83; S, 11.31
Found: C, 59.28; H, 8.80; N, 14.74; S, 11.05.

## Example 31

N'-[3-(1-Ethyl-1-methylpropyl)-5-isothiazolyl]-N-ethyl-N-methylurea

Reactant - methylethylamine
Yield = 0.88 g (65%)

MP = 203-205°

Calculated for $C_{13}H_{23}N_3OS$:

Theory: C, 57.96; H, 8.61; N, 15.60; S, 11.90

Found: C, 57.78; H, 8.34; N, 15.55; S, 12.04.

Example 32

N'-[3-(1-Ethyl-1-methylpropyl)-5-isothiazolyl]-N,N-diethylurea

Reactant - diethylamine

Yield = 1.2 g (85%)

MP = 176-178°

Calculated for $C_{14}H_{25}N_3OS$:

Theory: C, 59.33; H, 8.89; N, 14.83; S, 11.31

Found: C, 59.54; H, 8.61; N, 15.07; S, 11.60.

Example 33

N'-[3-(1-Ethyl-1-methylpropyl)-5-isothiazolyl]-N-methyl-N-(1-methylpropyl)urea

Reactant - methylsecbutylamine

Yield = 1.05 g (71%)

MP = 203-205°

Calculated for $C_{15}H_{27}N_3OS$:

Theory: C, 60.57; H, 9.15; N, 14.13

Found: C, 60.71; H, 9.28; N, 14.04.

## Example 34

N'-[3-(1-Methylpropyl)-5-isothiazolyl]-N-methyl-N-(1-methylpropyl)urea

Reactant - methylsecbutylamine
Yield = 0.7 g (54%)
MP = 173-174°
Calculated for $C_{13}H_{23}N_3OS$:
Theory: C, 57.96; H, 8.61; N, 15.60
Found: C, 57.86; H, 8.34; N, 15.74.

## Example 35

N'-[3-(1,1-Dimethylethyl)-5-isothiazolyl]-N-methyl-N-(1-methylpropyl)urea

Reactant - methylsecbutylamine
Yield = 4.6 g (59%)
MP = 235-237°
Calculated for $C_{13}H_{23}N_3OS$:
Theory: C, 57.96; H, 8.61; N, 15.60
Found: C, 57.94; H, 8.38; N, 15.60.

## Example 36

N'-[3-(1-Ethylpropyl)-5-isothiazolyl]-N-methyl-N-(1-methylethyl)urea

Reactant - methylisopropylamine
Yield = 0.95 g (70%)
MP = 191-193°
Calculated for $C_{13}H_{23}N_3OS$:
Theory: C, 57.96; H, 8.61; N, 15.60
Found: C, 58.09; H, 8.42; N, 15.33.

## Example 37

N'-[3-(1-Ethylpropyl)-5-isothiazolyl]-N-ethyl-N-methylurea

Reactant - methylethylamine
MP = 167-169°
Calculated for $C_{12}H_{21}N_3OS$:
Theory: C, 56.44; H, 8.29; N, 16.45
Found: C, 56.67; H, 8.35; N, 16.64.

## Example 38

N'-[3-(2-Methylpropyl)-5-isothiazolyl]-N-methyl-N-(1-methylethyl)urea hydrate

Reactant - methylisopropylamine
Yield = 2 g (80%)

MP = 140-142°

Calculated for $C_{12}H_{21}N_3OS \cdot H_2O$:

Theory: C, 52.72; H, 8.48; N, 15.37; S, 11.73

Found: C, 52.55; H, 8.20; N, 15.14; S, 11.55.

Example 39

N'-[3-(2-Methylpropyl)-5-isothiazolyl]-N-methoxy-N-methylurea

Reactant - methoxymethylamine

Yield = 0.9 g (35%)

MP = 82-85°

Calculated for $C_{10}H_{17}N_3O_2S$:

Theory: C, 49.36; H, 7.04; N, 17.27; S, 13.18

Found: C, 49.14; H, 6.89; N, 16.97; S, 12.90.

Example 40

N'-[3-(2-Methylpropyl)-5-isothiazolyl]-N,N-dimethylurea

Reactant - dimethylamine

Yield = 1.8 g (79%)

MP = 103-106°

Calculated for $C_{10}H_{17}N_3OS$:

Theory: C, 52.84; H, 7.54; N, 18.48; S, 14.10

Found: C, 52.80; H, 7.63; N, 18.20; S, 13.82.

Example 41

N'-(3-Ethyl-5-isothiazolyl)-N-methoxy-N-methylurea

Reactant - methoxymethylamine
Yield - 0.77 g (35%)
MP = 107-109°
Calculated for $C_8H_{13}N_3O_2S$:
Theory: C, 44.63; H, 6.09; N, 19.52; S, 14.89
Found: C, 44.60; H, 5.78; N, 18.23; S, 14.63.

Example 42

N'-[3-(1,1-Dimethylethyl)-5-isothiazolyl]-N-butyl-N-methylurea

Reactant - methylbutylamine
Yield = 2.9 g (43%)
MP = 180-182°
Calculated for $C_{13}H_{23}N_3OS$:
Theory: C, 57.96; H, 8.61; N, 15.60; S, 11.90
Found: C, 57.76; H, 8.39; N, 15.46; S, 11.65.

Example 43

N'-[3-(2-Methylpropyl)-5-isothiazolyl]-N-methyl-N-(1-methylpropyl)urea

Reactant - methylsecbutylamine
Yield = 1.4 g (52%)
MP = 166-168°

Calculated for $C_{13}H_{23}N_3OS$:

Theory: C, 57.96; H, 8.61; N, 15.60; S, 11.90

Found: C, 58.14; H, 8.84; N, 15.88; S, 11.60.

Example 44

N'-[3-(1,1-Dimethylethyl)-5-isothiazolyl]-N,N-dimethylurea

Reactant - dimethylamine hydrochloride

Yield = 0.5 g (13%)

MP = 184-186°

Calculated for $C_{10}H_{16}N_3OS$:

Theory: C, 52.84; H, 7.54; N, 18.48

Found: C, 52.86; H, 7.32; N, 18.21.

Example 45

N'-[3-(1-Methylcyclopropyl)-5-isothiazolyl]-N-methoxy-N-methylurea

Reactant - methoxymethylamine

Yield = 0.5 g (20%)

MP = 135-137°

Calculated for $C_{10}H_{15}N_3O_2S$:

Theory: C, 49.77; H, 6.27; N, 17.41; S, 13.29

Found: C, 50.00; H, 5.99; N, 17.19; S, 13.02.

Example 46

N'-(3-Cyclopentyl-5-isothiazolyl)-N-methoxy-N-methylurea

Reactant - methoxymethylamine
Yield = 0.6 g (23%)
MP = <60°
Calculated for $C_{11}H_{17}N_3O_2S$
Theory: C, 51.74; H, 6.71; N, 16.43; S, 12.56
Found: C, 51.55; H, 6.73; N, 16.27; S, 12.37.

Example 47

N'-[3-(1,1-Dimethylethyl)-5-isothiazolyl]-N',N-dimethylurea

Two grams of 3-(1,1-dimethylethyl)-5-methylaminoisothiazole, 2.0 g of methyl isocyanate, and 3 drops of dibutyltin diacetate were stirred in 35 ml of benzene under reflux for about 16 hours. Ten ml of hexane were added and the precipitate formed was isolated and washed with hexane. The product weighed 2.3 g (86%) and had a MP of 217-220°.

The following elemental analysis was obtained:
Calculated for $C_{10}H_{17}N_3OS$:
Theory: C, 52.84; H, 7.54; N, 18.48
Found: C, 52.92; H, 7.71; N, 18.37.

The following example was prepared following the general procedure of Example 47, except ethyl isocyanate was used.

Example 48

N'-[3-(1,1-Dimethylethyl)-5-isothiazolyl]-N'-ethyl-N-methylurea

Yield = 2.7 g (87%)
MP = 177-180°
Calculated for $C_{11}H_{19}N_3OS$:
Theory: C, 54.74; H, 7.94; N, 17.41
Found: C, 54.80; H, 7.66; N, 17.18.

Example 49

N'-[3-(1,1-Dimethylethyl)-5-isothiazolyl]-N-methylurea

The following were mixed together and refluxed for about 64 hours: 1.56 g of 3-(1,1-dimethylethyl)-5-aminoisothiazole, 40 ml of ethyl acetate, and an excess of methyl isocyanate. The mixture was cooled and hexane was added. The precipitate which formed was filtered and dried. It weighed 0.8 g (37%) and had a MP of 239-241°.

The following elemental analysis was obtained:
Calculated for $C_9H_{15}N_3OS$:
Theory: C, 50.68; H, 7.09; N, 19.70; S, 15.03
Found: C, 50.66; H, 7.25; N, 19.49; S, 15.02.

The following example was prepared using the general procedure of Example 49, except ethyl isocyanate was used.

Example 50

N'-[3-(1,1-Dimethylethyl)-5-isothiazolyl]-N-ethylurea

Yield = 2 g (44%)
MP = 195-196°
Calculated for $C_{10}H_{17}N_3OS$:
Theory: C, 52.84; H, 7.54; N, 18.48; S, 14.10
Found: C, 52.60; H, 7.54; N, 18.58; S, 13.97.

Example 51

N'-[3-(1,1-Dimethylethyl)-5-isothiazolyl]-N'-ethyl-N-methyl-N-(1-methylethyl)urea

One gram (0.02 mole) of sodium hydride in a 50% solution of mineral oil was washed with hexane to remove the mineral oil. Thirty ml of dimethylformamide was added and the solution was cooled to 0°. To this was added 5.1 g (0.02 mole) of N'-[3-(1,1-dimethylethyl)-5-isothiazolyl]-N-(1-methylethyl)-N-methylurea and the solution was stirred for about 15 minutes at about 0-5°. A 0.32 mole portion of ethyl iodine was added and a vigorous exotherm occurred. The temperature was held below 15° by using an ice-acetone mixture. The solution was stirred for about one hour, and then allowed to warm to room temperature.

Then it was poured into 600 ml of water and stirred for about 16 hours.

The precipitate that formed was filtered and dried. It weighed 3.7 g (65%) and had a MP of 65-67°. It was recrystallized from an ethanol-water mixture and dried for about 16 hours. MP of 69-71°.

The following elemental analysis was obtained:

Calculated for $C_{14}H_{25}N_3OS$:

Theory: C, 59.36; H, 8.83; N, 14.84; S, 11.31

Found: C, 59.42; H, 8.84; N, 14.90; S, 11.54.

The following examples were prepared using the general procedure of Example 51, except the appropriately substituted halide and isothiazole were used.

Example 52

N'-[3-(1,1-Dimethylethyl)-5-isothiazolyl]-N',N-diethyl-N-methylurea

Yield = 2.2 g (55%)

MP = 61-64°

Calculated for $C_{13}H_{23}N_3OS$:

Theory: C, 57.96; H, 8.61; N, 15.60; S, 11.90

Found: C, 58.03; H, 8.32; N, 15.45; S, 11.88.

## Example 53

N'-[3-(1,1-Dimethylethyl)-5-isothiazolyl]-N',N-dimethyl-N-methoxyurea

Yield = 2.1 g (33%)
MP = 103-105°
Calculated for $C_{11}H_{19}N_3O_2S$:
Theory: C, 51.34; H, 7.44; N, 16.33; S, 12.46
Found: C, 51.62; H, 7.35; N, 16.12; S, 12.51.

## Example 54

N'-[3-(1,1-Dimethylethyl)-5-isothiazolyl]-N',N,N-triethylurea

Yield = 1.3 g (28%)
Calculated for $C_{14}H_{25}N_3OS$:
Theory: C, 59.33; H, 8.89; N, 14.83
Found: C, 59.43; H, 8.68; N, 14.61.
NMR ($CDCl_3$): δ6.4 (s, 1H); δ3.7 (q, 2H); δ3.3 (q, 4H); δ1.35 (s, 9H); δ1.3 (t, 3H); δ1.2 (t, 6H).

## Example 55

N'-[3-(1,1-Dimethylethyl)-5-isothiazolyl]-N-methoxy-N-methylurea monohydrochloride

The N'-[3-(1,1-dimethylethyl)-5-isothiazolyl]-N-methoxy-N-methylurea of Example 17 was dissolved in

ethyl ether and then hydrogen chloride was bubbled in for about 15 minutes. Afterwards, the solution was stirred rapidly for about one hour, and a white solid was precipitated. The yield was 2.5 g (47%), with a MP of 185-190° with decomposition.

The following elemental analysis was obtained:
Calculated for $C_{10}H_{17}N_3O_2S \cdot HCl$:

Theory: C, 42.93; H, 6.48; N, 15.02
Found: C, 43.20; H, 6.59; N, 14.78.

## Example 56

N'-[3-(1,1-Dimethylethyl)-5-isothiazolyl]-N-methoxy-N-methylurea

In 25 ml of pyridine, 7.8 g (0.05 mole) of 5-amino-3-(1,1-dimethylethyl)isothiazole was dissolved. Added dropwise, with stirring, was 6.8 g (0.005 mole) of N-methoxy-N-methyl carbamoyl chloride. (The chloride was prepared from phosgene and O,N-dimethylhydroxylamine in ethyl acetate, as described by CA 74:42183r and French Patent 1,584,840.)

The mixture became warm and then was cooled in an ice bath. After the carbamoyl chloride was completely added, stirring continued for about 16 hours at room temperature. Then the mixture was poured slowly into an ice-water mixture. An oily material slowly solidified after several hours. The resulting solid was broken up, then collected by suction filtration, and washed using cold water. The solid was pressed as dry as possible, then transferred to a beaker. More ice and water was added and the mixture was stirred vigorously for several hours. The creamed-colored solid formed was collected by suction filtration, washed with water, pressed, and then air-dried. The solid weighed 10.6 g (88%) and had a melting point of 104-106.

Example 57

N'-[3-(1,1-Dimethylethyl)-5-isothiazolyl]-N-methoxy-N-methylurea

The above reaction of Example 56 was repeated, except 156 g (1.0 mole) of the isothiazole, 135.8 g (1.1 mole) of the chloride, and 500 ml of pyridine were used.

Yield = 206 g (85%)
MP = 102-104°
Calculated for $C_{10}H_{17}N_3O_2S$:
Theory: C, 49.36; H, 7.04; N, 17.27; S, 13.18
Found: C, 49.53; H, 6.89; N, 17.28; S, 13.36.

## Example 58

N'-[3-(1,1-Dimethylethyl)-5-isothiazolyl]-N-methoxy-N-methylurea

To a slurry of 27.6 g of phenyl N-[3-(1,1-dimethylethyl)-5-isothiazolyl]carbamate in 160 ml of benzene, was added 7.6 g of hydroxylamine hydrochloride, and then 16.9 ml of triethylamine was added dropwise. The solution is refluxed and run to completion.

The resulting residue was dissolved in 220 ml of methanol, and then 33.1 ml of dimethyl sulfate and 36.5 ml of 10N sodium hydroxide were added at the same rate, using two addition funnels. The reaction mixture was kept below 20° with an ice bath. The reaction was complete within about 1 hour and the ice bath was removed, then the reaction mixture was stirred for an additional 1 and 1/2 hours. Afterwards, the mixture was poured onto crushed ice and ethyl acetate, and then stirred for about 1 hour. The organic and aqueous layers were separated and the aqueous layer was reextracted with two 200 ml portions of ethyl acetate. The organic layers were combined, dried over magnesium sulfate, and filtered. The yield was 24.5 g (100%). MP = 104-106°.

## Algicidal Method

Furthermore, a method is provided for controlling the growth of aquatic algae which comprises contacting the algae to be controlled or the water in which

the algae is growing with an algicidally-effective amount of a compound of the formula

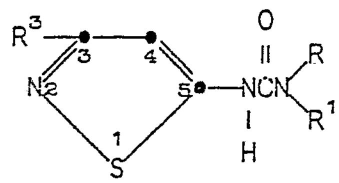

VII

wherein

R and $R^1$ are hydrogen, $C_1$-$C_3$ alkyl, n-butyl, sec-butyl, $C_2$-$C_6$ alkenyl, or $C_1$-$C_2$ alkoxy;

provided that only one of R or $R^1$ is hydrogen;

provided that only one of R or $R^1$ is $C_1$-$C_2$ alkoxy;

provided that one of R or $R^1$ is less than three carbon atoms; or

R and $R^1$ together with the nitrogen atom form a pyrrolidyl, piperidyl, morpholinyl, or piperazinyl ring;

$R^3$ is $C_2$-$C_{10}$ alkyl, benzyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, or ($C_3$-$C_6$ cycloalkyl)$C_1$-$C_4$ alkyl;

or an aquatically-acceptable acid-addition salt thereof.

Preferred compounds are those wherein:

$R^3$ is $C_3$-$C_{10}$ alkyl.

More preferred compounds of formula VII within this method are those in which:

R and $R^1$ are $C_1$-$C_3$ alkyl or $C_1$-$C_2$ alkoxy; provided that only one of R or $R^1$ is $C_1$-$C_2$ alkoxy; and that one of R or $R^1$ is less than three carbon atoms; and

$R^3$ is $C_3$-$C_6$ alkyl or $C_3$-$C_6$ cycloalkyl.

Even more preferred compounds of formula VII are where:

R is $C_1$-$C_3$ alkyl; $R^1$ is methoxy or methyl; and

$R^3$ is $C_3$-$C_4$ alkyl or cyclohexyl.

Representative compounds of formula VII are:

N'-(3-cyclohexyl-5-isothiazolyl)-N-methoxy-N-methylurea;

N'-(3-cyclohexyl-5-isothiazolyl)-N,N-dimethylurea;

N'-[3-(1,1-dimethylethyl)-5-isothiazolyl]-N-methoxy-N-methylurea;

N'-[3-(1-methylpropyl)-5-isothiazolyl]-N-methoxy-N-methylurea;

N'-[3-(1-methylethyl)-5-isothiazolyl]-N,N-dimethylurea;

N'-(3-butyl-5-isothiazolyl)-N,N-dimethylurea;

N'-[3-(1-methylethyl)-5-isothiazolyl]-N-methoxy-N-methylurea;

N'-[3-(1-methylethyl)-5-isothiazolyl]-N-methyl-N-(1-methylethyl)urea;

N'-[3-(1-methylethyl)-5-isothiazolyl]-N-ethyl-N-methylurea;

N'-[3-(1-methylpropyl)-5-isothiazolyl]-N-methyl-N-(1-methylpropyl)urea;

N'-[3-(2-methylpropyl)-5-isothiazolyl]-N-methoxy-N-methylurea;

N'-[3-(2-methylpropyl)-5-isothiazolyl]-N-methyl-N-(1-methylethyl)urea;

N'-[3-(2-methylpropyl)-5-isothiazolyl]-N,N-dimethylurea; and

N'-[3-(1-ethylpropyl)-5-isothiazolyl]-N-ethyl-N-methylurea.

The compounds of formula VII have been evaluated in a standard aquatic algicide test designed to show algicidal activity. In a primary screen, the compounds were evaluated against Chlorella vulgaris, Scenedesmus quadricauda, Anacystis nidulans, Chlamydomonas moewusii, Stichococcus bascillaris, Anabaena flos-aquae, Anabaena spiroides (blue-green), and Anabaena sp. (1551). These algae species were grown on agar slants containing artificial media (Hughes' media). The agar slants were employed in the inoculation of the test media, which itself is an aqueous Hughes' media. Five milliliters of sterile Hughes' media was used to wash the agar slants, and this was then added to 400 ml of sterile Hughes' media. Two milliliters of the inoculated media was then added to each of several 12 ml disposable vials.

The test compounds of formula VII were formulated for evaluation by dissolving 10 mg of compound in a solution of 0.5 ml of acetone and 4.5 ml of sterile 0.1% aqueous polyoxyethylene sorbitan monooleate (Tween 80). Aliquot portions (10 ml) of the formulated test compounds were then added to the vials containing the algae species Chlorella, Scenedesmus, Anacystis, and Anabaena flos-aquae, for a 10 ppm screen. Compounds

that were considered active at 10 ppm were tested using all of the algae species at concentrations of 1 ppm and 0.5 ppm. These compounds were formulated by adding 1.0 ml of acetone and 11.0 ml of Sterile 0.1% aqueous polyexyethylene sorbitan monooleate (Tween 80). Aliquots of 0.1 ml and 0.05 ml each were added to 10 ml of inoculated media to obtain 1 ppm and 0.5 ppm, respectively. Visual observations and comparisons to non-treated control vials were made 7 days after treatment. Activity ratings were made on a scale of 1 to 5 according to the following meanings:

1= no effect
2= slight effect
3= moderate effect
4= heavy effect
5= 100% control.

Table I which follows presents the algicidal activity of compounds of formula VII evaluated according to the foregoing procedures.

Table I

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 1 | 10 | 4 | 4 | 4 | | | 5 | | |
| | 1 | 3 | 5 | 5 | 2 | 4 | 5 | 5 | 4 |
| | 0.5 | 2 | 5 | 4 | 1 | 4 | 5 | 5 | 3 |
| 2 | 10 | 4 | 5 | 5 | | | 1 | | |
| | 1 | 4 | 5 | 4 | 4 | 5 | 4 | 5 | 5 |
| | 0.5 | 3 | 4 | 3 | 3 | 5 | 3 | 3 | 4 |
| 3 | 10 | 5 | 5 | 5 | | | 3 | | |
| | 1 | 3 | 5 | 3 | 4 | 5 | 5 | 5 | 5 |
| | 0.5 | 1 | 5 | 3 | 2 | 5 | 4 | 4 | 4 |
| 4 | 10 | 5 | 4 | 4 | | | 5 | | |
| | 0.5 | 5 | 4 | 5 | 5 | 4 | 4 | 4 | 4 |
| | 0.1 | 3 | 4 | 4 | 4 | 4 | 3 | 4 | 3 |
| 5 | 10 | 5 | 5 | 5 | | | 5 | | |
| | 1 | 5 | 5 | 5 | 5 | 1 | 5 | 5 | 5 |
| | 0.5 | 5 | 5 | 5 | 4 | 5 | 5 | 5 | 5 |
| 6 | 10 | 5 | 5 | 5 | | | | | |
| | 1 | 5 | 5 | 5 | 5 | 5 | 5 | 5 | 5 |
| | 0.5 | 5 | 5 | 5 | 5 | 4 | 5 | 5 | 5 |

Table I continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 7 | 10 | 5 | 5 | 5 | | | 5 | | |
| | 1 | 5 | 5 | 5 | 1 | 5 | 5 | 5 | 5 |
| | 0.5 | 4 | 5 | 4 | 1 | 4 | 3 | 5 | 5 |
| 8 | 10 | 5 | 5 | 5 | | | 5 | | |
| | 1 | 5 | 5 | 5 | 3 | 4 | 5 | 5 | 5 |
| | 0.5 | 4 | 5 | 4 | 2 | 4 | 4 | 5 | 4 |
| 9 | 10 | 5 | 5 | 5 | | | 5 | | |
| | 1 | 4 | 5 | 3 | 2 | 3 | 2 | 5 | 4 |
| | 0.5 | 1 | 1 | 1 | 1 | 2 | 1 | 5 | 2 |
| 10 | 10 | 5 | 5 | 5 | | | 5 | | |
| | 1 | 4 | 5 | 5 | 1 | 4 | 3 | 5 | 4 |
| | 0.5 | 3 | 4 | 4 | 1 | 3 | 2 | 5 | 3 |
| 11 | 10 | 5 | 5 | 5 | | | 5 | | |
| | 1 | 2 | 4 | 1 | 2 | 3 | 3 | 5 | 3 |
| | 0.5 | 1 | 2 | 1 | 1 | 2 | 1 | 5 | 2 |
| 12 | 10 | 4 | 4 | 5 | | | 5 | | |
| | 1 | 1 | 1 | 1 | 1 | 3 | 1 | 5 | 1 |
| | 0.5 | 1 | 1 | 1 | 1 | 2 | 1 | 4 | 1 |

Table I continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 13 | 10 | 5 | 5 | 5 | | | 5 | | |
| | 1 | 5 | 5 | 3 | 1 | 5 | 1 | 5 | 4 |
| | 0.5 | 4 | 5 | 2 | 1 | 4 | 1 | 4 | 3 |
| 14 | 10 | 4 | 4 | 5 | | | 5 | | |
| | 1 | 1 | 3 | 1 | 2 | 3 | 4 | 5 | 2 |
| | 0.5 | 1 | 1 | 1 | 1 | 2 | 1 | 5 | 1 |
| 15 | 10 | 5 | 5 | 5 | | | 5 | | |
| | 1 | 5 | 5 | 5 | 5 | 4 | 5 | 5 | 5 |
| | 0.5 | 5 | 5 | 5 | 5 | 4 | 3 | 5 | 4 |
| 16 | 10 | 5 | 5 | 5 | | | 5 | | |
| | 1 | 5 | 5 | 5 | 3 | 5 | 3 | 5 | 4 |
| | 0.5 | 5 | 5 | 4 | 1 | 4 | 2 | 5 | 4 |
| 17 | 10 | 5 | 5 | 5 | | | 5 | | |
| | 1 | 5 | 5 | 4 | 3 | 5 | 1 | 5 | 5 |
| | 0.5 | 4 | 5 | 1 | 2 | 5 | 1 | 5 | 4 |
| 18 | 10 | 5 | 5 | 5 | | | 5 | | |
| | 1 | 4 | 5 | 4 | 3 | 4 | 5 | 4 | 5 |
| | 0.5 | 3 | 5 | 3 | 3 | 4 | 3 | 4 | 4 |
| 19 | 10 | 4 | 4 | 5 | | | 4 | | |
| | 1 | 1 | 1 | 1 | 1 | 3 | 1 | 3 | 1 |
| | 0.5 | 1 | 1 | 1 | 1 | 1 | 1 | 2 | 1 |

## Table I continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 20 | 10 | 5 | 5 | 5 | | | 5 | | |
| | 1 | 3 | 4 | 5 | 1 | 4 | 3 | 5 | 4 |
| | 0.5 | 1 | 3 | 4 | 1 | 3 | 2 | 5 | 2 |
| 22 | 10 | 4 | 4 | 5 | | | 4 | | |
| | 1 | 4 | 4 | 4 | 3 | 4 | 3 | 4 | 4 |
| | 0.5 | 3 | 3 | 4 | 3 | 4 | 3 | 4 | 3 |
| 23 | 10 | 5 | 5 | 5 | | | 5 | | |
| | 1 | 3 | 4 | 5 | 1 | 4 | 4 | 5 | 4 |
| | 0.5 | 2 | 4 | 5 | 1 | 3 | 4 | 5 | 3 |
| 24 | 10 | 4 | 4 | 5 | | | 4 | | |
| | 1 | 5 | 4 | 5 | 4 | 4 | 5 | 4 | 4 |
| | 0.5 | 4 | 4 | 4 | 4 | 4 | 4 | 4 | 4 |
| 25 | 10 | 4 | 4 | 5 | | | 4 | | |
| | 1 | 1 | 3 | 3 | 1 | 3 | 3 | 3 | 3 |
| | 0.5 | 1 | 3 | 3 | 1 | 3 | 1 | 4 | 2 |
| 26 | 10 | 5 | 5 | 5 | | | 5 | | |
| | 1 | 4 | 5 | 5 | 1 | 4 | 4 | 5 | 4 |
| | 0.5 | 1 | 3 | 4 | 1 | 3 | 3 | 5 | 3 |

Table I continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 27 | 10 | 4 | 4 | 4 | | | 4 | | |
| | 1 | 4 | 4 | 4 | 4 | 4 | 3 | 4 | 4 |
| | 0.5 | 1 | 3 | 3 | 3 | 4 | 2 | 4 | 2 |
| 28 | 10 | 4 | 4 | 5 | | | 5 | | |
| 30 | 10 | 4 | 4 | 4 | | | 3 | | |
| 31 | 10 | 4 | 4 | 4 | | | 4 | | |
| 32 | 10 | 5 | 5 | 5 | | | 5 | | |
| | 1 | 1 | 2 | 3 | 1 | 4 | 1 | 5 | 1 |
| | 0.5 | 1 | 1 | 1 | 1 | 2 | 1 | 1 | 1 |
| 33 | 10 | 3 | 4 | 3 | | | 3 | | |
| 34 | 10 | 5 | 5 | 5 | | | 5 | | |
| | 1 | 4 | 5 | 5 | 2 | 4 | 4 | 5 | 5 |
| | 0.5 | 1 | 2 | 3 | 1 | 2 | 1 | 5 | 3 |
| 35 | 10 | 5 | 5 | 5 | | | 5 | | |
| | 1 | 4 | 5 | 3 | 4 | 5 | 2 | 5 | 4 |
| | 0.5 | 4 | 4 | 1 | 4 | 4 | 1 | 5 | 3 |
| 36 | 10 | 5 | 5 | 5 | | | 5 | | |
| | 1 | 1 | 1 | 4 | 1 | 3 | 2 | 5 | 3 |
| | 0.5 | 1 | 1 | 3 | 1 | 3 | 1 | 5 | 1 |

Table I continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 37 | 10 | 5 | 5 | 5 | | | 5 | | |
| | 1 | 4 | 5 | 5 | 1 | 3 | 3 | 5 | 4 |
| | 0.5 | 1 | 2 | 4 | 1 | 2 | 1 | 5 | 3 |
| 38 | 10 | 5 | 5 | 5 | | | 5 | | |
| | 1 | 4 | 4 | 4 | 4 | 4 | 4 | 4 | 4 |
| | 0.5 | 4 | 4 | 4 | 4 | 4 | 4 | 4 | 1 |
| 39 | 10 | 5 | 5 | 5 | | | 5 | | |
| | 1 | 4 | 5 | 5 | 4 | 4 | 4 | 5 | 4 |
| | 0.5 | 4 | 4 | 5 | 3 | 4 | 4 | 5 | 3 |
| 40 | 10 | 5 | 5 | 5 | | | 5 | | |
| | 1 | 4 | 5 | 5 | 4 | 4 | 5 | 4 | 4 |
| | 0.5 | 4 | 5 | 4 | 4 | 4 | 4 | 4 | 4 |
| 41 | 10 | 5 | 5 | 5 | | | 5 | | |
| | 1 | 3 | 4 | 4 | | | 4 | | |
| | 0.5 | 3 | 2 | 4 | | | 4 | | |
| 42 | 10 | 5 | 5 | 5 | | | 5 | | |
| | 1 | | 3 | 1 | 2 | 5 | 4 | 5 | 4 |
| | 0.5 | | 1 | 1 | 1 | 4 | 1 | 5 | 4 |

Table I continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 44 | 10 | 5 | 5 | 5 | | | 5 | | |
| | 1 | 5 | 5 | 5 | 3 | 4 | 4 | 5 | 4 |
| | 0.5 | 4 | 5 | 3 | 2 | 3 | 3 | 5 | 4 |
| 49 | 10 | 5 | 5 | 5 | | | 5 | | |
| | 1 | 1 | 5 | 1 | 1 | 2 | 1 | 5 | 2 |
| | 0.5 | 1 | 3 | 1 | 1 | 1 | 1 | 5 | 1 |
| 50 | 1 | 1 | 1 | 2 | 1 | 1 | 1 | 3 | 2 |
| | 0.5 | 1 | 1 | 1 | 1 | 1 | 1 | 1 | 1 |
| 55 | 10 | 5 | 5 | 5 | | | 5 | | |
| | 1 | 4 | 5 | 3 | 3 | 5 | 3 | 4 | 5 |
| | 0.5 | 3 | 5 | 1 | 2 | 5 | 1 | 3 | 5 |

Several of the compounds provided by formula VII were evaluated in a secondary aquatic algicide screen. The test was carried out as described above, except that lower levels of compound were employed. The results of the secondary screen are reported in Table II which follows.

## Table II

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 5 | .05 | 3 | 4 | 1 | 1 | 3 | 1 | 5 | 1 |
| | .1 | 4 | 5 | 1 | 1 | 4 | 1 | 5 | 3 |
| | .5 | 5 | 5 | 4 | 4 | 4 | 5 | 5 | 4 |
| 6 | .05 | 3 | 4 | 1 | 1 | 4 | 1 | 5 | 2 |
| | .1 | 3 | 5 | 1 | 1 | 4 | 1 | 5 | 3 |
| | .5 | 5 | 5 | 5 | 5 | 4 | 5 | 4 | 4 |
| 7 | .05 | 1 | 1 | 1 | 1 | 1 | 1 | 5 | 1 |
| | .1 | 2 | 3 | 1 | 1 | 1 | 1 | 5 | 3 |
| | .5 | 4 | 5 | 3 | 1 | 5 | 4 | 5 | 5 |
| 8 | .05 | 1 | 1 | 1 | 1 | 4 | 1 | 5 | 1 |
| | .1 | 3 | 3 | 1 | 1 | 4 | 1 | 5 | 3 |
| | .5 | 4 | 5 | 4 | 1 | 5 | 4 | 5 | 5 |
| 10 | .05 | 3 | 4 | 2 | 3 | 1 | 1 | 2 | 3 |
| | .1 | 3 | 4 | 4 | 4 | 3 | 1 | 2 | 2 |
| | .5 | 4 | 4 | 4 | 4 | 4 | 4 | 4 | 3 |
| 13 | .05 | 1 | 1 | 1 | 1 | 1 | 1 | 1 | 1 |
| | .1 | 1 | 2 | 1 | 1 | 2 | 1 | 5 | 1 |
| | .5 | 3 | 5 | 2 | 1 | 5 | 1 | 5 | 2 |

Table II continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 15 | .05 | 1 | 1 | 1 | 1 | 1 | 1 | 5 | 1 |
| | .1 | 4 | 4 | 1 | 1 | 4 | 1 | 5 | 2 |
| | .5 | 5 | 5 | 5 | 4 | 5 | 4 | 5 | 5 |
| 16 | .05 | 2 | 1 | 1 | 1 | 4 | 1 | 1 | 1 |
| | .1 | 4 | 4 | 1 | 1 | 4 | 1 | 1 | 3 |
| | .5 | 5 | 5 | 2 | 2 | 5 | 3 | 4 | 4 |
| 17 | .05 | 1 | 1 | 1 | 1 | 4 | 1 | 1 | 1 |
| | .1 | 3 | 4 | 1 | 1 | 4 | 1 | 4 | 1 |
| | .5 | 4 | 5 | 1 | 1 | 5 | 1 | 5 | 4 |
| 20 | .05 | 1 | 3 | 1 | 2 | 1 | 1 | 1 | 1 |
| | .1 | 1 | 4 | 3 | 3 | 1 | 1 | 2 | 1 |
| | .5 | 4 | 4 | 4 | 4 | 4 | 3 | 3 | 2 |
| 23 | .05 | 1 | 3 | 2 | 2 | 1 | 1 | 2 | 1 |
| | .1 | 1 | 4 | 3 | 3 | 1 | 1 | 3 | 2 |
| | .5 | 4 | 4 | 4 | 4 | 4 | 4 | 3 | 3 |
| 24 | .05 | 1 | 1 | 1 | 3 | 3 | 1 | 5 | 1 |
| | .1 | 1 | 3 | 1 | 2 | 4 | 1 | 5 | 3 |
| | .5 | 3 | 5 | 2 | 3 | 5 | 1 | 5 | 4 |

Table II continued

Aquatic Algicide Activity

Control Ratings

| Compound of Example No. | Concentration ppm | Chlorella vulgaris | Scenedesmus quadricauda | Anacystis nidulans | Chlamydomonas | Stichococcus | Anabaena flos | Anabaena blue green | Anabaena |
|---|---|---|---|---|---|---|---|---|---|
| 26 | .05 | 1 | 4 | 4 | 3 | 1 | 2 | 3 | 2 |
| | .1 | 3 | 4 | 4 | 4 | 4 | 3 | 3 | 3 |
| | .5 | 4 | 4 | 4 | 4 | 4 | 4 | 3 | 3 |
| 34 | .05 | 1 | 4 | 3 | 3 | 1 | 1 | 2 | 1 |
| | .1 | 3 | 4 | 4 | 4 | 2 | 2 | 3 | 1 |
| | .5 | 4 | 4 | 4 | 4 | 4 | 4 | 4 | 3 |
| 35 | .05 | 1 | 1 | 1 | 1 | 1 | 1 | 5 | 1 |
| | .1 | 1 | 3 | 1 | 2 | 3 | 1 | 5 | 2 |
| | .5 | 4 | 5 | 1 | 2 | 4 | 1 | 2 | 4 |
| 37 | .05 | 1 | 1 | 4 | 1 | 1 | 1 | 1 | 1 |
| | .1 | 1 | 4 | 2 | 3 | 1 | 1 | 2 | 1 |
| | .5 | 3 | 4 | 3 | 4 | 3 | 3 | 3 | 2 |
| 39 | .05 | 3 | 4 | 4 | 4 | 2 | 2 | 2 | 3 |
| | .1 | 3 | 4 | 4 | 4 | 4 | 2 | 3 | 3 |
| | .5 | 4 | 4 | 4 | 4 | 4 | 4 | 3 | 2 |
| 44 | .05 | 1 | 1 | 1 | 1 | 1 | 1 | 1 | 1 |
| | .1 | 1 | 3 | 1 | 1 | 1 | 1 | 1 | 1 |
| | .5 | 1 | 5 | 1 | 1 | 1 | 1 | 4 | 4 |

The compounds of formula VII have also been evaluated as an algicide on planktonic algae under field conditions in 16 liter buckets maintained outdoors and containing a layer of earth at the bottem. The buckets were filled with well-water, and then innoculated with green algae from slants (Scenedsmus). The algae bloom occurred in an untreated control in 24 hours.

The compounds of formula VII were formulated as a wettable powder, described in the Formulations section hereinafter, or as a technical solution of 1:9 ratio by volume of acetone:0.1% by volume of Tween 80. The appropriate volume of water was added to the formulation to obtain the solutions containing 0.1 to 1.0 ppm of the active compound of formula VII. The formulation was added to the bucket 24 hours after innoculation. The activity was determined by visual observation based upon non-treated controls. Plant injury ratings were made visually in a scale of 0-10 with 0 being no injury and 10 being plant death. The injury rating was multiplied by 10 to obtain a percent inhibition. The results of these tests are reported in Table III.

Table III

ALGAE CONTROL-FIELD DATA

PERCENT INHIBITION-DAYS AFTER TREATMENT

| Compound of Example No. | Concentration ppm | DAY 4 | DAY 7 |
|---|---|---|---|
| 6 | 0.1 | 40.0 | 96.7 |
| | 0.3 | 40.0 | 100.0 |
| | 1.0 | 36.7 | 100.0 |
| 8 | 0.1 | 33.3 | 50.0 |
| | 0.3 | 35.0 | 70.0 |
| | 1.0 | 31.7 | 93.3 |
| 15 | 0.1 | 36.7 | 73.3 |
| | 0.3 | 33.3 | 80.0 |
| | 1.0 | 33.3 | 86.7 |
| 16 | 0.1 | 36.7 | 60.0 |
| | 0.3 | 43.3 | 70.0 |
| | 1.0 | 40.0 | 73.3 |
| 17 | 0.1 | 36.7 | 66.7 |
| | 0.3 | 36.7 | 90.0 |
| | 1.0 | 33.3 | 90.0 |
| Control | 0 | 10.0 | 0.0 |

Terrestrial Herbicidal Method

A method of controlling the growth of unwanted terrestrial vegetation comprises contacting the vegetation to be controlled or the soil in which the vegetation is growing with a herbicidally-effective amount of a compound of the formula

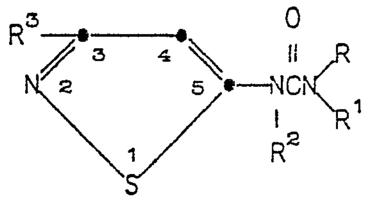

IX

wherein

R and $R^1$ are hydrogen, $C_1$-$C_3$ alkyl, n-butyl, sec -butyl, $C_2$-$C_6$ alkenyl, or $C_1$-$C_2$ alkoxy;

provided that only one of R or $R^1$ is hydrogen;

provided that only one of R or $R^1$ is $C_1$-$C_2$ alkoxy;

provided that one of R or $R^1$ is less than three carbon atoms; or

R and $R^1$ together with the nitrogen atom form a pyrrolidyl, piperidyl, morpholinyl, or piperazinyl ring;

$R^2$ is hydrogen or $C_1$-$C_2$ alkyl; and

$R^3$ is $C_2$-$C_{10}$ alkyl, benzyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, or ($C_3$-$C_6$ cycloalkyl)$C_1$-$C_4$ alkyl;

or an agriculturally-acceptable acid-addition salt thereof.

Preferred compounds of formula IX within this method are those in which:

R and $R^1$ are alkyl or $C_1$-$C_2$ alkoxy;
$R^2$ is hydrogen and
$R^3$ is $C_2$-$C_6$ alkyl.

Even more preferred compounds of formula IX are where:

R is $C_1$-$C_3$ alkyl; $R^1$ is methoxy or methyl; and

$R^3$ is $C_2$-$C_4$ alkyl.

Most preferred compounds of formula IX are:

N'-[3-(1,1-dimethylethyl)-5-isothiazolyl]-N-methoxy-N-methylurea;

N'-[3-(1,1-dimethylethyl)-5-isothiazolyl]-N,N-dimethylurea;

N'-(3-ethyl-5-isothiazolyl)-N-methoxy-N-methylurea;

N'-[3-(1-methylethyl)-5-isothiazolyl]-N-methoxy-N-methylurea;

N'-[3-(1-methylethyl)-5-isothiazolyl]-N-methyl-N-(1-methylethyl)urea;

N'-[3-(1-methylethyl)-5-isothiazolyl]-N-ethyl-N-methylurea;

N'-[3-(1-methylpropyl)-5-isothiazolyl]-N-methoxy-N-methylurea;

N'-[3-(2-methylpropyl)-5-isothiazolyl]-N-methoxy-N-methylurea;

N'-[3-(2-methylpropyl)-5-isothiazolyl]-N-methyl-N-(1-methylethyl)urea;

N-[3-(2-methylpropyl)-5-isothiazolyl]-N,N-dimethylurea; and

N'-[3-(1,1-dimethylethyl)-5-isothiazolyl]-N-methoxy-N-methylurea monohydrochloride.

The isothiazolylureas provided by formula IX exhibit both terrestrial and aquatic herbicidal activity and accordingly are useful in the control and elimination of unwanted vegetative growth.

The herbicides of formula IX are effective terrestrially in both preemergent and postemergent control of a wide variety of grasses and broadleaf weeds. Commonly encountered unwanted terrestrial vegetation which is subject to control with the herbicidal ureas of formula IX include:

Wild Oats (*Avena fatua*)
Catchweed Bedstraw (*Galium aparine*)
Scentless Mayweed (*Matricaria inodora*)
Ladysthumb (*Polygonum persicaria*)
Common Chickweed (*Stellaria media*)
Ivyleaf Speedwell (*Veronica hederaefolia*)
Blackgrass (*Alopecurus myosuroides*)
Chrysanthemum (*Chrysanthemum spp.*)
Common Purslane (*Portulaca oleracea*)
Sida (*Sida spp.*)
Bristly Starbur (*Acanthospermum hispidum*)
Goosegrass (*Eleusine indica*)
Smooth Pigweed (*Amaranthus hybridus*)
Alexandergrass (*Brachiaria plantaginea*)
Tall Morningglory (*Ipomoea purpurea*)
Common Lambsquarters (*Chenopodium album*)
Green Smartweed (*Polygonum scabrum*)
Green Foxtail (*Setaria viridis*)

Redroot Pigweed (*Amaranthus retroflexus*)
Wild Buckwheat (*Polygonum convolvulus*)
Brazil Calalilly (*Richardia brasiliensis*)
Natal Grass (*Rhynchelytrum roseum*)
Ryegrass (*Lolium rigidum*)
Kapeweed (*Cryptostemma calendula*)
Purple Loosestrife (*Lythrum salicaria*)
Wild Radish (*Raphanus raphanistrum*)
Wireweed (*Polygonum aviculare*)
Henbit (*Lamium amplexicaule*)
Wild Mustard (*Brassica kaber*)
Barnyard Grass (*Echinochloa crus-galli*)
Foxtail Millet (*Setaria italica*)
Velvetleaf (*Abutilon theophrasti*)
Indian Mustard (*Brassica juncea*)
Birdseye Speedwell (*Veronica persica*)
Canada Thistle (*Cirsium arvense*)
Wild Chamomile (*Matricaria cham@milla*)
Annual Bluegrass (*Poa annua*)
Buttercup (*Ranunculus spp.*)
Field Speedwell (*Veronica agrestis*)
Field Violet (*Viola arvensis*)
Field Pennycress (*Thlaspi arvense*)
Wild Violet (*Viola tricolor*)
Shirley Poppy (*Papaver rhoeas*)
Field Poppy (*Papaver dubium*)
Foolsparsley (*Aethusa cynapium*)
Field Chickweed (*Cerastium arvense*)
Southern Sandbur (*Cenchrus echinatus*)
Large Crabgrass (*Digitaria sanguinalis*)
Cheat (*Bromus secalinus*)

Morningglory (*Ipomea spp.*)
Common Ragweed (*Ambrosia artemisiifolia*)
Common Milkweed (*Asclepias syriaca*)
Giant Foxtail (*Setaria faberi*)
Common Cocklebur (*Xanthium pensylvanicum*)
Spurred Anoda (*Anoda cristata*)
Sicklepod (*Cassia obtusifolia*)
Yellow Nutsedge (*Cyperus esculentus*)
Jimsonweed (*Datura stramonium*)
Prickly Sida (*Sida spinosa*)
Corn Gromwell (*Lithospermum arvense*)
Yellow Foxtail (*Setaria glauca*)
Tansymustard (*Descurainia pinnata*)
Pepperweed (*Lepidium spp.*)
Bromegrass (*Bromus spp.*)
Garden Spurge (*Euphorbia hirta*)
Crowfootgrass (*Dactyloctenium aegyptium*)
Florida Beggarweed (*Desmodium tortuosum*)
Spotted Spurge (*Euphorbia maculata*)
Smallflower Morningglory (*Jacquemontia tamnifolia*)
Browntop Millet (*Panicum ramosum*)
Coast Fiddleneck (*Amsinckia intermedia*)
Wild Turnip (*Brassica campestris*)
Black Mustard (*Brassica nigra*)
Shepherdspurse (*Capsella bursa-pastoris*)
Italian Ryegrass (*Lolium multiflorum*)
London Rocket (*Sisymbrium irio*)
Redmaids Rockpurslane (*Calandrinia caulescens*)
Common Groundsel (*Senecio vulgaris*)
Ivyleaf Morningglory (*Ipomoea hederacea*)
Fall Panicum (*Panicum dichotomiflorun*)

Powell Amaranth (*Amaranthus powellii*)
Texas Panicum (*Panicum texanum*)
Hemp Sesbania (*Sesbania exaltata*)
Annual Sowthistle (*Sonchus oleraceus*)
Field Bindweed (*Convolvulus arvensis*)
Erect Knotweed (*Polygonum erectum*)
Venice Mallow (*Hibiscus trionum*)
Zinnia (*Zinnia elegens*)
Nightshade (*Solanum spp.*)

The present compounds of formula IX have also been found safe on a wide variety of desirable plant species, thereby exhibiting their unique selective capacity. Representative examples of relatively tolerant plant species, depending on the concentration of active compound of formula IX employed, include the following:

Corn (*Zea mays*)
Wheat (*Triticum aestivum*)
Soybean (*Glycine max*)
Rice (*Oryza sativa*)
Barley (*Hordeum vulgare*)
Cotton (*Gossypium hirsutum*)
Sorghum (*Sorghum vulgare v. saccharatum*)
Sugarcane (*Saccharum officinarum*)
Peanut (*Arachis hypogaea*)
Alfalfa (*Medicago sativa*)
Cucumber (*Cucumis sativus*)
Tomato (*Lycopersicon esculentum*)
Sugar beets (*Beta vulgaris*)

The compounds of formula IX have been found to be particularly useful for the control of weeds in cereal grains, such as wheat, when applied either preemergence or postemergence to the locus.

A test used to evaluate herbicidal efficacy was conducted at a compound concentration of 15 pounds per acre (16.8 kilograms per hectare). In this test a standard sand:soil mixture (1:1) was sterilized and added to separate containers and tomato, large crabgrass, and pigweed seeds were planted by row.

The test compounds of formula IX were formulated for application by dissolving the compound into a solvent, containing acetone, ethanol, and a blend of anionic and nonionic surfactants. The solvent/compound solution was diluted with deionized water and applied postemergence to some planted containers and preemergence to others. Postemergent treatment was made 11 to 13 days after planting, while preemergent treatment was made one day after planting.

Following treatment the containers were moved to the greenhouse and watered as necessary. Observations were made 10 to 13 days after treatment using untreated control plants as standards. The degree of herbicidal activity was determined by rating the treated plants on a scale of 1 to 5. On this scale "1" indicates no injury, "2" is slight injury, "3" is moderate injury, "4" is severe injury, and "5" indicates death to the plant or no seedling emergence. Also, the various types of injury of each test species were coded as follows:

A = abscission of leaves
B = burned
C = chlorosis
D = death
E = epinasty
F = formation effects
G = dark green
I = increased plant growth
L = local necrosis
N = no germination
P = purple pigmentation
R = reduced germination
S = stunting
U = unclassified injury

Table IV which follows presents the terrestrial herbicidal activity of the compounds of formula IX at 15 pounds per acre (16.8 kilograms per hectare).

Table IV
Terrestrial Herbicidal Activity

Plant Species

| Compound of Example No. | Pre-emergence | | | Post-emergence | | |
|---|---|---|---|---|---|---|
| | Tomato | Large Crab-grass | Pig-weed | Tomato | Large Crab-grass | Pig-weed |
| 1 | 3RS | 4RS | 4RS | 5D | 5D | 5D |
| 2 | 5D | 4BS | 5D | 5D | 5D | 5D |
| 3 | 5D | 5D | 5D | 5D | 5D | 5D |
| 4 | 2S | 2RS | 2RS | 5D | 5D | 5D |
| 7 | 2S | 3RS | 3RS | 5D | 5D | 5D |
| 9 | 1 | 1 | 3RS | 2CBS | 2CBS | 1 |
| 12 | 1 | 1 | 1 | 1 | 1 | 1 |
| 13 | 4RS | 4RS | 4RS | 5D | 5D | 5D |
| 18 | 4RS | 4BS | 5N | 4BS | 5D | 5D |
| 22 | 2S | 4RS | 4RS | 5D | 2BS | 1 |
| 24 | 3S | 4BS | 4BS | 5D | 5D | 5D |
| 27 | 1 | 2S | 1 | 2CS | 3BS | 1 |
| 28 | 1 | 1 | 1 | 5D | 4BS | 4BS |
| 30 | 1 | 1 | 1 | 1 | 1 | 1 |
| 34 | 1 | 1 | 1 | 4BS | 2BS | 4BS |
| 35 | 4RS | 4RS | 4RS | 5D | 4BS | 4BS |
| 40 | 3CS | 4RS | 4RS | 5D | 5D | 5D |
| 42 | 1 | 2S | 2RS | 5D | 2BS | 5D |
| 47 | 4RS | 4RS | 4RS | 5D | 5D | 5D |
| 48 | 2RS | 1 | 1 | 4CS | 3BS | 4BS |
| 50 | 1 | 1 | 2BS | 1 | 3BS | 5D |
| 51 | 1 | 3RS | 2RS | 5D | 4BS | 5D |
| 52 | 3RS | 4RS | 4RS | 5D | 5D | 5D |
| 53 | 1 | 2S | 2S | 5D | 5D | 5D |
| 55 | 4BS | 4BS | 5D | 5D | 5D | 5D |

The herbicidal activity of some of the compounds of formula IX was further evaluated at various application rates in a multiple species greenhouse test. Several additional weed and crop species were utilized to determine the herbicidal activity and selectivity of the test compounds. Lower concentrations of the test compounds were obtained by serial dilution of the above described formulation with a mixture of the surfactant and deionized water. In Tables V and VI the compounds were evaluated according to the general procedure outlined above.

## Table V

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 1 | 1 | 1 | 1 | 1 | 1 | 1 | 1 | 2 | 2 | 2 | 2 | 4 | 2 | 2 | 5 | 4 | 3 | 4 | 1 | 3 | 4 | 3 | 2 | | 3 | 4 | 5 | 4 | 2 | 4 | 5 | 5 |
| | 2 | 1 | 3 | 3 | 3 | 4 | 4 | 2 | 5 | 5 | 5 | 5 | 4 | 4 | 5 | 4 | 3 | 5 | 3 | 4 | 5 | 5 | 4 | | 3 | 4 | 5 | 5 | 2 | 5 | 5 | 4 |
| | 4 | 4 | 5 | 5 | 4 | 5 | 4 | 3 | 5 | 4 | 4 | 5 | 4 | 4 | 5 | 4 | 4 | 4 | 5 | 4 | 5 | 4 | 3 | | 4 | 4 | 5 | 4 | 3 | 4 | 5 | 5 |
| | 8 | | | | | | | | | 4 | 4 | | 4 | 3 | 5 | 4 | 4 | 5 | | 4 | 5 | 5 | 4 | | 5 | 5 | 5 | 5 | 3 | 5 | 5 | 5 |
| 2 | 1 | 3 | 5 | 4 | 4 | 5 | 4 | 2 | 5 | 4 | 4 | 5 | 5 | 5 | 5 | 5 | 4 | 5 | 5 | 5 | 5 | 4 | 4 | | 4 | 5 | 5 | 4 | 4 | 5 | 5 | 5 |
| | 2 | 3 | 4 | 5 | 4 | 5 | 5 | 4 | 5 | 5 | 5 | 5 | 5 | 5 | 5 | 5 | 4 | 5 | 5 | 5 | 5 | 4 | 4 | | 5 | 5 | 5 | 5 | 2 | 5 | 5 | 5 |
| | 4 | 5 | 5 | 5 | 5 | 5 | 5 | 5 | 5 | 5 | 5 | 5 | 5 | 5 | 5 | 5 | 5 | 5 | 5 | 5 | 5 | 5 | 5 | | 5 | 5 | 5 | 5 | 5 | 5 | 5 | 5 |
| | 8 | | | | | | | | | 5 | 4 | | 5 | 5 | 5 | 4 | 5 | 5 | | 5 | 5 | 5 | 4 | | 5 | 5 | 5 | 5 | 5 | 5 | 5 | 5 |
| 3 | 1 | 3 | 3 | 4 | 2 | 4 | 4 | 2 | 5 | 4 | 4 | 5 | 4 | 5 | 4 | 4 | 2 | 2 | 5 | 5 | 4 | 4 | 3 | | 4 | 4 | 4 | 4 | 2 | 5 | 5 | 5 |
| | 2 | 5 | 5 | 4 | 4 | 5 | 5 | 4 | 5 | 5 | 5 | 5 | 4 | 5 | 5 | 5 | 4 | 5 | 5 | 5 | 5 | 4 | 4 | | 4 | 5 | 5 | 4 | 3 | 5 | 5 | 5 |
| | 4 | 4 | 5 | 5 | 4 | 5 | 5 | 4 | 5 | 5 | 4 | 5 | 5 | 5 | 5 | 5 | 4 | 5 | 5 | 5 | 5 | 3 | 5 | | 5 | 5 | 5 | 5 | 4 | 5 | 5 | 5 |
| | 8 | | | | | | | | | 4 | 4 | | 5 | 5 | 5 | 5 | 4 | 4 | | 5 | 5 | 3 | 4 | | 5 | 5 | 5 | 4 | 4 | 5 | 5 | 5 |
| 4 | 1 | 1 | 3 | 3 | 2 | 4 | 4 | 2 | 5 | 4 | 2 | 5 | 3 | 2 | 3 | 3 | 1 | 2 | 3 | 2 | 3 | 5 | 4 | | 4 | 4 | 5 | 4 | 4 | 5 | 5 | 5 |
| | 2 | 2 | 4 | 4 | 2 | 3 | 4 | 2 | 5 | 4 | 2 | 5 | 4 | 5 | 5 | 4 | 2 | 4 | 5 | 4 | 5 | 5 | 4 | | 4 | 4 | 5 | 5 | 5 | 5 | 5 | 5 |
| | 4 | 2 | 4 | 3 | 3 | 3 | 4 | 2 | 5 | 5 | 4 | 5 | 4 | 5 | 5 | 5 | 4 | 5 | 5 | 4 | 5 | 5 | 4 | | 5 | 5 | 5 | 5 | 4 | 5 | 5 | 5 |
| | 8 | | | | | | | | | 5 | 4 | | 4 | 4 | 5 | 4 | 4 | 4 | | 4 | 5 | 5 | 5 | | 5 | 5 | 5 | 5 | 5 | 5 | 5 | 5 |

Table V cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 5 | 1 | 2 | 2 | 3 | 1 | 2 | 4 | 3 | 3 | 3 | 3 | 5 | 5 | 4 | 4 | 5 | 2 | 4 | 4 | 4 | 3 | | | 2 | 3 | | 3 | 4 | | 4 | 3 | 5 |
| | 2 | 3 | 5 | 3 | 2 | 3 | 4 | 2 | 5 | 4 | 4 | 5 | 5 | 4 | 3 | 5 | 3 | 4 | 5 | 5 | 4 | | | 2 | 3 | | 4 | 3 | | 4 | 3 | 3 |
| | 4 | 4 | 4 | 3 | 2 | 2 | 4 | 3 | 5 | 3 | 4 | 5 | 5 | 3 | 4 | 5 | 3 | 5 | 4 | 4 | 5 | | | 3 | | | 4 | 4 | | 4 | 3 | 4 |
| | 8 | 5 | | | | | | | | | | | 5 | | 5 | 5 | | 4 | | 4 | 5 | | | 3 | 4 | | 4 | 4 | | 4 | 4 | 4 |
| 6 | 1 | 1 | 1 | 1 | 1 | 1 | 3 | 2 | 1 | 1 | 1 | 3 | 4 | 3 | 5 | 4 | 1 | 4 | 3 | 1 | 2 | | | 3 | 5 | | 4 | 4 | | 5 | 5 | 5 |
| | 2 | 1 | 1 | 2 | 1 | 2 | 2 | 2 | 3 | 5 | 3 | 5 | 4 | 5 | 5 | 1 | 5 | 3 | 2 | 4 | 4 | | | 4 | 5 | | 5 | 4 | | 5 | 4 | 4 |
| | 8 | 4 | | | | | | | | | | | 5 | | 5 | 5 | | 5 | | 5 | 5 | | | 5 | 5 | | 5 | 5 | | 5 | 5 | 5 |
| 7 | 1 | 2 | 2 | 3 | 2 | 4 | 4 | 3 | 4 | 4 | 4 | 4 | 5 | 4 | 4 | 4 | 3 | 5 | 1 | 3 | 3 | | | 3 | 4 | | 5 | 4 | | 5 | 4 | 5 |
| | 2 | 3 | 2 | 3 | 2 | 5 | 5 | 3 | 5 | 5 | 3 | 5 | 5 | 4 | 4 | 4 | 4 | 5 | 4 | 4 | 5 | | | 2 | 5 | | 5 | 4 | | 5 | 4 | 5 |
| | 4 | 2 | 3 | 2 | 3 | 5 | 5 | 3 | 5 | 2 | 3 | 5 | 4 | 4 | 5 | 4 | 4 | 5 | 3 | 4 | 5 | | | 4 | 4 | | 4 | 4 | | 5 | 5 | 5 |
| | 8 | 4 | | | | | | | | | | | 5 | | 5 | 5 | | 5 | | 5 | 5 | | | 4 | 5 | | 5 | 5 | | 5 | 5 | 5 |
| 8 | 1 | 1 | 2 | 4 | 4 | 5 | 4 | 2 | 5 | 5 | 4 | 5 | 5 | 5 | 4 | 4 | 3 | 5 | 2 | 5 | 5 | | | 3 | 4 | | 4 | 4 | | 5 | 5 | 5 |
| | 2 | 3 | 5 | 4 | 4 | 5 | 5 | 4 | 5 | 5 | 4 | 5 | 5 | 5 | 5 | 4 | 5 | 5 | 5 | 5 | 5 | | | 4 | 5 | | 5 | 5 | | 5 | 5 | 5 |
| | 4 | 4 | 5 | 4 | 4 | 5 | 5 | 4 | 5 | 5 | 4 | 5 | 5 | 5 | 5 | 5 | 5 | 5 | 5 | 5 | 5 | | | 5 | 5 | | 5 | 5 | | 5 | 5 | 5 |
| | 8 | 5 | | | | | | | | | | | 5 | | 5 | 5 | | 5 | | 5 | 5 | | | 3 | 5 | | 5 | 5 | | 5 | 5 | 5 |
| 9 | 1 | 1 | 1 | 2 | 1 | 1 | 4 | 1 | 2 | 1 | 1 | 5 | 2 | 4 | 4 | 3 | 1 | 1 | 1 | 1 | 1 | | | | | | | | | | | |
| | 2 | 2 | 2 | 1 | 1 | 2 | 4 | 2 | 2 | 1 | 1 | 5 | 4 | 3 | 4 | 4 | 1 | 1 | 4 | 1 | 3 | | | | | | | | | | | |
| | 4 | 4 | 1 | 2 | 2 | 4 | 4 | 2 | 2 | 2 | 3 | 5 | 4 | 3 | 4 | 4 | 1 | 1 | 4 | 2 | 4 | | | | | | | | | | | |
| | 8 | 1 | | | | | | | | | | | 4 | | 5 | 3 | | 1 | | 1 | 1 | | | 1 | 1 | | 1 | 1 | | 1 | 2 | 2 |

Table V cont'd.

PLANT SPECIES

Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 10 | 1 | 1 | 1 | 1 | 2 | 3 | 4 | 1 | 5 | 1 | 1 | 5 | 4 | 4 | 5 | 4 | 3 | 3 | 2 | 2 | 4 |
| | 2 | 1 | 1 | 1 | 2 | 5 | 4 | 1 | 4 | 3 | 1 | 5 | 4 | 4 | 5 | 4 | 2 | 3 | 2 | 2 | 4 |
| | 4 | 1 | 1 | 3 | 2 | 5 | 4 | 2 | 4 | 3 | 3 | 5 | 4 | 4 | 5 | 4 | 3 | 3 | 2 | 4 | 4 |
| | 8 | 3 | | | | | | | | | | | 4 | | 5 | 4 | | 5 | | 4 | 5 |
| 11 | 1 | 1 | 1 | 1 | 1 | 2 | 2 | 1 | 1 | 1 | 2 | 5 | 4 | 2 | 5 | 3 | 2 | 2 | 1 | 3 | 4 |
| | 2 | 1 | 1 | 2 | 1 | 3 | 3 | 1 | 1 | 1 | 2 | 5 | 4 | 4 | 5 | 4 | 1 | 2 | 1 | 3 | 4 |
| | 4 | 1 | 1 | 1 | 1 | 3 | 4 | 1 | 2 | 1 | 1 | 5 | 4 | 4 | 5 | 3 | 2 | 2 | 1 | 3 | 4 |
| | 8 | 1 | | | | | | | | | | | 4 | | 5 | 4 | | 4 | | 4 | 5 |
| 12 | 1 | 1 | 1 | 1 | 1 | 1 | 3 | 1 | 1 | 1 | 1 | 3 | 2 | 1 | 3 | 1 | 1 | 1 | 1 | 1 | 2 |
| | 2 | 1 | 1 | 1 | 1 | 1 | 3 | 1 | 1 | 1 | 1 | 3 | 2 | 2 | 4 | 1 | 1 | 1 | 1 | 1 | 3 |
| | 4 | 1 | 1 | 1 | 1 | 1 | 5 | 2 | 1 | 1 | 1 | 5 | 3 | 3 | 5 | 2 | 1 | 3 | 2 | 4 | 4 |
| | 8 | 1 | | | | | | | | | | | 4 | | 5 | 2 | | 2 | | 4 | 4 |
| 13 | 1 | 1 | 1 | 1 | 1 | 2 | 5 | 2 | 5 | 5 | 2 | 5 | 4 | 5 | 5 | 4 | 4 | 4 | 5 | 5 | 4 |
| | 2 | 1 | 1 | 3 | 2 | 3 | 5 | 3 | 5 | 5 | 4 | 5 | 4 | 4 | 5 | 4 | 4 | 5 | 5 | 5 | 5 |
| | 4 | 2 | 3 | 3 | 3 | 4 | 5 | 3 | 5 | 5 | 4 | 5 | 4 | 4 | 5 | 4 | 5 | 5 | 5 | 5 | 5 |
| | 8 | 3 | | | | | | | | | | | 5 | | 5 | 4 | | 5 | | 4 | 4 |

Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 10 | 1 | | | 2 | 4 | | 5 | 4 | | 4 | 5 | 4 |
| | 2 | | | 3 | 4 | | 5 | 4 | | 4 | 5 | 4 |
| | 4 | | | 2 | 4 | | 5 | 4 | | 5 | 4 | 4 |
| | 8 | | | 3 | 5 | | 5 | 5 | | 5 | 5 | 5 |
| 11 | 1 | | | 1 | 3 | | 5 | 2 | | 3 | 3 | 3 |
| | 2 | | | 1 | 3 | | 5 | 2 | | 3 | 3 | 4 |
| | 4 | | | 1 | 3 | | 5 | 2 | | 3 | 3 | 3 |
| | 8 | | | 1 | 4 | | 4 | 3 | | 3 | 3 | 4 |
| 12 | 1 | | | | | | | | | | | |
| | 2 | | | | | | | | | | | |
| | 4 | | | | | | | | | | | |
| | 8 | | | 1 | 2 | | 2 | 1 | | 1 | 2 | 3 |
| 13 | 1 | | | 1 | 3 | | 4 | 3 | | 4 | 4 | 4 |
| | 2 | | | 1 | 3 | | 4 | 3 | | 4 | 4 | 4 |
| | 4 | | | 1 | 4 | | 4 | 3 | | 4 | 4 | 4 |
| | 8 | | | 2 | 4 | | 5 | 4 | | 4 | 3 | 4 |

## Table V cont'd.

### PLANT SPECIES

**Pre-emergence**

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 14 | 1 | 1 | 1 | 1 | 1 | 1 | 2 | 1 | 1 | 1 | 1 | 3 | 2 | 1 | 4 | 1 | 1 | 1 | 1 | 1 | 2 |
| | 2 | 1 | 1 | 1 | 1 | 1 | 2 | 1 | 1 | 1 | 1 | 5 | 2 | 2 | 4 | 1 | 1 | 1 | 1 | 1 | 2 |
| | 4 | 1 | 1 | 2 | 1 | 1 | 3 | 2 | 1 | 1 | 1 | 5 | 3 | 2 | 4 | 1 | 1 | 1 | 1 | 1 | 3 |
| | 8 | 1 | | | | | | | | | | | 4 | | 5 | 2 | | 3 | | 3 | 4 |
| 15 | 1 | 1 | 1 | 1 | 1 | 1 | 4 | 2 | 2 | 3 | 3 | 3 | 3 | 4 | 4 | 3 | 1 | 1 | 3 | 1 | 4 |
| | 2 | 1 | 2 | 2 | 3 | 5 | 5 | 2 | 3 | 4 | 5 | 4 | 3 | 4 | 3 | 3 | 1 | 3 | 3 | 1 | 2 |
| | 4 | 1 | 2 | 2 | 2 | 3 | 4 | 1 | 3 | 3 | 3 | 5 | 3 | 3 | 4 | 4 | 1 | 1 | 1 | 1 | 3 |
| | 8 | 1 | | | | | | | | | | | 4 | | 5 | 4 | | 5 | | 2 | 4 |
| 16 | 1 | 1 | 1 | 1 | 1 | 1 | 4 | 2 | 3 | 1 | 2 | 4 | 3 | 3 | 3 | 2 | 1 | 1 | 2 | 1 | 1 |
| | 2 | 1 | 1 | 1 | 1 | 1 | 4 | 2 | 2 | 1 | 1 | 4 | 3 | 1 | 4 | 3 | 3 | 3 | 2 | 1 | 5 |
| | 4 | 2 | 1 | 2 | 1 | 4 | 2 | 1 | 2 | 2 | 3 | 5 | 3 | 3 | 5 | 3 | 3 | 2 | 3 | 2 | 4 |
| | 8 | 2 | | | | | | | | | | | 4 | | 4 | 4 | | 4 | | 3 | 4 |
| 17 | 1 | 3 | 4 | 4 | 3 | 5 | 5 | 5 | 5 | 5 | 5 | 5 | 5 | 5 | 5 | 5 | 5 | 5 | 5 | 5 | 5 |
| | 2 | 5 | 4 | 5 | 5 | 5 | 5 | 5 | 5 | 5 | 4 | 5 | 5 | 5 | 5 | 5 | 5 | 5 | 5 | 5 | 5 |
| 18 | 1 | 4 | 4 | 3 | 3 | 4 | 4 | 4 | 3 | 3 | 5 | 5 | 5 | 5 | 5 | 5 | 4 | 5 | 5 | 4 | 5 |
| | 2 | 4 | 3 | 3 | 3 | 4 | 5 | 4 | 4 | 4 | 4 | 5 | 5 | 5 | 5 | 5 | 3 | 5 | 5 | 4 | 5 |
| | 4 | 5 | 4 | 3 | 4 | 5 | 4 | 4 | 4 | 4 | 4 | 5 | 5 | 4 | 5 | 5 | 4 | 5 | 4 | 5 | 5 |
| | 8 | | | | | | | | | 5 | 5 | | 5 | 5 | 5 | 4 | 4 | 5 | | 5 | 5 |

**Post-emergence**

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 14 | 1 | | | | | | | | | | | |
| | 2 | | | | | | | | | | | |
| | 4 | | | | | | | | | | | |
| | 8 | | | 1 | 1 | | 2 | 1 | | 1 | 1 | 2 |
| 15 | 1 | | | | | | | | | | | |
| | 2 | | | | | | | | | | | |
| | 4 | | | | | | | | | | | |
| | 8 | | | 1 | 2 | | 2 | 1 | | 2 | 2 | 2 |
| 16 | 1 | | | 1 | 2 | | 2 | 1 | | 1 | 1 | 2 |
| | 2 | | | 1 | 2 | | 2 | 1 | | 1 | 1 | 3 |
| | 4 | | | 1 | 3 | | 3 | 2 | | 2 | 2 | 3 |
| | 8 | | | 1 | 4 | | 4 | 3 | | 4 | 3 | 3 |
| 17 | 1 | | | 3 | 5 | | 5 | 5 | | 5 | 5 | 5 |
| | 2 | | | 5 | 5 | | 5 | 4 | | 5 | 5 | 5 |
| 18 | 1 | 5 | 3 | | 4 | 5 | 5 | 3 | 4 | 4 | 4 | 4 |
| | 2 | 3 | 4 | | 4 | 4 | 4 | 4 | 2 | 4 | 3 | 4 |
| | 4 | 4 | 4 | | 3 | 4 | 5 | 4 | 2 | 5 | 5 | 5 |
| | 8 | 2 | 4 | | 5 | 4 | 5 | 5 | 3 | 5 | 5 | 3 |

Table V cont'd.

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 19 | 1 | | 1 | 2 | 1 | 1 | 2 | 1 | 1 | 3 | 1 | 5 | 4 | 5 | 4 | 3 | 3 | 3 | 3 | 4 | 3 | | | | | | | | | | | |
| | 2 | 1 | 1 | 1 | 2 | 2 | 4 | 2 | 2 | 2 | 3 | 5 | 2 | 3 | 5 | 2 | 2 | 5 | 2 | 3 | 1 | | | | | | | | | | | |
| | 4 | 1 | 1 | 2 | 2 | 2 | 4 | 2 | 4 | 3 | 3 | 5 | 3 | 3 | 3 | 4 | 2 | 5 | 4 | 3 | 2 | | | | | | | | | | | |
| | 8 | | | | | | | | | 4 | 4 | | 5 | 5 | 5 | 5 | 3 | 5 | | 3 | 4 | 1 | 1 | | 1 | 3 | 4 | 2 | 1 | 3 | 2 | 3 |
| 20 | 1 | 1 | 1 | 1 | 1 | 2 | 4 | 1 | 2 | 1 | 1 | 5 | 2 | 4 | 4 | 1 | 1 | 4 | 1 | 5 | 2 | 1 | 1 | | 1 | 3 | | 2 | 1 | 1 | 3 | 2 |
| | 2 | 1 | 1 | 1 | 1 | 4 | 5 | 2 | 5 | 3 | 1 | 5 | 1 | 1 | 5 | 2 | 2 | 5 | 3 | 5 | 5 | 2 | 2 | | 2 | 3 | 2 | 2 | 2 | 2 | 5 | 2 |
| | 4 | 1 | 3 | 4 | 2 | 4 | 5 | 2 | 5 | 3 | 2 | 5 | 3 | 4 | 5 | 4 | 3 | 5 | 4 | 5 | 5 | 2 | 3 | | 2 | 3 | 3 | 3 | 2 | 4 | 5 | 2 |
| | 8 | | | | | | | | | 5 | 4 | | 5 | 5 | 5 | 5 | 5 | 5 | | 5 | 5 | 3 | 3 | | 3 | 4 | 4 | 1 | 4 | 3 | 2 | 3 |
| 21 | 1 | 1 | 1 | 2 | 1 | 1 | 1 | 2 | 2 | 1 | 1 | 5 | 4 | 4 | 3 | 4 | 1 | 4 | 2 | 2 | 2 | | | | | | | | | | | |
| | 2 | 1 | 1 | 2 | 2 | 3 | 2 | 2 | 3 | 2 | 2 | 5 | 4 | 5 | 4 | 3 | 2 | 3 | 2 | 5 | 2 | | | | | | | | | | | |
| | 4 | 2 | 1 | 2 | 2 | 3 | 4 | 2 | 5 | 2 | 2 | 5 | 4 | 5 | 5 | 4 | 2 | 4 | 2 | 3 | 4 | | | | | | | | | | | |
| | 8 | | | | | | | | | 1 | 3 | | 5 | 5 | 5 | 5 | 3 | 5 | | 5 | 5 | 1 | 3 | | 3 | 4 | 3 | 1 | 1 | 3 | 3 | 1 |
| 22 | 1 | 1 | 1 | 1 | 2 | 3 | 4 | 2 | 1 | 1 | 1 | 3 | 1 | 1 | 3 | 2 | 1 | 4 | 1 | 2 | 1 | | | | | | | | | | | |
| | 2 | 1 | 1 | 1 | 2 | 3 | 4 | 2 | 2 | 1 | 1 | 5 | 1 | 4 | 2 | 4 | 2 | 1 | 5 | 5 | 4 | | | | | | | | | | | |
| | 4 | 1 | 2 | 2 | 2 | 3 | 4 | 2 | 3 | 2 | 1 | 5 | 4 | 4 | 5 | 4 | 2 | 4 | 2 | 3 | 3 | | | | | | | | | | | |
| | 8 | | | | | | | | | 5 | 3 | | 4 | 5 | 5 | 4 | 2 | 5 | | 4 | 5 | 1 | 1 | | 1 | 4 | 3 | 1 | 1 | 2 | 1 | 2 |
| 23 | 1 | 1 | 1 | 1 | 1 | 5 | 3 | 2 | 4 | 3 | 4 | 5 | 3 | 5 | 5 | 3 | 1 | 5 | 3 | 5 | 5 | 5 | 4 | | 4 | 5 | 5 | 2 | 2 | 4 | 5 | 4 |
| | 2 | 1 | 2 | 3 | 3 | 5 | 4 | 4 | 5 | 3 | 3 | 5 | 3 | 4 | 5 | 4 | 2 | 4 | 3 | 5 | 4 | 4 | 3 | | 4 | 5 | 5 | 4 | | 4 | 5 | 5 |
| | 4 | 2 | 2 | 2 | 2 | 5 | 5 | 2 | 5 | 3 | 3 | 5 | 4 | 3 | 5 | 2 | 4 | 5 | 3 | 4 | 3 | 5 | 4 | | 4 | 5 | | 4 | 4 | 4 | 5 | 5 |
| | 8 | | | | | | | | | 5 | 5 | | 3 | 5 | 5 | 5 | 5 | 5 | | 5 | 5 | 3 | 4 | | 5 | 5 | 5 | 5 | 2 | 4 | 5 | 4 |

## Table V cont'd.

### PLANT SPECIES

| Compound of Example No. | appln. rate lbs/A | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 24 | 1 | 1 | 3 | 3 | 3 | 5 | 4 | 2 | 5 | 3 | 4 | 5 | 5 | 3 | 3 | 3 | 2 | 5 | 2 | 5 | 3 | 2 | 2 | | 4 | 5 | 5 | 4 | 4 | 4 | 5 | 5 |
| | 2 | 2 | 5 | 4 | 2 | 5 | 5 | 3 | 5 | 5 | 3 | 5 | 4 | 3 | 5 | 4 | 3 | 5 | 4 | 4 | 4 | 5 | 4 | | 5 | 5 | 5 | 5 | 5 | 5 | 5 | 5 |
| | 4 | 2 | 5 | 4 | 4 | 5 | 4 | 2 | 5 | 5 | 4 | 5 | 4 | 4 | 4 | 5 | 4 | 4 | 5 | 5 | 4 | 5 | 4 | | 5 | 5 | 5 | 5 | 5 | 5 | 5 | 5 |
| | 8 | | | | | | | | | 5 | 5 | | 5 | 5 | 5 | 5 | 5 | 5 | | 5 | 5 | 4 | 5 | | 5 | 5 | 5 | 5 | 4 | 5 | 5 | 5 |
| 25 | 1 | 1 | 1 | 1 | 1 | 1 | 1 | 1 | 1 | 1 | 1 | 5 | 1 | 1 | 3 | 1 | 1 | 1 | 1 | 1 | 1 | | | | | | | | | | | |
| | 2 | 1 | 1 | 1 | 1 | 1 | 2 | 1 | 1 | 1 | 1 | 5 | 1 | 1 | 3 | 1 | 1 | 3 | 2 | 1 | 1 | | | | | | | | | | | |
| | 4 | 1 | 2 | 1 | 1 | 1 | 4 | 1 | 2 | 2 | 1 | 4 | 1 | 1 | 4 | 1 | 1 | 4 | 2 | 1 | 1 | | | | | | | | | | | |
| | 8 | | | | | | | | | 3 | 1 | | 5 | 5 | 5 | 2 | 2 | 3 | | 3 | 5 | 1 | 1 | | 1 | 1 | 1 | 1 | 1 | 1 | 1 | 1 |
| 26 | 1 | 1 | 1 | 1 | 2 | 5 | 4 | 2 | 5 | 4 | 3 | 5 | 4 | 4 | 4 | 4 | 1 | 3 | 4 | 5 | 4 | 1 | 1 | | 1 | 2 | 2 | 2 | 1 | 2 | 2 | 2 |
| | 2 | 1 | 2 | 2 | 1 | 5 | 4 | 2 | 4 | 3 | 3 | 5 | 4 | 5 | 5 | 4 | 2 | 5 | 1 | 5 | 5 | 3 | 2 | | | 5 | | 3 | 4 | 2 | 4 | 3 |
| | 4 | 2 | 2 | 4 | 2 | 5 | 5 | 2 | 5 | 3 | 3 | 5 | 4 | 4 | 5 | 5 | 2 | 5 | 4 | 5 | 5 | | | | 3 | 5 | 5 | 4 | 4 | 3 | 5 | 4 |
| | 8 | | | | | | | | | 3 | 3 | | 5 | 5 | 5 | 5 | 3 | 5 | | 5 | 5 | 3 | 4 | | 4 | 4 | 3 | 4 | 3 | 3 | 3 | 3 |
| 27 | 1 | 1 | 1 | 1 | 1 | 2 | 2 | 2 | 2 | 1 | 1 | 5 | 1 | 1 | 4 | 1 | 1 | 1 | 1 | 1 | 1 | | | | | | | | | | | |
| | 2 | 1 | 1 | 2 | 2 | 4 | 4 | 2 | 1 | 1 | 1 | 5 | 2 | 1 | 4 | 2 | 1 | 2 | 1 | 2 | 2 | | | | | | | | | | | |
| | 4 | 1 | 3 | 2 | 2 | 5 | 5 | 2 | 3 | 1 | 1 | 5 | 3 | 5 | 5 | 4 | 2 | 2 | 1 | 5 | 3 | | | | | | | | | | | |
| | 8 | | | | | | | | | 3 | 2 | | 3 | 3 | 5 | 3 | 3 | 5 | | 4 | 3 | 1 | 1 | | 1 | 3 | 3 | 1 | 1 | 1 | 1 | 1 |

Table V cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 28 | 1 | 1 | 1 | 1 | 1 | 2 | 1 | 1 | 1 | 2 | 2 | 5 | 4 | | 3 | 1 | 1 | 4 | 2 | 1 | 1 | 1 | 1 | | 1 | 2 | 3 | 2 | 2 | 2 | 3 | 2 |
| | 2 | 1 | 1 | 1 | 1 | 2 | 4 | 2 | 3 | 1 | 4 | 5 | 4 | | 4 | 2 | 2 | 4 | 1 | 2 | 5 | 1 | 2 | | 2 | 2 | 3 | 2 | 2 | 2 | 2 | 2 |
| | 4 | 1 | 1 | 1 | 2 | 4 | 4 | 2 | 3 | 1 | 4 | 5 | 4 | | 4 | 2 | 2 | 4 | 2 | 5 | 5 | 1 | 2 | | 2 | 3 | 3 | 2 | 2 | 3 | 2 | 2 |
| | 8 | | | | | | | | | 4 | 4 | | 5 | 5 | 5 | 5 | 5 | 5 | | 5 | 5 | 2 | 4 | | 4 | 4 | 5 | 1 | 3 | 3 | 2 | 3 |
| 29 | 1 | 1 | 1 | 1 | 1 | 2 | 4 | 2 | 2 | 2 | 1 | 5 | 4 | | 4 | 2 | 1 | 4 | 2 | 1 | 1 | 3 | 2 | | 3 | 5 | 2 | 3 | 3 | 4 | 5 | 4 |
| | 2 | 1 | 1 | 2 | 2 | 3 | 4 | 2 | 5 | 5 | 4 | 5 | 4 | | 4 | 2 | 1 | 5 | 1 | 5 | 1 | 3 | 2 | | 4 | 5 | 4 | 3 | 2 | 4 | 5 | 4 |
| | 4 | 2 | 5 | 2 | 1 | 4 | 4 | 2 | 5 | 3 | 4 | 5 | 5 | | 5 | 4 | 4 | 5 | 2 | 5 | 5 | 4 | 3 | | 4 | 5 | 5 | 4 | 3 | 4 | 4 | 5 |
| | 8 | | | | | | | | | 5 | 5 | | 5 | 5 | 5 | 5 | 5 | 5 | | 5 | 5 | 5 | 5 | | 5 | 5 | 5 | 5 | 5 | 5 | 5 | 5 |
| 30 | 1 | 1 | 1 | 1 | 1 | 1 | 1 | 1 | 1 | 1 | 1 | 2 | 1 | | 1 | 1 | 1 | 1 | 1 | 1 | 1 | | | | | | | | | | | |
| | 2 | 1 | 1 | 1 | 1 | 1 | 2 | 1 | 1 | 1 | 1 | 4 | 1 | | 2 | 1 | 1 | 2 | 1 | 1 | 1 | | | | | | | | | | | |
| | 4 | 1 | 1 | 1 | 1 | 1 | 2 | 1 | 1 | 1 | 1 | 4 | 1 | | 1 | 1 | 1 | 3 | 2 | 2 | 1 | | | | | | | | | | | |
| | 8 | | | | | | | | | 5 | 4 | | 4 | 5 | 5 | 3 | 3 | 4 | | 4 | 4 | 1 | 1 | | 1 | 3 | 3 | 1 | 1 | 1 | 1 | 2 |
| 31 | 1 | 1 | 1 | 1 | 1 | 1 | 1 | 1 | 1 | 1 | 1 | 3 | 1 | | 2 | 1 | 1 | 1 | 1 | 1 | 1 | 1 | 1 | | 1 | 1 | 1 | 1 | 1 | 1 | 1 | 1 |
| | 2 | 1 | 1 | 1 | 1 | 2 | 3 | 1 | 1 | 1 | 1 | 2 | 2 | | 2 | 1 | 3 | 1 | 1 | 1 | 1 | 1 | 1 | | 1 | 1 | 1 | 1 | 1 | 1 | 1 | 1 |
| | 4 | 1 | 1 | 1 | 1 | 2 | 3 | 1 | 1 | 1 | 1 | 5 | 4 | | 2 | 2 | | | | | | | | | | | | | | | | |
| | 8 | | | | | | | | | 2 | 3 | | 3 | 3 | 4 | 2 | 1 | 4 | | 3 | 2 | 2 | 2 | | 1 | 3 | 4 | 2 | 2 | 2 | 4 | 3 |

Table V cont'd.

PLANT SPECIES

Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 32 | 1 | 1 | 1 | 1 | 1 | 1 | 1 | 1 | 1 | 1 | 1 | 2 | 2 | | 2 | 1 | 1 | 1 | 1 | 1 | 1 |
| | 2 | 1 | 1 | 1 | 1 | 1 | 1 | 1 | 1 | 1 | 1 | 1 | 2 | | 3 | 1 | 1 | 1 | 1 | 2 | 1 |
| | 4 | 1 | 1 | 1 | 1 | 1 | 1 | 1 | 1 | 2 | 1 | 4 | 1 | | 1 | 1 | 3 | 2 | 1 | 2 | 1 |
| | 8 | | | | | | | | | 2 | 3 | | 2 | 3 | 4 | 1 | 2 | 4 | | 2 | 1 |
| 33 | 8 | | | | | | | | | 2 | 1 | | 1 | 3 | 3 | 1 | 1 | 4 | | 1 | 1 |
| 34 | 1 | 1 | 2 | 1 | 1 | 1 | 3 | 2 | 3 | 1 | 1 | 4 | 1 | 3 | 4 | 4 | 1 | 4 | 3 | 2 | 4 |
| | 2 | 1 | 3 | 1 | 2 | 1 | 3 | 2 | 3 | 1 | 1 | 4 | 1 | 3 | 4 | 4 | 2 | 4 | 5 | 5 | 3 |
| | 4 | 2 | 5 | 2 | 3 | 4 | 5 | 3 | 5 | 3 | 3 | 5 | 3 | 5 | 4 | 4 | 2 | 5 | 5 | 5 | 3 |
| | 8 | | | | | | | | | 4 | 4 | | 4 | 5 | 5 | 4 | 4 | 5 | | 5 | 5 |
| 35 | 1 | 1 | 1 | 1 | 2 | 1 | 4 | 3 | 1 | 2 | 1 | 3 | 1 | 1 | 2 | 4 | 2 | 1 | 3 | 5 | 2 |
| | 2 | 1 | 2 | 1 | 2 | 1 | 4 | 3 | 1 | 2 | 1 | 5 | 3 | 5 | 4 | 4 | 2 | 4 | 1 | 4 | 5 |
| | 4 | 3 | 3 | 1 | 2 | 1 | 5 | 3 | 1 | 3 | 2 | 4 | 3 | 5 | 5 | 4 | 3 | 3 | 2 | 3 | 5 |
| | 8 | | | | | | | | | 5 | 4 | | 5 | 5 | 5 | 5 | 5 | 5 | | 5 | 5 |
| 36 | 8 | | | | | | | | | 2 | 3 | | 2 | 2 | 3 | 1 | 2 | 4 | | 1 | 2 |

Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 32 | 1 | | | | | | | | | | | |
| | 2 | | | | | | | | | | | |
| | 4 | | | | | | | | | | | |
| | 8 | 1 | 1 | | 2 | 3 | 3 | 1 | 1 | 1 | 2 | 2 |
| 33 | 8 | 1 | 1 | | 1 | 3 | 1 | 1 | 1 | 3 | 1 | 2 |
| 34 | 1 | 1 | 1 | | 2 | 2 | 2 | 1 | 1 | 1 | 2 | 2 |
| | 2 | 1 | 1 | | 2 | 2 | 3 | 1 | 1 | 1 | 1 | 2 |
| | 4 | 2 | 1 | | 2 | 4 | 2 | 1 | 1 | 3 | 2 | 2 |
| | 8 | 4 | 2 | | 2 | 2 | 2 | 4 | 1 | 5 | 2 | 3 |
| 35 | 1 | 3 | 2 | | 1 | 4 | | 2 | 3 | 3 | 2 | 4 |
| | 2 | 4 | 4 | | 3 | 4 | 4 | 2 | 2 | 5 | 5 | 4 |
| | 4 | 3 | 4 | | 2 | 5 | 4 | 4 | 2 | 4 | 3 | 4 |
| | 8 | 4 | 2 | | 3 | 2 | 3 | 4 | 2 | 5 | 4 | 4 |
| 36 | 8 | 1 | 1 | | 1 | 3 | 2 | 1 | 1 | 1 | 1 | 1 |

Table V cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 37 | 1 | 1 | 1 | 1 | 1 | 1 | 4 | 2 | 2 | 2 | 2 | 4 | 3 | 3 | 4 | 3 | 1 | 1 | 2 | 3 | 2 | | | | | | | | | | | |
| | 2 | 2 | 1 | 2 | 1 | 4 | 5 | 2 | 3 | 2 | 2 | 4 | 4 | 2 | 5 | 4 | 2 | 3 | 2 | 3 | 3 | | | | | | | | | | | |
| | 4 | 3 | 1 | 2 | 3 | 3 | 5 | 3 | 4 | 2 | 5 | 4 | 5 | 5 | 5 | 4 | 2 | 3 | 4 | 3 | 5 | | | | | | | | | | | |
| | 8 | | | | | | | | | 2 | 3 | | 4 | 2 | 4 | 2 | 2 | 3 | | 3 | 3 | 1 | 2 | | 2 | 2 | 2 | 3 | 2 | 1 | 2 | 2 |
| 38 | 1 | 2 | 1 | 1 | 2 | 3 | 3 | 2 | 3 | 2 | 1 | 4 | 4 | 2 | 4 | 3 | 2 | 2 | 2 | 3 | 3 | 4 | 2 | | 2 | 4 | 4 | 4 | 1 | 4 | 4 | 4 |
| | 2 | 2 | 3 | 2 | 2 | 4 | 4 | 2 | 5 | 5 | 2 | 5 | 4 | 4 | 4 | 4 | 2 | 4 | 3 | 4 | 4 | 3 | 2 | | 3 | 4 | 5 | 5 | 1 | 5 | 5 | 4 |
| | 4 | 3 | 4 | 3 | 3 | 5 | 4 | 4 | 5 | 4 | 4 | 5 | 4 | 4 | 5 | 4 | 2 | 5 | 3 | 4 | 5 | 3 | 3 | | 4 | 4 | 5 | 5 | 2 | 5 | 5 | 4 |
| | 8 | | | | | | | | | 5 | 4 | | 4 | 4 | 5 | 4 | 3 | 4 | | 3 | 5 | 4 | 3 | | 5 | 4 | 5 | 4 | 3 | 5 | 4 | 5 |
| 39 | 1 | 1 | 3 | 2 | 2 | 5 | 3 | 2 | 5 | 4 | 3 | 5 | 5 | 4 | 5 | 4 | 4 | 5 | 2 | 5 | 5 | 5 | 4 | | 5 | 5 | 5 | 5 | 4 | 5 | 5 | 5 |
| | 2 | 3 | 3 | 3 | 3 | 5 | 5 | 3 | 5 | 4 | 4 | 5 | 5 | 5 | 5 | 5 | 4 | 5 | 5 | 5 | 5 | 5 | 4 | | 5 | 5 | 5 | 5 | 4 | 5 | 5 | 5 |
| | 4 | 3 | 4 | 4 | 3 | 5 | 5 | 3 | 5 | 5 | 4 | 5 | 5 | 5 | 5 | 5 | 4 | 5 | 5 | 5 | 5 | 5 | 4 | | 5 | 5 | 5 | 5 | 5 | 5 | 4 | 5 |
| | 8 | | | | | | | | | 5 | 5 | | 4 | 5 | 5 | 5 | 5 | 5 | | 5 | 5 | 5 | 4 | | 5 | 5 | 5 | 5 | 5 | 5 | 5 | 5 |
| 40 | 1 | 2 | 2 | 1 | 1 | 2 | 3 | 1 | 2 | 2 | 2 | 5 | 4 | 3 | 3 | 4 | 1 | 2 | 2 | 2 | 2 | 3 | 4 | | 5 | 4 | 5 | 4 | 2 | 4 | 4 | 5 |
| | 2 | 2 | 1 | 1 | 1 | 3 | 4 | 2 | 5 | 2 | 2 | 5 | 5 | 4 | 5 | 4 | 2 | 4 | 2 | 2 | 4 | 4 | 4 | | 5 | 5 | 4 | 5 | 2 | 5 | 5 | 5 |
| | 4 | 2 | 3 | 2 | 2 | 3 | 4 | 3 | 4 | 2 | 2 | 5 | 5 | 3 | 4 | 4 | 2 | 4 | 3 | 4 | 2 | 4 | 4 | | 5 | 5 | 5 | 5 | 2 | 5 | 4 | 5 |
| | 8 | | | | | | | | | 4 | 4 | | 5 | 4 | 5 | 4 | 4 | 4 | | 4 | 5 | 4 | 3 | | 5 | 5 | 5 | 4 | 2 | 5 | 5 | 4 |

Table V cont'd.

PLANT SPECIES

| Compound of Example No. | appln. rate lbs/A | Pre: Corn | Pre: Cotton | Pre: Soybean | Pre: Wheat | Pre: Alfalfa | Pre: Sugar Beet | Pre: Rice | Pre: Cucumber | Pre: Tomato | Pre: Barnyard Grass | Pre: Lambsquarter | Pre: Large Crabgrass | Pre: Mustard | Pre: Pigweed | Pre: Foxtail | Pre: Wild Oats | Pre: Velvetleaf | Pre: Jimsonweed | Pre: Morningglory | Pre: Zinnia | Post: Tomato | Post: Barnyard Grass | Post: Corn | Post: Large Crabgrass | Post: Mustard | Post: Pigweed | Post: Foxtail | Post: Wild Oats | Post: Velvetleaf | Post: Morningglory | Post: Zinnia |
|---|---|---|---|---|---|---|---|---|---|---|---|---|---|---|---|---|---|---|---|---|---|---|---|---|---|---|---|---|---|---|---|---|
| 41 | 1 | 2 | 1 | 2 | 1 | 5 | 4 | 1 | 5 | 4 | 2 | 4 | 4 | 4 | 5 | 4 | 3 | 4 | 2 | 5 | 3 | 5 | 4 | | 5 | 5 | 5 | 4 | 3 | 5 | 5 | 5 |
| | 2 | 3 | 3 | 3 | 2 | 5 | 5 | 1 | 5 | 5 | 2 | 5 | 5 | 4 | 5 | 4 | 4 | 5 | 4 | 5 | 5 | 5 | 5 | | 5 | 5 | 5 | 5 | 4 | 5 | 5 | 5 |
| | 4 | 3 | 2 | 3 | 3 | 5 | 5 | 2 | 5 | 5 | 3 | 5 | 5 | 5 | 5 | 4 | 5 | 4 | 3 | 5 | 5 | 5 | 5 | | 4 | 5 | 5 | 5 | 4 | 5 | 5 | 5 |
| | 8 | | | | | | | | | 5 | 3 | | 5 | 4 | 5 | 4 | 4 | 5 | | 3 | 5 | 5 | 5 | | 5 | 5 | 5 | 5 | 4 | 5 | 5 | 5 |
| 43 | 1 | 1 | 1 | 1 | 2 | 3 | 4 | 2 | 1 | 1 | 1 | 3 | 1 | 1 | 3 | 2 | 1 | 4 | 1 | 2 | 1 | | | | | | | | | | | |
| | 2 | 1 | 1 | 1 | 2 | 3 | 4 | 2 | 2 | 1 | 1 | 5 | 1 | 4 | 2 | 4 | 2 | 1 | 5 | 5 | 4 | | | | | | | | | | | |
| | 4 | 1 | 2 | 2 | 2 | 3 | 4 | 2 | 3 | 1 | 1 | 5 | 4 | 4 | 5 | 4 | 2 | 4 | 2 | 3 | 3 | | | | | | | | | | | |
| | 8 | | | | | | | | | 5 | 3 | | 4 | 5 | 5 | 4 | 2 | 5 | | 4 | 5 | | | | | | | | | | | |
| 44 | 1 | 2 | 1 | 4 | 4 | 4 | 4 | 3 | 4 | 4 | 3 | 5 | 5 | 4 | 5 | 4 | 5 | 5 | 4 | 4 | 4 | | | 2 | 4 | | 5 | 4 | | 5 | 5 | 5 |
| | 2 | 4 | 3 | 5 | 4 | 4 | 5 | 3 | 5 | 4 | 3 | 5 | 5 | 5 | 4 | 4 | 5 | 5 | 2 | 4 | 5 | | | 2 | 5 | | 5 | 4 | | 5 | 4 | 5 |
| | 4 | 3 | 2 | 5 | 4 | 4 | 4 | 4 | 5 | 5 | 3 | 5 | 5 | 5 | 5 | 5 | 5 | 5 | 4 | 4 | 5 | | | 3 | 4 | | 5 | 4 | | 5 | 5 | 5 |
| | 8 | 4 | | | | | | | | | | | 5 | | 5 | 5 | | 5 | | 4 | 5 | | | 4 | 4 | | 5 | 4 | | 5 | 4 | 5 |
| 45 | 1 | 1 | 1 | 1 | 4 | 5 | | | | 1 | 5 | 5 | 4 | 4 | | 4 | | 5 | 4 | 5 | | 4 | 3 | | 4 | 4 | 5 | 4 | 3 | 5 | 4 | 4 |
| | 2 | 1 | 5 | 5 | 4 | 5 | | | 5 | | 5 | 5 | 5 | 5 | 5 | 4 | 5 | 5 | 5 | 5 | | 4 | 4 | | 4 | 4 | 5 | 4 | 4 | 5 | 5 | 5 |
| | 4 | 3 | 5 | 5 | 5 | 5 | 4 | 4 | 5 | 4 | 5 | 5 | 5 | 5 | | 5 | 5 | 5 | 5 | 5 | 5 | 5 | 4 | | 4 | 5 | 5 | 4 | 4 | 5 | 5 | 5 |
| | 8 | | | | | | | | | 5 | 5 | | 5 | 4 | 5 | 5 | 5 | 5 | | 5 | 5 | 3 | 4 | | 5 | 5 | 5 | 5 | 5 | 4 | 5 | 5 |
| 46 | 1 | 1 | 4 | 2 | 2 | 5 | 4 | 2 | 5 | 1 | 2 | 5 | 4 | 2 | 5 | 2 | 2 | 4 | 2 | 5 | 5 | 4 | 4 | | 4 | 4 | 4 | 5 | 4 | 5 | 5 | 5 |
| | 2 | 1 | 4 | 4 | 4 | 5 | 5 | 3 | 5 | 2 | 4 | 5 | 4 | 4 | 5 | 5 | 4 | 4 | 3 | 5 | 5 | 4 | 4 | | 4 | 3 | 4 | 4 | 4 | 5 | 5 | 5 |
| | 4 | 3 | 5 | 4 | 5 | 5 | 4 | 5 | 5 | 2 | 4 | 5 | 4 | 4 | 5 | 5 | 4 | 4 | 4 | 5 | 5 | 4 | 4 | | 4 | 4 | 4 | 4 | 5 | 5 | 5 | 5 |
| | 8 | | | | | | | | | 4 | 5 | | 4 | | 4 | 5 | 5 | 5 | | 5 | 5 | 4 | 4 | | 4 | 5 | 5 | 5 | 4 | 5 | 5 | 5 |

Table V cont'd.

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 47 | 1 | 1 | 1 | 3 | 2 | 5 | 5 | 2 | 5 | 3 | 3 | 5 | 4 | 5 | 5 | 2 | 2 | 3 | 4 | 4 | 5 | 5 | 2 | | 4 | 5 | | 2 | 4 | 4 | 5 | 4 |
| | 2 | 1 | 2 | 3 | 3 | 5 | 4 | 2 | 5 | 5 | 4 | 5 | 4 | 5 | 5 | 4 | 3 | 5 | 5 | 4 | 5 | 5 | 4 | | 4 | 5 | | 4 | 4 | 4 | 5 | 4 |
| | 4 | 2 | 4 | 3 | 2 | 5 | 5 | 2 | 5 | 4 | 4 | 5 | 4 | 4 | 5 | 4 | 3 | 5 | 4 | 4 | 5 | 5 | 3 | | | 5 | | 4 | 4 | 4 | 5 | 5 |
| | 8 | | | | | | | | | 3 | 3 | | 5 | 5 | 5 | 4 | 5 | 5 | | 5 | 5 | 4 | 3 | | 4 | 3 | 4 | 3 | 4 | 3 | 4 | 4 |
| 48 | 1 | 1 | 1 | 1 | 1 | 1 | 2 | 1 | 2 | 1 | 1 | 4 | 2 | | 2 | 1 | 1 | 3 | 2 | 1 | 1 | | | | | | | | | | | |
| | 2 | 1 | 1 | 1 | 1 | 1 | 3 | 1 | 2 | 1 | 1 | 4 | 1 | | 3 | 1 | 1 | 2 | 2 | 1 | 1 | | | | | | | | | | | |
| | 4 | 1 | 1 | 1 | 1 | 2 | 3 | 2 | 2 | 2 | 2 | 3 | 2 | | 3 | 1 | 2 | 2 | 1 | 3 | 2 | | | | | | | | | | | |
| | 8 | | | | | | | | | 2 | 4 | | 3 | 4 | 4 | 3 | 1 | 4 | | 3 | 2 | 1 | 2 | | 1 | 3 | 3 | 2 | 1 | 2 | 2 | 2 |
| 49 | 1 | 2 | 1 | 2 | 1 | 4 | 4 | 1 | 2 | 1 | 2 | 5 | 3 | 2 | 5 | 3 | 2 | 2 | 1 | 2 | 3 | | | 1 | 2 | | 4 | 2 | | 4 | 2 | 4 |
| | 2 | 2 | 1 | 2 | 1 | 4 | 4 | 2 | 2 | 1 | 1 | 5 | 4 | 4 | 5 | 4 | 3 | 5 | 2 | 4 | 4 | | | 1 | 3 | | 4 | 2 | | 4 | 4 | 4 |
| | 4 | 3 | 1 | 3 | 1 | 3 | 4 | 1 | 3 | 1 | 1 | 5 | 4 | 4 | 5 | 4 | 3 | 4 | 3 | 4 | 4 | | | 2 | 3 | | 5 | 2 | | 4 | 4 | 4 |
| | 8 | 2 | | | | | | | | | | | 4 | | 5 | 4 | | 5 | | 5 | 5 | | | 2 | 3 | | 4 | 3 | | 4 | 3 | 4 |
| 50 | 8 | | | | | | | | | 1 | 2 | | 3 | 3 | 3 | 2 | 2 | 2 | | 3 | 3 | 1 | 2 | | 2 | 3 | 4 | 2 | 1 | 1 | 1 | 1 |
| 51 | 1 | 1 | 1 | 1 | 1 | 3 | 3 | 1 | 2 | 1 | 1 | 4 | 3 | 3 | 4 | 1 | 1 | 1 | 2 | 1 | 1 | 2 | 1 | | 2 | 3 | 4 | 1 | 1 | 1 | 3 | 3 |
| | 2 | 1 | 1 | 1 | 1 | 3 | 4 | 1 | 2 | 1 | 1 | 5 | 3 | 3 | 4 | 1 | 1 | 2 | 3 | 2 | 1 | 2 | 1 | | 3 | 3 | 4 | 2 | 1 | 1 | 2 | 3 |
| | 4 | 1 | 1 | 1 | 1 | 4 | 4 | 1 | 2 | 2 | 1 | 5 | 4 | 3 | 5 | 3 | 1 | 2 | 3 | 2 | 2 | 2 | 1 | | 4 | 4 | 5 | 3 | 1 | 2 | 4 | 4 |
| | 8 | | | | | | | | | 2 | 2 | | 4 | 4 | 5 | 3 | 2 | 2 | | 2 | 3 | 5 | 1 | | 4 | 4 | 5 | 3 | 2 | 4 | 4 | 4 |

Table V cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 53 | 1 | 1 | 1 | 1 | 1 | 1 | 3 | 1 | 1 | 1 | 1 | 4 | 1 | 1 | 1 | 1 | 1 | 3 | 1 | 1 | 1 | | | 1 | 2 | | 3 | 2 | | 3 | 3 | 3 |
| | 2 | 1 | 1 | 1 | 1 | 1 | 3 | 2 | 2 | 1 | 1 | 5 | 3 | 3 | 3 | 2 | 1 | 3 | 3 | 3 | 3 | | | 1 | 3 | | 4 | 2 | | 4 | 4 | 3 |
| | 4 | 1 | 1 | 1 | 1 | 1 | 4 | 1 | 3 | 4 | 2 | 5 | 4 | 4 | 4 | 4 | 1 | 3 | 4 | 3 | 3 | | | 1 | 4 | | 4 | 3 | | 4 | 3 | 3 |
| | 8 | 1 | | | | | | | | | | | 5 | | 5 | 5 | | 5 | | 3 | 4 | | | 2 | 5 | | 5 | 4 | | 5 | 4 | 4 |
| 54 | 1 | 1 | 1 | 1 | 1 | 1 | 1 | 1 | 1 | 1 | 1 | 5 | 3 | 4 | 4 | 1 | 1 | 1 | 1 | 1 | 1 | 2 | 1 | | 1 | 3 | 3 | 1 | 1 | 1 | 2 | 1 |
| | 2 | 1 | 1 | 1 | 1 | 1 | 1 | 1 | 2 | 1 | 1 | 5 | 3 | 4 | 3 | 2 | 1 | 2 | 1 | 2 | 1 | 2 | 1 | | 2 | 3 | 3 | 2 | 1 | 2 | 3 | 2 |
| | 4 | 1 | 1 | 1 | 1 | 1 | 3 | 1 | 2 | 1 | 1 | 5 | 3 | 4 | 4 | 3 | 1 | 3 | 2 | 3 | 2 | 3 | 1 | | 4 | 4 | 4 | 2 | 1 | 2 | 3 | 3 |
| | 8 | | | | | | | | | 1 | 1 | | 2 | 4 | 4 | 2 | 1 | 2 | | 1 | 1 | 4 | 2 | | 4 | 4 | 5 | 3 | 3 | 4 | 3 | 3 |
| 55 | 1 | 2 | 5 | 2 | 4 | 5 | 5 | 3 | 5 | 2 | 5 | 5 | 5 | 5 | 5 | 5 | 4 | 5 | 5 | 5 | 5 | 4 | 4 | | 5 | 5 | 5 | 5 | 5 | 5 | 5 | 5 |
| | 2 | 3 | 5 | 4 | 5 | 5 | 5 | 3 | 5 | 5 | 4 | 5 | 5 | 5 | 5 | 5 | 5 | 5 | 5 | 5 | 5 | 4 | 4 | | 5 | 5 | 5 | 5 | 5 | 5 | 5 | 5 |
| | 4 | 5 | 5 | 4 | 5 | 5 | 5 | 5 | 5 | 5 | 5 | 5 | 5 | 5 | 5 | 5 | 5 | 5 | 5 | 5 | 5 | 5 | 5 | | 5 | 5 | 5 | 5 | 5 | 5 | 5 | 5 |
| | 8 | | | | | | | | | 5 | 5 | 5 | 5 | 5 | 5 | 5 | 5 | 5 | 5 | 5 | 5 | 5 | 5 | | 5 | 5 | 5 | 5 | 5 | 5 | 5 | 5 |

## Table VI

### PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 2 | 1.0 | 2 | 1 | 3 | 2 | 5 | 5 | 2 | 5 | 2 | 4 | 5 | 5 | 5 | 5 | 5 | 3 | 4 | 5 | 4 | 5 | 4 | 5 | | 4 | 4 | 5 | 4 | 3 | 4 | 4 | 5 |
| | .5 | 2 | 1 | 1 | 1 | 4 | 4 | 2 | 3 | 1 | 1 | 5 | 4 | 5 | 4 | 4 | 2 | 2 | 2 | 4 | 3 | 3 | 2 | | 3 | 3 | 4 | 3 | 3 | 4 | 4 | 5 |
| | .25 | 1 | 1 | 1 | 1 | 1 | 3 | 1 | 1 | 1 | 1 | 5 | 3 | 4 | 4 | 2 | 1 | 3 | 2 | 2 | 2 | 4 | 2 | | 3 | 3 | 3 | 2 | 2 | 4 | 3 | 4 |
| | .25 | 1 | 1 | 1 | 1 | 1 | 4 | 2 | 3 | 1 | 1 | 4 | 4 | 4 | 4 | 2 | 3 | 2 | 2 | 2 | 2 | 4 | 2 | | 4 | 4 | 3 | 4 | 2 | 4 | 3 | 4 |
| | .13 | 1 | 1 | 1 | 1 | 2 | 2 | 1 | 3 | 1 | 1 | 4 | 2 | 2 | 4 | 2 | 2 | 2 | 2 | 1 | 2 | 3 | 2 | | 4 | 4 | 4 | 3 | 2 | 4 | 4 | 3 |
| | .06 | 1 | 1 | 1 | 1 | 1 | 1 | 1 | 1 | 1 | 1 | 1 | 2 | 1 | 1 | 1 | 1 | 1 | 1 | 1 | 1 | 2 | 1 | | 3 | 3 | 4 | 3 | 1 | 3 | 3 | 3 |
| 3 | 1.0 | 2 | 2 | 1 | 1 | 4 | 2 | 2 | 5 | 2 | 2 | 4 | 3 | 4 | 2 | 3 | 2 | 2 | 2 | 2 | 3 | 3 | 3 | | 4 | 4 | 4 | 3 | 2 | 4 | 4 | 5 |
| | .5 | 1 | 2 | 1 | 1 | 2 | 3 | 2 | 2 | 1 | 1 | 4 | 2 | 2 | 3 | 3 | 1 | 2 | 1 | 1 | 2 | 2 | 3 | | 3 | 3 | 3 | 2 | 2 | 3 | 4 | 5 |
| | .25 | 1 | 1 | 1 | 1 | 2 | 3 | 2 | 2 | 1 | 1 | 5 | 2 | 2 | 3 | 2 | 1 | 2 | 2 | 3 | 1 | 2 | 2 | | 2 | 2 | 3 | 3 | 1 | 3 | 4 | 3 |
| | .25 | 1 | 1 | 1 | 2 | 2 | 3 | 2 | 2 | 1 | 1 | 2 | 3 | 2 | 3 | 2 | 1 | 2 | 1 | 2 | 3 | | | | | | | | | | | |
| | .13 | 1 | 1 | 1 | 1 | 2 | 1 | 2 | 1 | 1 | 1 | 1 | 2 | 2 | 2 | 1 | 1 | 2 | 1 | 2 | 1 | | | | | | | | | | | |
| | .06 | 1 | 1 | 1 | 1 | 1 | 1 | 1 | 1 | 1 | 1 | 1 | 2 | 2 | 2 | 2 | 1 | 2 | 1 | 2 | 1 | | | | | | | | | | | |
| 4 | 1.0 | 1 | 2 | 3 | | 4 | 4 | 2 | 5 | 4 | 1 | 5 | 4 | 4 | 4 | 4 | 1 | 3 | 4 | 3 | 4 | 4 | 2 | | 4 | 2 | 4 | 4 | 2 | 5 | 5 | 4 |
| | .5 | 1 | 2 | 1 | | 4 | 4 | 2 | 3 | 2 | 1 | 5 | 3 | 4 | 3 | 1 | 1 | 2 | 3 | 2 | 5 | 4 | 2 | | 3 | 3 | 4 | 4 | 2 | 5 | 5 | 4 |
| | .25 | 1 | 1 | 1 | 1 | 1 | 1 | 1 | 1 | 1 | 1 | 5 | 2 | 1 | 3 | 1 | 1 | 1 | 3 | 1 | 2 | 3 | 1 | | 3 | 2 | 4 | 3 | 2 | 4 | 3 | 4 |
| | .13 | 2 | 1 | 2 | 1 | 1 | 1 | 1 | 2 | 1 | 1 | 2 | 1 | 1 | 1 | 1 | 1 | 1 | 1 | 1 | 1 | 2 | 1 | | 4 | 3 | 5 | 3 | 1 | 4 | 5 | 5 |
| | .06 | 1 | 1 | 1 | 1 | 1 | 1 | 1 | 1 | 1 | 1 | 2 | 1 | 2 | 1 | 1 | 1 | 1 | 1 | 1 | 1 | 2 | 2 | | 2 | 3 | 3 | 3 | 1 | 2 | 4 | 3 |

Table VI cont'd.

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 5 | 1.0 | 1 | 1 | 1 | 1 | 1 | 1 | 1 | 1 | 1 | 1 | 3 | 4 | 2 | 2 | 4 | 2 | 5 | 2 | 4 | 3 | | | 2 | 4 | | | 4 | | 4 | 4 | 4 |
| | .5 | 1 | 1 | 1 | 1 | 1 | 1 | 1 | 1 | 1 | 1 | 2 | 4 | 1 | 1 | 1 | 1 | 4 | 2 | 1 | 1 | | | 1 | 4 | | 3 | 3 | | 4 | 4 | 4 |
| | .25 | 1 | 1 | 1 | 1 | 1 | 1 | 1 | 1 | 1 | 1 | 1 | 1 | 1 | 1 | 1 | 1 | 1 | 1 | 1 | 1 | | | 1 | 3 | | 4 | 4 | | 4 | 4 | 4 |
| | .13 | | | | | | | | | | | | | | | | | | | | | | | 1 | 3 | | 4 | 3 | | 4 | 4 | 4 |
| | .06 | | | | | | | | | | | | | | | | | | | | | | | 1 | 1 | | 2 | 1 | | 2 | 2 | 4 |
| | .03 | | | | | | | | | | | | | | | | | | | | | | | 1 | 2 | | 3 | 2 | | 2 | 2 | 3 |
| | .016 | | | | | | | | | | | | | | | | | | | | | | | 1 | 3 | | 3 | 3 | | 4 | 4 | 4 |
| 6 | .5 | 1 | 2 | 1 | 1 | 1 | 2 | 1 | 1 | 1 | 1 | 3 | 3 | 2 | 5 | 4 | 1 | 3 | 2 | 1 | 2 | | | 2 | 3 | | 4 | 4 | | 4 | 3 | 4 |
| | .25 | 1 | 1 | 1 | 1 | 1 | 1 | 1 | 1 | 1 | 1 | 1 | 2 | 1 | 4 | 1 | 1 | 1 | 1 | 1 | 1 | | | 2 | 3 | | 4 | 4 | | 4 | 2 | 3 |
| | .13 | 1 | 1 | 1 | 1 | 1 | 1 | 1 | 1 | 1 | 1 | 1 | 1 | 1 | 3 | 1 | 1 | 1 | 1 | 1 | 1 | | | 1 | 3 | | 3 | 3 | | 3 | 3 | 4 |
| 7 | 1.0 | 1 | 1 | 3 | 4 | 5 | 3 | 3 | 5 | 4 | 4 | 4 | 4 | 4 | 5 | 5 | 3 | 5 | 4 | 5 | 5 | | | 2 | 4 | | 5 | 4 | | 4 | 4 | 4 |
| | .5 | 1 | 1 | 3 | 4 | 5 | 1 | 2 | 2 | 2 | 2 | 5 | 4 | 3 | 4 | 3 | 4 | 4 | 2 | 3 | 3 | | | 1 | 4 | | 4 | 3 | | 4 | 4 | 4 |
| | .25 | 1 | 1 | 1 | 1 | 2 | 3 | 1 | 2 | 1 | 2 | 2 | 3 | 3 | 2 | 3 | 1 | 1 | 1 | 3 | 1 | | | 1 | 3 | | 3 | 3 | | 5 | 4 | 4 |
| | .13 | | | | | | | | | | | | | | | | | | | | | | | 1 | 3 | | 4 | 2 | | 3 | 3 | 3 |
| | .06 | | | | | | | | | | | | | | | | | | | | | | | 1 | 2 | | 3 | 2 | | 1 | 2 | 2 |

Table VI cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 8 | 1.0 | 1 | 1 | 3 | 4 | 5 | 3 | 3 | 5 | 4 | 4 | 4 | 4 | 4 | 5 | 5 | 3 | 5 | 4 | 5 | 5 | | | 2 | 4 | | 5 | 4 | | 5 | 4 | 4 |
| | .5 | 1 | 1 | 3 | 4 | 5 | 1 | 2 | 2 | 2 | 2 | 5 | 4 | 3 | 4 | 3 | 4 | 4 | 2 | 3 | 3 | | | 1 | 4 | | 4 | 3 | | 4 | 4 | 4 |
| | .25 | 1 | 1 | 1 | 1 | 2 | 3 | 1 | 2 | 1 | 2 | 2 | 3 | 3 | 2 | 3 | 1 | 1 | 1 | 3 | 1 | | | 1 | 3 | | 3 | 3 | | 4 | 4 | 4 |
| | .25 | | | | | | | | | | | | | | | | | | | | | | | 3 | 3 | | 4 | 3 | | 3 | 3 | 3 |
| | .13 | | | | | | | | | | | | | | | | | | | | | | | 1 | 3 | | 4 | 2 | | 3 | 3 | 3 |
| | .06 | | | | | | | | | | | | | | | | | | | | | | | 1 | 2 | | 3 | 2 | | 1 | 2 | 2 |
| 9 | 1.0 | 1 | 1 | 1 | 1 | 2 | 3 | 1 | 3 | 1 | 1 | 3 | 3 | 2 | 1 | 3 | 1 | 1 | 3 | 2 | 2 | | | | | | | | | | | |
| | .5 | 1 | 1 | 1 | 1 | 1 | 1 | 1 | 1 | 1 | 1 | 3 | 2 | 2 | 2 | 2 | 1 | 1 | 2 | 1 | 1 | | | | | | | | | | | |
| | .25 | 1 | 1 | 1 | 1 | 1 | 1 | 1 | 1 | 1 | 1 | 1 | 1 | 1 | 1 | 1 | 1 | 1 | 1 | 1 | 1 | | | | | | | | | | | |
| 10 | 1.0 | 1 | 1 | 4 | 2 | 4 | 4 | 2 | 4 | 2 | 3 | 4 | 4 | 4 | 5 | 4 | 1 | 5 | 3 | 5 | 4 | | | 2 | 4 | | 5 | 4 | | 5 | 5 | 4 |
| | .5 | 1 | 1 | 3 | 1 | 2 | 4 | 1 | 1 | 1 | 1 | 4 | 4 | 3 | 4 | 1 | 1 | 3 | 1 | 2 | 3 | | | 1 | 4 | | 5 | 4 | | 4 | 4 | 4 |
| | .25 | 1 | 1 | 2 | 1 | 1 | 2 | 1 | 1 | 1 | 1 | 4 | 4 | 1 | 4 | 1 | 1 | 1 | 1 | 1 | 1 | | | 1 | 4 | | 4 | 4 | | 5 | 4 | 4 |
| | .25 | 1 | 1 | 1 | 1 | 1 | 1 | 1 | 1 | 1 | 1 | 1 | 1 | 1 | 3 | 2 | 1 | 1 | 1 | 1 | 1 | | | 1 | 3 | | 4 | 3 | | 3 | 3 | 4 |
| | .13 | 1 | 1 | 1 | 1 | 1 | 1 | 1 | 1 | 1 | 1 | 1 | 1 | 1 | 1 | 1 | 1 | 1 | 1 | 1 | 1 | | | 1 | 1 | | 3 | 2 | | 2 | 2 | 3 |
| | .06 | 1 | 1 | 1 | 1 | 1 | 1 | 1 | 1 | 1 | 1 | 1 | 1 | 1 | 1 | 1 | 1 | 1 | 1 | 1 | 1 | | | 1 | 2 | | 2 | 2 | | 3 | 3 | 4 |
| 12 | 1.0 | 1 | 2 | 3 | 1 | 3 | 3 | 1 | 2 | 2 | 2 | 5 | 4 | 2 | 3 | 3 | 1 | | 1 | 4 | 2 | | | 2 | 3 | | 5 | 3 | | 4 | 3 | 3 |
| | .5 | 1 | 2 | 3 | 1 | 1 | 3 | 1 | 3 | 1 | 1 | 4 | 4 | 1 | 3 | 1 | 1 | 1 | 1 | 1 | 2 | | | 2 | 3 | | 3 | 3 | | 3 | 4 | 3 |
| | .25 | 1 | 2 | 3 | 1 | 1 | 3 | 1 | 1 | 1 | 1 | 3 | 2 | 1 | 2 | 2 | 1 | | 1 | 1 | 1 | | | 1 | 2 | | 3 | 3 | | 3 | 3 | 3 |

## Table VI cont'd.

### PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 13 | 1.0 | 1 | 1 | 2 | 2 | 3 | 4 | 1 | 2 | 3 | 3 | 4 | 4 | 3 | 3 | 3 | 2 | 1 | 1 | 1 | 2 | | | 1 | 4 | | 5 | 3 | | 5 | 4 | 4 |
| | .5 | 1 | 1 | 2 | 1 | 2 | 2 | 2 | 2 | 4 | 2 | 3 | 2 | 2 | 4 | 1 | 1 | 1 | 1 | 2 | 3 | | | 2 | 3 | | 5 | 3 | | 4 | 4 | 4 |
| | .25 | 1 | 1 | 1 | 1 | 1 | 1 | 1 | 1 | 1 | 1 | 2 | 2 | 4 | 2 | 3 | 1 | 2 | 1 | 2 | 2 | | | 1 | 2 | | 4 | 3 | | 4 | 2 | 4 |
| | .25 | | | | | | | | | | | | | | | | | | | | | | | 1 | 3 | | 5 | 3 | | 4 | 4 | 4 |
| | .13 | | | | | | | | | | | | | | | | | | | | | | | 1 | 3 | | 4 | 2 | | 2 | 2 | 3 |
| | .06 | | | | | | | | | | | | | | | | | | | | | | | 1 | 1 | | 3 | 1 | | 1 | 1 | 2 |
| 15 | 1.0 | 1 | 1 | 1 | 1 | 1 | 1 | 1 | 1 | 1 | 1 | 5 | 1 | 1 | 1 | 1 | 1 | 1 | 1 | 1 | 1 | | | | | | | | | | | |
| | .5 | 1 | 1 | 1 | 1 | 1 | 1 | 1 | 1 | 1 | 1 | 4 | 1 | 1 | 1 | 1 | 1 | 1 | 1 | 1 | 1 | | | | | | | | | | | |
| | .25 | 1 | 1 | 1 | 1 | 1 | 1 | 1 | 1 | 1 | 1 | 4 | 1 | 1 | 1 | 1 | 1 | 1 | 1 | 1 | 1 | | | | | | | | | | | |
| 17 | 1.0 | 2 | 4 | 5 | 3 | 5 | 5 | 3 | 5 | 5 | 4 | 5 | 5 | 5 | 5 | 5 | 3 | 5 | 5 | 4 | 5 | | | 3 | 5 | | 5 | 4 | | 5 | 4 | 5 |
| | .5 | 1 | 2 | 5 | 2 | 5 | 4 | 2 | 5 | 3 | 3 | 5 | 5 | 5 | 5 | 5 | 2 | 5 | 5 | 4 | 5 | | | 2 | 5 | | 5 | 4 | | 5 | 4 | 5 |
| | .25 | 1 | 1 | 1 | 1 | 3 | 4 | 2 | 5 | 2 | 1 | 4 | 3 | 4 | 4 | 3 | 1 | 5 | 1 | 3 | 3 | | | 1 | 3 | | 5 | 3 | | 4 | 4 | 5 |
| | .25 | 1 | 1 | 2 | 3 | 3 | 4 | 2 | 4 | 2 | 1 | 5 | 4 | 3 | 4 | 1 | 3 | 4 | 1 | 1 | 2 | | | 1 | 3 | | 3 | 2 | | 4 | 3 | 5 |
| | .13 | 1 | 1 | 1 | 2 | 2 | 2 | 1 | 1 | 1 | 1 | 4 | 1 | 2 | 3 | 2 | 1 | 3 | 1 | 1 | 4 | | | 1 | 3 | | 4 | 2 | | 4 | 2 | 4 |
| | .06 | 1 | 1 | 1 | 1 | 1 | 1 | 1 | 2 | 1 | 1 | 1 | 1 | 1 | 1 | 1 | 1 | 1 | 1 | 1 | 3 | | | 1 | 2 | | 3 | 1 | | 3 | 1 | 2 |

## Table VI cont'd.

PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 18 | 1.0 | 1 | 1 | 1 | 1 | 2 | 1 | 1 | 2 | 1 | 1 | 4 | 4 | | 4 | 4 | 3 | 3 | 1 | 5 | 3 | 2 | 2 | | 4 | | 5 | 4 | 2 | 4 | 4 | 4 |
| | .5 | 1 | 1 | 1 | 2 | 3 | 1 | 1 | 3 | 1 | 2 | 1 | 3 | | 2 | 4 | 2 | 2 | 1 | 4 | 3 | 2 | 2 | | 4 | | 4 | 3 | 3 | 4 | 4 | 4 |
| | .25 | 1 | 1 | 1 | 1 | 1 | 1 | 1 | 1 | 1 | 1 | 1 | 2 | | 1 | 1 | 1 | 1 | 1 | 1 | 1 | 2 | 2 | | 2 | | 3 | 3 | 2 | 3 | 4 | 3 |
| 19 | 1.0 | 1 | 1 | 1 | 1 | 1 | 3 | 1 | 1 | 2 | 1 | 5 | 2 | 3 | 3 | 3 | 2 | 4 | 2 | 4 | 2 | | | | | | | | | | | |
| | .5 | 1 | 1 | 1 | 1 | 1 | 1 | 1 | 1 | 1 | 1 | 3 | 2 | 3 | 3 | 2 | 1 | 2 | 1 | 1 | 1 | | | | | | | | | | | |
| | .25 | 1 | 1 | 1 | 1 | 1 | 1 | 1 | 1 | 1 | 1 | 1 | 1 | 1 | 2 | 1 | 1 | 1 | 1 | 1 | 1 | | | | | | | | | | | |
| 20 | 1.0 | 1 | 1 | 2 | 2 | 2 | 4 | 3 | 5 | 2 | 4 | 5 | 4 | 4 | 5 | 5 | 1 | 4 | 3 | 4 | 5 | | | | | | | | | | | |
| | .5 | 1 | 1 | 1 | 1 | 1 | 1 | 1 | 2 | 2 | 3 | 5 | 3 | 4 | 4 | 4 | 2 | 4 | 2 | 4 | 4 | | | | | | | | | | | |
| | .25 | 1 | 1 | 1 | 1 | 1 | 1 | 1 | 1 | 1 | 1 | 1 | 3 | 3 | 4 | 1 | 1 | 2 | 1 | 1 | 1 | | | | | | | | | | | |
| 21 | 1.0 | 1 | 1 | 1 | 1 | 2 | 1 | 2 | 2 | 1 | 2 | 5 | 4 | 3 | 4 | 2 | 1 | 2 | 2 | 4 | 2 | | | | | | | | | | | |
| | .5 | 1 | 1 | 1 | 1 | 1 | 1 | 1 | 1 | 1 | 1 | 2 | 2 | 1 | 4 | 1 | 1 | 1 | 1 | 3 | 1 | | | | | | | | | | | |
| | .25 | 1 | 1 | 1 | 1 | 1 | 1 | 1 | 1 | 1 | 1 | 2 | 1 | 1 | 3 | 1 | 1 | 2 | 1 | 1 | 1 | | | | | | | | | | | |
| 22 | 1.0 | 1 | 1 | 1 | 1 | 4 | 4 | 2 | 2 | 1 | 2 | 5 | 2 | 3 | 5 | 4 | 2 | 4 | 3 | 4 | 5 | | | | | | | | | | | |
| | .5 | 1 | 1 | 1 | 1 | 1 | 2 | 2 | 1 | 1 | 1 | 5 | 2 | 3 | 4 | 2 | 1 | 1 | 3 | 1 | 3 | | | | | | | | | | | |
| | .25 | 1 | 1 | 1 | 1 | 1 | 1 | 1 | 1 | 1 | 1 | 5 | 3 | 4 | 5 | 2 | 2 | 2 | 3 | 2 | 2 | | | | | | | | | | | |
| | .25 | 2 | 1 | 1 | 4 | 1 | 2 | 1 | 1 | 2 | 1 | 3 | 1 | 1 | 3 | 1 | 1 | 1 | 1 | 2 | 1 | | | | | | | | | | | |
| | .13 | 1 | 1 | 1 | 1 | 1 | 1 | 1 | 1 | 1 | 1 | 1 | 1 | 1 | 1 | 1 | 1 | 1 | 1 | 1 | 1 | | | | | | | | | | | |
| | .06 | 1 | 1 | 1 | 1 | 1 | 1 | 1 | 1 | 1 | 1 | 3 | 1 | 1 | | 1 | 1 | 1 | 1 | 1 | 1 | | | | | | | | | | | |

Table VI cont'd.

PLANT SPECIES

| Compound of Example No. | appln. rate lbs/A | Pre-emergence Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 23 | 1.0 | 1 | 1 | 4 | 3 | 4 | 4 | 2 | 5 | 4 | 4 | 5 | 4 | 5 | 5 | 4 | 2 | 5 | 4 | 5 | 5 | 3 | 2 | | 4 | | 4 | 2 | 2 | 4 | 5 | 5 |
| | .5 | 1 | 1 | 2 | 1 | 2 | 4 | 2 | 3 | 1 | 1 | 5 | 4 | 4 | 4 | 4 | 3 | 4 | 4 | 5 | 3 | 3 | 1 | | 4 | | 4 | 2 | 2 | 3 | 4 | 4 |
| | .25 | 1 | 1 | 1 | 1 | 1 | 1 | 1 | 2 | 1 | 1 | 5 | 2 | 1 | 3 | 1 | 1 | 1 | 1 | 1 | 1 | 2 | 1 | | 3 | | 3 | 1 | 1 | 4 | 4 | 4 |
| | .25 | 1 | 1 | 1 | 1 | 2 | 3 | 2 | 3 | 1 | 2 | 5 | 1 | 2 | 2 | 3 | 1 | 1 | 2 | 4 | 1 | 2 | 2 | | 3 | 4 | 4 | 2 | 2 | 3 | 4 | 4 |
| | .13 | 1 | 1 | 1 | 1 | 2 | 1 | 2 | 2 | 1 | 1 | 4 | 1 | 1 | 1 | 1 | 1 | 1 | 2 | 2 | 1 | 1 | 2 | | 2 | 3 | 4 | 2 | 1 | 3 | 2 | 3 |
| | .06 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | | 2 | 2 | 3 | 2 | 1 | 2 | 2 | 2 |
| 24 | 1.0 | 4 | 5 | 3 | 2 | 4 | 5 | 4 | 5 | 3 | 3 | 5 | 5 | 5 | 5 | 5 | 4 | 5 | 3 | 5 | 5 | 4 | 1 | | 5 | | 4 | 4 | 5 | 4 | 4 | 5 |
| | .5 | 3 | 2 | 2 | 1 | 3 | 5 | 2 | 3 | 1 | 1 | 5 | 2 | 4 | 4 | 5 | 3 | 4 | 5 | 5 | 4 | 5 | 1 | | 4 | | 4 | 2 | 4 | 4 | 4 | 5 |
| | .25 | 1 | 1 | 2 | 1 | 3 | 3 | 1 | 2 | 1 | 1 | 5 | 3 | 1 | 4 | 2 | 2 | 4 | 2 | 3 | 4 | 3 | 1 | | 3 | | 2 | 2 | 3 | 3 | 4 | 5 |
| | .25 | 1 | 1 | 1 | 1 | 2 | 3 | 3 | 3 | 1 | 1 | 5 | 4 | 2 | 5 | 1 | 1 | 5 | 2 | 1 | 1 | 3 | 2 | | 3 | 4 | 4 | 4 | 3 | 4 | 4 | 4 |
| | .13 | 1 | 1 | 1 | 2 | 1 | 1 | 2 | 2 | 1 | 1 | 1 | 4 | 1 | 3 | 1 | 1 | 1 | 1 | 1 | 1 | 2 | 2 | | 2 | 4 | 4 | 2 | 2 | 4 | 3 | 4 |
| | .06 | 1 | 1 | 1 | 2 | 1 | 3 | 2 | 1 | 1 | 1 | 1 | 1 | 1 | 1 | 1 | 1 | 1 | 1 | 1 | 1 | 1 | 1 | | 2 | 3 | 4 | 2 | 1 | 3 | 2 | 3 |
| 25 | 1.0 | 1 | 1 | 1 | 1 | 2 | 2 | 1 | 2 | 1 | 1 | 5 | 3 | 3 | 3 | 2 | 1 | 3 | 1 | 1 | 2 | | | | | | | | | | | |
| | .5 | 1 | 1 | 1 | 1 | 1 | 1 | 1 | 1 | 1 | 1 | 4 | 2 | 1 | 2 | 1 | 1 | 1 | 1 | 1 | 1 | | | | | | | | | | | |
| | .25 | 1 | 1 | 1 | 1 | 1 | 1 | 1 | 1 | 1 | 1 | 4 | 1 | 1 | | 1 | 1 | 1 | 1 | 1 | 1 | | | | | | | | | | | |
| 26 | 1.0 | 1 | 1 | 2 | 1 | 2 | 4 | 2 | 3 | 2 | 2 | 5 | 4 | 4 | 5 | 4 | 2 | 4 | 3 | 5 | 5 | | | | | | | | | | | |
| | .5 | 1 | 1 | 1 | 1 | 2 | 2 | 1 | 3 | 2 | 2 | 4 | 4 | 3 | 3 | 4 | 1 | 2 | 1 | 5 | 2 | | | | | | | | | | | |
| | .25 | 1 | 1 | 1 | 1 | 1 | 1 | 1 | 1 | 1 | 1 | 2 | 3 | 2 | 4 | 1 | 1 | 1 | 1 | 3 | 1 | | | | | | | | | | | |

Table VI cont'd.

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 27 | 1.0 | 1 | 1 | 1 | 1 | 1 | 2 | 1 | 1 | 1 | 1 | 4 | 3 | 4 | 3 | 1 | 1 | 1 | 1 | 1 | 2 | | | | | | | | | | | |
| | .5 | 1 | 1 | 1 | 1 | 1 | 1 | 1 | 1 | 1 | 1 | 4 | 2 | 1 | 4 | 1 | 1 | 2 | 1 | 1 | 1 | | | | | | | | | | | |
| | .25 | 1 | 1 | 1 | 1 | 1 | 1 | 1 | 1 | 1 | 1 | 1 | 1 | 1 | 2 | 1 | 1 | 1 | 2 | 2 | 1 | | | | | | | | | | | |
| 28 | 1.0 | 1 | 1 | 1 | 1 | 1 | 2 | 2 | 1 | 4 | 2 | 4 | 2 | 1 | 2 | 2 | 2 | 1 | 1 | 2 | 2 | | | | | | | | | | | |
| | .5 | 1 | 1 | 1 | 1 | 1 | 3 | 3 | 2 | 1 | 1 | 4 | 3 | 1 | 2 | 1 | 1 | 2 | 1 | 2 | 1 | | | | | | | | | | | |
| | .25 | 1 | 1 | 1 | 1 | 1 | 2 | 1 | 2 | 1 | 1 | 2 | 2 | 1 | 1 | 1 | 2 | 1 | 1 | 2 | 1 | | | | | | | | | | | |
| 29 | 1.0 | 1 | 1 | 2 | 1 | 2 | 1 | 2 | 2 | 3 | 4 | 5 | 4 | 2 | 4 | 1 | 2 | 4 | 2 | 1 | 1 | 4 | 2 | | 4 | 5 | 3 | 3 | 4 | 4 | 4 | 4 |
| | .5 | 1 | 1 | 1 | 1 | 1 | 2 | 1 | 2 | 1 | 2 | 4 | 4 | 1 | 3 | 1 | 1 | 2 | 1 | 1 | 1 | 4 | 2 | | 2 | 4 | 3 | 2 | 3 | 3 | 4 | 3 |
| | .25 | 1 | 1 | 1 | 1 | 1 | 1 | 1 | 1 | 1 | 1 | 4 | 1 | 1 | 1 | 1 | 1 | 2 | 2 | 1 | 1 | 2 | 1 | | 2 | 3 | 3 | 2 | 2 | 4 | 3 | 3 |
| 34 | 1.0 | 1 | 1 | 1 | 2 | 1 | 4 | 2 | 1 | 2 | 2 | 5 | 2 | 3 | 4 | 3 | 1 | 3 | 1 | 3 | 3 | | | | | | | | | | | |
| | .5 | 1 | 1 | 1 | 2 | 1 | 2 | 1 | 1 | 1 | 2 | 4 | 1 | 3 | 3 | 2 | 1 | 3 | 1 | 1 | 4 | | | | | | | | | | | |
| | .25 | 1 | 1 | 1 | 2 | 1 | 1 | 1 | 1 | 1 | 2 | 4 | 1 | 1 | 1 | 1 | 1 | 3 | 1 | 1 | 1 | | | | | | | | | | | |

Table VI cont'd.

PLANT SPECIES

Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 35 | 1.0 | 1 | 1 | 2 | 1 | 2 | 4 | 3 | 2 | 4 | 4 | 5 | 4 | 4 | 5 | 5 | 1 | 4 | 3 | 3 | 4 |
| | .5 | 1 | 2 | 1 | 1 | 2 | 5 | 3 | 2 | 4 | 5 | 5 | 1 | 4 | 3 | 4 | 1 | 4 | 1 | 2 | 3 |
| | .25 | 1 | 1 | 1 | 1 | 1 | 1 | 1 | 1 | 1 | 1 | 4 | 1 | 3 | 3 | 4 | 1 | 3 | 1 | 1 | 1 |
| | .25 | 1 | 1 | 1 | 1 | 1 | 3 | 1 | 1 | 2 | 1 | 4 | 2 | 3 | 3 | 2 | 2 | 4 | 2 | 1 | 2 |
| | .13 | 1 | 1 | 4 | 2 | 1 | 4 | 1 | 1 | 1 | 2 | 5 | 2 | 3 | 2 | 3 | 1 | 2 | 1 | 2 | 2 |
| | .06 | 1 | 1 | 1 | 1 | 1 | 1 | 1 | 1 | 1 | 2 | 2 | 1 | 2 | 1 | 1 | 1 | 1 | 1 | 1 | 1 |
| 37 | 1.0 | 1 | 1 | 2 | 2 | 3 | 2 | 1 | 2 | 2 | 2 | 4 | 3 | 2 | 4 | 3 | 2 | 3 | 2 | 2 | 3 |
| | .5 | 1 | 1 | 2 | 2 | 1 | 3 | 1 | 2 | 2 | 2 | 4 | 2 | 2 | 2 | 2 | 1 | 2 | 2 | 2 | 4 |
| | .25 | 1 | 1 | 2 | 1 | 1 | 1 | 1 | 1 | 1 | 1 | 3 | 1 | 1 | 1 | 1 | 1 | 1 | 1 | 1 | 1 |
| 38 | 1.0 | 1 | 1 | 2 | | 1 | 4 | 2 | 2 | 2 | 2 | 5 | 3 | 3 | 3 | 5 | 1 | 2 | 2 | 1 | 2 |
| | .5 | 1 | 2 | 2 | | 1 | 2 | 1 | 1 | 2 | 1 | 5 | 3 | 2 | 3 | 3 | 1 | 2 | 1 | 2 | 2 |
| | .25 | 1 | 1 | 1 | | 1 | 2 | 1 | 1 | 1 | 1 | 5 | 1 | 1 | 2 | 1 | 1 | 1 | 1 | 1 | 1 |
| 39 | 1.0 | 1 | 1 | 1 | 1 | 1 | 1 | 1 | 1 | 1 | 1 | 4 | 1 | 1 | 1 | 4 | 1 | 2 | 1 | 1 | 1 |
| | .5 | 1 | 1 | 1 | 1 | 1 | 1 | 1 | 1 | 1 | 1 | 4 | 1 | 1 | 2 | 4 | 1 | 1 | 1 | 1 | 1 |
| | .25 | 1 | 1 | 1 | 1 | 1 | 1 | 1 | 1 | 1 | 1 | 4 | 1 | 2 | 1 | 3 | 1 | 1 | 1 | 1 | 1 |

Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 35 | 1.0 | 1 | 2 | | 2 | 3 | 2 | 2 | 2 | 2 | 4 | 3 |
| | .5 | 1 | 3 | | 2 | 3 | 2 | 1 | 1 | 2 | 2 | 2 |
| | .25 | 1 | 1 | | 1 | 2 | 2 | 1 | 1 | 2 | 2 | 2 |
| | .25 | | | | | | | | | | | |
| | .13 | | | | | | | | | | | |
| | .06 | | | | | | | | | | | |
| 37 | 1.0 | | | | | | | | | | | |
| | .5 | | | | | | | | | | | |
| | .25 | | | | | | | | | | | |
| 38 | 1.0 | 4 | 1 | | 4 | 4 | 5 | 4 | 1 | 4 | 4 | 3 |
| | .5 | 2 | 1 | | 2 | 2 | 4 | 3 | 1 | 3 | 4 | 4 |
| | .25 | 3 | 1 | | 1 | 3 | 4 | 1 | 1 | 1 | 2 | 2 |
| 39 | 1.0 | 4 | 1 | | 4 | 4 | 5 | 3 | 1 | 5 | 4 | 4 |
| | .5 | 2 | 1 | | 2 | 2 | 4 | 2 | 1 | 2 | 5 | 2 |
| | .25 | 1 | 1 | | 2 | 2 | 1 | 1 | 1 | 1 | 2 | 2 |

Table VI cont'd.

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 40 | 1.0 | 2 | 2 | 3 | 2 | 4 | 4 | 2 | 4 | 3 | 3 | 5 | 5 | 4 | 5 | 4 | 3 | 4 | 3 | 5 | 5 | 5 | 3 | | 4 | 4 | 5 | 4 | 2 | 4 | 4 | 5 |
| | .5 | 2 | 3 | 1 | 2 | 3 | 4 | 2 | 3 | 1 | 1 | 4 | 4 | 3 | 5 | 4 | 2 | 3 | 2 | 5 | 3 | 4 | 2 | | 3 | 4 | 5 | 3 | 2 | 5 | 4 | 4 |
| | .25 | 2 | 2 | 1 | 1 | 2 | 3 | 1 | 1 | 1 | 1 | 4 | 3 | 3 | 3 | 4 | 1 | 2 | 1 | 1 | 2 | 4 | 2 | | 2 | 2 | 5 | 4 | 1 | 4 | 4 | 3 |
| | .25 | 1 | 1 | 1 | 1 | 3 | 2 | 2 | 2 | 1 | 1 | 5 | 2 | 2 | 2 | 2 | 1 | 1 | 2 | 2 | 3 | 3 | 2 | | 2 | 4 | 5 | 4 | 1 | 4 | 3 | 4 |
| | .13 | 1 | 1 | 1 | 1 | 1 | 1 | 2 | 2 | 1 | 1 | 2 | 1 | 1 | 2 | 1 | 1 | 1 | 1 | 1 | 1 | 3 | 1 | | 2 | 3 | 4 | 3 | 1 | 3 | 4 | 5 |
| | .06 | 1 | 1 | 1 | 1 | 1 | 1 | 1 | 1 | 1 | 1 | 1 | 1 | 1 | 1 | 1 | 1 | 1 | 1 | 1 | 1 | 1 | 1 | | 1 | 2 | 4 | 2 | 1 | 1 | 3 | 3 |
| 41 | 1.0 | 1 | 1 | 4 | 4 | 4 | 4 | 3 | 5 | 5 | 4 | 5 | 4 | 4 | 5 | 3 | 4 | 5 | 3 | 4 | 5 | 4 | 3 | | 4 | 4 | 5 | 4 | 3 | 5 | 4 | 5 |
| | .5 | 1 | 1 | 2 | 3 | 4 | 4 | 3 | 5 | 5 | 3 | 5 | 4 | 5 | 5 | 5 | 4 | 5 | 3 | 5 | 5 | 4 | 3 | | 4 | 4 | 5 | 4 | 3 | 5 | 4 | 4 |
| | .25 | 1 | 1 | 1 | 2 | 3 | 4 | 2 | 3 | 4 | 3 | 5 | 4 | 5 | 5 | 4 | 2 | 5 | 3 | 4 | 5 | 4 | 1 | | 3 | 4 | 5 | 4 | 2 | 5 | 3 | 4 |
| | .25 | 2 | 2 | 2 | 3 | 3 | 2 | 1 | 2 | 1 | 2 | 4 | 4 | 2 | 4 | 2 | 3 | 4 | 2 | 4 | 3 | 4 | 3 | | 4 | 4 | 5 | 4 | 2 | 4 | 4 | 3 |
| | .13 | 4 | 4 | 3 | 2 | 3 | 4 | 4 | 4 | 4 | 3 | 5 | 4 | 5 | 4 | 4 | 4 | 3 | 5 | 4 | 4 | 3 | 2 | | 4 | 3 | 5 | 4 | 2 | 4 | 3 | 4 |
| | .06 | 3 | 3 | 2 | 3 | 2 | 3 | 2 | 4 | 3 | 2 | 4 | 4 | 4 | 3 | 4 | 3 | 4 | 5 | 4 | 4 | 3 | 1 | | 4 | 3 | 5 | 3 | 1 | 4 | 3 | 4 |
| | .06 | 1 | 1 | 1 | 1 | 1 | 1 | 1 | 1 | 1 | 1 | 2 | 1 | 2 | 2 | 1 | 1 | 1 | 1 | 2 | 1 | 2 | 1 | | 1 | 3 | 3 | 2 | 1 | 3 | 2 | 2 |
| | .03 | 1 | 1 | 1 | 1 | 1 | 1 | 1 | 1 | 1 | 1 | 3 | 2 | 2 | 2 | 2 | 1 | 1 | 1 | 1 | 1 | 2 | 1 | | 3 | 4 | 5 | 2 | 1 | 4 | 2 | 2 |
| | .016 | 1 | 1 | 1 | 1 | 1 | 1 | 2 | 1 | 1 | 1 | 1 | 1 | 1 | 1 | 1 | 1 | 1 | 1 | 1 | 1 | 1 | 1 | | 1 | 2 | 3 | 2 | 1 | 1 | 1 | 1 |

## Table VI cont'd.

### PLANT SPECIES

| Compound of Example No. | appln. rate lbs/A | Pre-emergence: Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Post-emergence: Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|---|---|---|---|---|---|---|---|---|---|---|---|---|---|---|---|---|---|---|---|
| 44 | 1.0 | 1 | 1 | 1 | 1 | 1 | 1 | 1 | 1 | 1 | 1 | 3 | 1 | 1 | 4 | 1 | 1 | 1 | 1 | 1 | 1 | | | 1 | 3 | | 4 | 3 | | 4 | 3 | 4 |
| | .5 | 1 | 1 | 1 | 1 | 1 | 1 | 1 | 1 | 1 | | 4 | 1 | 1 | 2 | 1 | 1 | 1 | 1 | 1 | 1 | | | 1 | 2 | | 4 | 2 | | 3 | 2 | 3 |
| | .25 | 1 | 2 | 2 | 1 | 1 | 1 | 1 | 1 | 1 | 1 | 1 | 1 | 1 | 1 | 1 | 1 | 1 | 1 | 1 | 1 | | | 1 | 1 | | 3 | 2 | | 3 | 1 | 3 |
| 45 | 1.0 | 1 | 4 | 3 | 2 | 4 | 4 | 2 | 5 | 1 | 2 | 5 | 5 | 5 | 4 | 5 | 4 | 5 | 5 | 5 | 5 | 4 | 3 | | 3 | 5 | 4 | 3 | 2 | 4 | 4 | 4 |
| | .5 | 1 | 1 | 1 | 1 | 1 | 4 | | 5 | 1 | 2 | 4 | 5 | 4 | 5 | 5 | 2 | 5 | 1 | 3 | 5 | 2 | 2 | | 2 | 4 | 4 | 3 | 3 | 4 | 4 | 4 |
| | .25 | 1 | 1 | 1 | 1 | 1 | 1 | 1 | 2 | 1 | 1 | 4 | 4 | 3 | 4 | 2 | 1 | 3 | 1 | 2 | 1 | 2 | 1 | | 2 | 3 | 4 | 2 | 1 | 3 | 3 | 2 |
| 46 | 1.0 | 1 | 1 | 2 | 1 | 1 | 3 | 1 | 3 | 1 | 1 | | 4 | 4 | 5 | 3 | 2 | 5 | 2 | 3 | 4 | 4 | 3 | | 4 | 5 | 5 | 5 | 3 | 4 | 5 | 5 |
| | .5 | 1 | 1 | 2 | 1 | 4 | 3 | 1 | 3 | 1 | 1 | | 5 | 5 | 5 | 3 | 2 | 4 | 3 | 4 | 3 | 3 | 3 | | 4 | 5 | 5 | 4 | 2 | 5 | 5 | 5 |
| | .25 | 1 | 1 | 1 | 1 | 3 | 3 | 1 | 1 | 1 | 1 | | 4 | 1 | 5 | 1 | 1 | 1 | 1 | 4 | 1 | 2 | 2 | | 3 | 4 | 4 | 3 | 2 | 4 | 4 | 5 |
| 47 | 1.0 | 1 | 1 | 1 | 1 | 3 | 4 | 2 | 3 | 3 | 2 | 5 | 4 | 3 | 4 | 3 | 2 | 4 | 3 | 5 | 4 | 4 | 2 | | 3 | | 5 | 3 | 2 | 3 | 4 | 4 |
| | .5 | 3 | 2 | 2 | 1 | 3 | 5 | 3 | 3 | 2 | 2 | 5 | 4 | 4 | 4 | 3 | 2 | 3 | 3 | 3 | 2 | 3 | 2 | | 2 | | 3 | 2 | 2 | 3 | 4 | 3 |
| | .25 | 3 | 1 | 1 | 1 | 1 | 4 | 3 | 3 | 2 | 3 | 5 | 2 | 2 | 3 | 1 | 2 | 3 | 1 | 3 | 1 | 2 | 1 | | 2 | | 3 | 2 | 1 | 3 | 4 | 3 |
| | .25 | 1 | 1 | 1 | 2 | 2 | 2 | 2 | 3 | 1 | 1 | 4 | 2 | 1 | 4 | 1 | 1 | 2 | 2 | 2 | 2 | | | | | | | | | | | |
| | .13 | 1 | 1 | 2 | 3 | 1 | 2 | 1 | 2 | 1 | 1 | 1 | 3 | 1 | 2 | 2 | 1 | 1 | 1 | 1 | 1 | | | | | | | | | | | |
| | .06 | 1 | 1 | 1 | 1 | 1 | 1 | 1 | 1 | 1 | 1 | 1 | 1 | 1 | 1 | 1 | 1 | 1 | 1 | 1 | 1 | | | | | | | | | | | |

Table VI cont'd.

PLANT SPECIES

| Compound of Example No. | appln. rate lbs/A | Pre-emergence | | | | | | | | | | | | | | | | | | | | Post-emergence | | | | | | | | | | |
|---|---|---|---|---|---|---|---|---|---|---|---|---|---|---|---|---|---|---|---|---|---|---|---|---|---|---|---|---|---|---|---|---|
| | | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia | Tomato | Barnyard Grass | Corn | Large Crabgrass | Mustard | Pigweed | Foxtail | Wild Oats | Velvetleaf | Morningglory | Zinnia |
| 49 | 1.0 | 2 | 1 | 2 | 2 | 3 | 3 | 1 | 1 | 1 | 1 | 5 | 4 | 4 | 4 | 2 | 2 | 5 | 4 | 4 | 4 | | | 3 | 4 | | 5 | 3 | | 3 | 3 | 4 |
| | .5 | 1 | 1 | 2 | 1 | 2 | 4 | 1 | 2 | 1 | 1 | 3 | 3 | 1 | 1 | 1 | 2 | 1 | 1 | 1 | 2 | | | 2 | 4 | | 4 | 3 | | 4 | 4 | 4 |
| | .25 | 1 | 1 | 4 | 1 | 2 | 3 | 1 | 2 | 1 | 1 | 3 | 1 | 1 | 1 | 1 | 1 | 1 | 1 | 1 | 2 | | | 2 | 4 | | 4 | 3 | | 4 | 3 | 4 |
| | .25 | | | | | | | | | | | | | | | | | | | | | | | 1 | 2 | | 3 | 2 | | 4 | 4 | 4 |
| | .13 | | | | | | | | | | | | | | | | | | | | | | | 1 | 2 | | 2 | 1 | | 3 | 2 | 4 |
| | .06 | | | | | | | | | | | | | | | | | | | | | | | 2 | 1 | | 1 | 1 | | 3 | 2 | 3 |
| 51 | 1.0 | 1 | 1 | 2 | 1 | 2 | 2 | 2 | 2 | 1 | 1 | 5 | 2 | 2 | 4 | 4 | 1 | 1 | 1 | 1 | 1 | | | | | | | | | | | |
| | .5 | 1 | 1 | 2 | 1 | 1 | 1 | 1 | 1 | 1 | 1 | 3 | 3 | 1 | 3 | 2 | 1 | 2 | 1 | 1 | 1 | | | | | | | | | | | |
| | .25 | 1 | 1 | 2 | 1 | 2 | 1 | 2 | 2 | 1 | 1 | 4 | 2 | 2 | 4 | 2 | 2 | 2 | 1 | 2 | 1 | | | | | | | | | | | |
| | .25 | 1 | 1 | 1 | 1 | 1 | 1 | 1 | 1 | 1 | 1 | 2 | 1 | 1 | 1 | 1 | 1 | 1 | 1 | 2 | 1 | | | | | | | | | | | |
| | .13 | 1 | 1 | 1 | 1 | 1 | 1 | 1 | 1 | 1 | 1 | 1 | 1 | 1 | 1 | 1 | 1 | 1 | 1 | 1 | 1 | | | | | | | | | | | |
| | .06 | 1 | 1 | 1 | 1 | 1 | 1 | 1 | 1 | 1 | 1 | 1 | 1 | 1 | 1 | 1 | 1 | 1 | 1 | 1 | 1 | | | | | | | | | | | |
| 54 | 1.0 | 1 | 1 | 1 | 1 | 1 | 1 | 1 | 1 | 1 | 1 | 4 | 2 | 3 | 3 | 2 | 1 | 1 | 1 | 1 | 1 | | | | | | | | | | | |
| | .5 | 1 | 1 | 1 | 1 | 1 | 1 | 1 | 1 | 1 | 1 | 3 | 1 | 2 | 3 | 1 | 1 | 1 | 1 | 1 | 1 | | | | | | | | | | | |
| | .25 | 1 | 1 | 1 | 1 | 1 | 1 | 1 | 1 | 1 | 1 | 2 | 1 | 2 | 1 | 1 | 1 | 1 | 1 | 1 | 1 | | | | | | | | | | | |

Table VI cont'd.

PLANT SPECIES

Pre-emergence

| Compound of Example No. | appln. rate lbs/A | Corn | Cotton | Soybean | Wheat | Alfalfa | Sugar Beet | Rice | Cucumber | Tomato | Barnyard Grass | Lambsquarter | Large Crabgrass | Mustard | Pigweed | Foxtail | Wild Oats | Velvetleaf | Jimsonweed | Morningglory | Zinnia |
|---|---|---|---|---|---|---|---|---|---|---|---|---|---|---|---|---|---|---|---|---|---|
| 55 | 1.0 | 3 | 3 | 3 | 4 | 5 | 5 | 4 | 5 | 4 | 4 | 5 | 5 | 5 | 5 | 5 | 4 | 5 | 5 | 5 | 5 |
| | .5 | 3 | 3 | 3 | 3 | 5 | 5 | 3 | 5 | 4 | 5 | 5 | 5 | 5 | 5 | 5 | 4 | 5 | 5 | 5 | 5 |
| | .25 | 1 | 3 | 2 | 1 | 4 | 5 | 2 | 5 | 4 | 4 | 5 | 4 | 4 | 5 | 4 | 1 | 5 | 2 | 2 | 4 |
| | .25 | 1 | 1 | 4 | 2 | 3 | 4 | 1 | 4 | 4 | 3 | 5 | 4 | 3 | 4 | 2 | 1 | 4 | 2 | 2 | 3 |
| | .13 | 1 | 1 | 2 | 1 | 1 | 2 | 1 | 2 | 2 | 3 | 4 | 3 | 2 | 3 | 2 | 1 | 3 | 2 | 2 | 2 |
| | .06 | 2 | 1 | 2 | 2 | 1 | 1 | 1 | 2 | 1 | 2 | 2 | 2 | 2 | 1 | 1 | 1 | 2 | 1 | 2 | 1 |
| | .06 | | | | | | | | | | | | | | | | | | | | |
| | .03 | | | | | | | | | | | | | | | | | | | | |
| | .016 | | | | | | | | | | | | | | | | | | | | |

Post-emergence

| Compound of Example No. | appln. rate lbs/A | Tomato | Barnyard Grass | Corn | Large Crabgrass | Mustard | Pigweed | Foxtail | Wild Oats | Velvetleaf | Morningglory | Zinnia |
|---|---|---|---|---|---|---|---|---|---|---|---|---|
| 55 | 1.0 | 4 | 4 | | 5 | 4 | 5 | 4 | 4 | 5 | 5 | 5 |
| | .5 | 4 | 2 | | 2 | 4 | 3 | 4 | 3 | 4 | 5 | 4 |
| | .25 | 3 | 1 | | 4 | 4 | 3 | 4 | 2 | 4 | 4 | 4 |
| | .25 | 4 | 2 | | 4 | 4 | 4 | 3 | 3 | 5 | 4 | 5 |
| | .13 | 3 | 2 | | 3 | 3 | 4 | 3 | 1 | 4 | 4 | 5 |
| | .06 | 3 | 2 | | 3 | 3 | 3 | 3 | 1 | 4 | 3 | 4 |
| | .06 | 3 | 2 | | 3 | 3 | 3 | 3 | 1 | 4 | 3 | 3 |
| | .03 | 2 | 2 | | 2 | 2 | 2 | 2 | 1 | 2 | 3 | 3 |
| | .016 | 3 | 2 | | 3 | 3 | 3 | 3 | 1 | 4 | 3 | 3 |

The data generated indicates that compounds embraced by formula IX are useful as terrestrial herbicides against common weed varieties which are found in desirable crops such as corn, wheat, soybean, and the like.

Because of the potent terrestrial herbicidal activity possessed by the isothiazolylureas of formula IX, they are useful in the elimination and control of the growth of unwanted terrestrial vegetation. This can be carried out by applying the herbicidal compounds by any of several art-recognized methods. The compounds can be surface applied to soil prior to planting of crops or emergence of seedlings. If desired, the surface-applied herbicide can be incorporated into the soil by conventional means. The compounds can alternatively be applied early postemergence, for instance within about 2 weeks following seedling emergence.

The amount of herbicidal isothiazolylureas of formula IX to be employed is an amount which is effective in controlling the growth of unwanted vegetation. Such herbicidal amount will depend upon a number of factors, including the method of application, formulation, soil texture, soil moisture content, the expected population of unwanted vegetation, degree of incorporation, the extent of growth control desired, and related factors. The rate of application normally will be from about 0.01 to about 10.0 pounds per acre, and preferably from about 0.25 to about 5.0 pounds per acre. (These ranges are equivalent, respectively, to from about 0.011 to about 11.2 kilograms per hectare, and from about 0.28 to about 5.6 kilograms per hectare.)

Various compounds of formula IX were also surface applied, preemergence, or over the surface applied, post-emergence to assorted crops and weeds. The compounds were formulated as wettable powders (50W) and the formulations described in the Formulations section hereinafter. Plant injury ratings were made visually on a scale of 0-10 with 0 being no injury and 10 being plant death. The injury rating was multiplied by 10 to obtaint the percent inhibition. Three replications were performed at each rate and the average percent inhibition was recorded. The results of the tests are reported in Tables VII and VIII.

Table VII

SURFACE APPLIED - PREEMERGENCE

PERCENT INHIBITION - DAYS AFTER TREATMENT

| Compound of Example No. | Appln. Rate lb/A | Sorghum 19 | Sorghum 33 | Corn 19 | Corn 33 | Velvetleaf 19 | Velvetleaf 33 | Pigweed 19 | Pigweed 33 | Morningglory 19 | Morningglory 33 | Foxtail 19 | Foxtail 33 |
|---|---|---|---|---|---|---|---|---|---|---|---|---|---|
| 8 | 0.25 | 0.0 | 0.0 | 0.0 | 0.0 | 36.7 | 0.0 | 86.7 | 53.3 | 36.7 | 0.0 | 43.3 | 6.7 |
| | 0.5 | 10.0 | 0.0 | 0.0 | 0.0 | 80.0 | 40.0 | 90.0 | 76.7 | 33.3 | 10.0 | 78.3 | 40.0 |
| | 1.0 | 10.0 | 0.0 | 3.3 | 0.0 | 93.3 | 80.0 | 100.0 | 96.7 | 80.0 | 65.0 | 91.7 | 76.7 |
| | 2.0 | 16.7 | 20.0 | 0.0 | 3.3 | 95.0 | 97.7 | 100.0 | 99.7 | 93.3 | 88.3 | 98.3 | 92.0 |
| 10 | 0.25 | 6.7 | 0.0 | 0.0 | 0.0 | 33.3 | 0.0 | 96.7 | 88.3 | 0.0 | 0.0 | 36.7 | 0.0 |
| | 0.5 | 0.0 | 0.0 | 0.0 | 0.0 | 6.7 | 0.0 | 86.7 | 53.3 | 16.7 | 0.0 | 26.7 | 0.0 |
| | 1.0 | 13.3 | 3.3 | 0.0 | 0.0 | 46.7 | 23.3 | 100.0 | 100.0 | 46.7 | 30.0 | 70.0 | 46.7 |
| | 2.0 | 16.7 | 16.7 | 0.0 | 0.0 | 95.0 | 83.3 | 100.0 | 100.0 | 86.7 | 80.0 | 85.0 | 66.7 |
| 11 | 0.25 | 0.0 | 0.0 | 0.0 | 0.0 | 23.3 | 16.7 | 46.7 | 43.3 | 0.0 | 0.0 | 26.7 | 6.7 |
| | 0.5 | 3.3 | 0.0 | 5.0 | 0.0 | 10.0 | 0.0 | 78.3 | 16.7 | 0.0 | 0.0 | 16.7 | 0.0 |
| | 1.0 | 6.7 | 0.0 | 6.7 | 0.0 | 13.3 | 0.0 | 93.3 | 76.7 | 30.0 | 0.0 | 23.3 | 10.0 |
| | 2.0 | 0.0 | 0.0 | 0.0 | 0.0 | 33.3 | 0.0 | 100.0 | 95.0 | 65.0 | 26.7 | 66.7 | 36.7 |
| 13 | 0.25 | 0.0 | 0.0 | 0.0 | 0.0 | 26.7 | 0.0 | 70.0 | 83.3 | 23.3 | 0.0 | 36.7 | 0.0 |
| | 0.5 | 6.7 | 0.0 | 0.0 | 0.0 | 46.7 | 10.0 | 93.3 | 85.0 | 20.0 | 0.0 | 40.0 | 10.0 |
| | 1.0 | 10.0 | 0.0 | 0.0 | 0.0 | 86.7 | 46.7 | 100.0 | 93.3 | 83.3 | 33.3 | 66.7 | 40.0 |
| | 2.0 | 10.0 | 10.0 | 0.0 | 3.3 | 95.0 | 91.7 | 100.0 | 100.0 | 85.0 | 91.7 | 86.7 | 65.0 |

Table VII

SURFACE APPLIED - PREEMERGENCE

PERCENT INHIBITION - DAYS AFTER TREATMENT

| Compound of Example No. | Appln. Rate lb/A | Sorghum | | Corn | | Velvetleaf | | Pigweed | | Morningglory | | Foxtail | |
|---|---|---|---|---|---|---|---|---|---|---|---|---|---|
| | | 19 | 33 | 19 | 33 | 19 | 33 | 19 | 33 | 19 | 33 | 19 | 33 |
| 17 | 0.25 | 6.7 | 3.3 | 5.0 | 0.0 | 86.7 | 56.7 | 100.0 | 93.3 | 20.0 | 16.7 | 73.3 | 46.7 |
| | 0.5 | 0.0 | 0.0 | 0.0 | 0.0 | 91.7 | 86.7 | 100.0 | 100.0 | 75.0 | 63.3 | 94.3 | 66.7 |
| | 1.0 | 23.3 | 23.3 | 0.0 | 0.0 | 97.7 | 98.3 | 100.0 | 100.0 | 85.0 | 86.7 | 100.0 | 96.7 |
| | 2.0 | 60.0 | 60.0 | 71.7 | 73.3 | 100.0 | 100.0 | 100.0 | 100.0 | 95.0 | 99.3 | 100.0 | 100.0 |
| Control | 0.0 | 0.0. | 0.0 | 0.0 | 0.0 | 0.0 | 0.0 | 0.0 | 0.0 | 0.0 | 0.0 | 0.0 | 0.0 |

## Table VIII

OVER THE SURFACE APPLIED - POSTEMERGENCE

PERCENT INHIBITION - DAYS AFTER TREATMENT

| Compound of Example No. | Appln. Rate lb/A | Sorghum | | Corn | | Velvetleaf | | Pigweed | | Morningglory | | Ladys-thumb | Foxtail Millet | |
|---|---|---|---|---|---|---|---|---|---|---|---|---|---|---|
| | | 7 | 15 | 7 | 15 | 7 | 15 | 7 | 15 | 7 | 15 | 15 | 7 | 15 |
| 7 | 0.125 | 0.0 | 0.0 | 0.0 | 0.0 | 0.0 | 80.0 | 100.0 | 98.3 | 70.0 | 48.3 | 33.3 | 10.0 | 16.7 |
| | 0.25 | 0.0 | 0.0 | 6.7 | 0.0 | 36.7 | 95.0 | 100.0 | 100.0 | 76.7 | 63.3 | 50.0 | 30.0 | 33.3 |
| | 0.5 | 0.0 | 3.3 | 0.0 | 0.0 | 83.3 | 90.0 | 100.0 | 100.0 | 85.0 | 73.3 | 53.3 | 50.0 | 50.0 |
| 8 | 0.125 | 0.0 | 3.3 | 0.0 | 3.3 | 90.0 | 96.7 | 100.0 | 100.0 | 70.0 | 56.7 | 30.0 | 33.3 | 20.0 |
| | 0.25 | 0.0 | 13.3 | 0.0 | 10.0 | 96.7 | 100.0 | 100.0 | 100.0 | 83.3 | 60.0 | 43.3 | 46.7 | 53.3 |
| | 0.5 | 0.3 | 3.3 | 0.0 | 0.0 | 95.0 | 100.0 | 100.0 | 100.0 | 90.0 | 73.3 | 76.7 | 83.3 | 78.3 |
| 13 | 0.125 | 0.0 | 0.0 | 0.0 | 0.0 | 0.0 | 60.0 | 100.0 | 96.7 | 23.3 | 16.7 | 23.3 | 20.0 | 40.0 |
| | 0.25 | 0.0 | 0.0 | 0.0 | 0.0 | 26.7 | 73.3 | 100.0 | 100.0 | 26.7 | 16.7 | 6.7 | 6.7 | 0.0 |
| | 0.5 | 0.0 | 0.0 | 0.0 | 0.0 | 60.0 | 81.7 | 100.0 | 100.0 | 63.3 | 36.7 | 26.7 | 10.0 | 33.3 |
| 17 | 0.25 | 0.0 | 0.0 | 0.0 | 0.0 | 100.0 | 100.0 | 100.0 | 100.0 | 56.7 | 36.7 | 63.3 | 60.0 | 50.0 |
| | 0.5 | 0.0 | 3.3 | 0.0 | 0.0 | 96.7 | 100.0 | 100.0 | 100.0 | 83.3 | 63.3 | 96.7 | 90.0 | 91.7 |
| Control | 0.0 | 0.0 | 0.0 | 0.0 | 0.0 | 0.0 | 0.0 | 0.0 | 0.0 | 0.0 | 0.0 | 0.0 | 0.0 | 0.0 |

Aquatic Herbicidal Method

A method of controlling the growth of unwanted aquatic vegetation is also provided which comprises contacting the vegetation to be controlled or the water in which the vegetation is growing with an aquatic herbicidally-effective amount of a compound of the formula

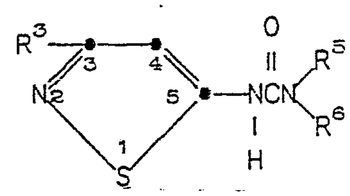

X

wherein

$R^3$ is $C_2$-$C_{10}$ alkyl, benzyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, or ($C_3$-$C_6$ cycloalkyl)$C_1$-$C_4$ alkyl;

$R^5$ and $R^6$ are $C_1$-$C_3$ alkyl, n-butyl, sec - butyl, $C_2$-$C_6$ alkenyl, or $C_1$-$C_2$ alkoxy;

provided that one of $R^5$ or $R^6$ is methyl;

or an aquatically-acceptable acid-addition salt thereof.

More preferred compounds of formula X within this method are those in which:

$R^3$ is $C_2$-$C_6$ alkyl or $C_3$-$C_6$ cycloalkyl; and

$R^5$ and $R^6$ are $C_1$-$C_3$ alkyl, n-butyl, secondary butyl, or $C_1$-$C_2$ alkoxy, provided that one of $R^5$ or $R^6$ is methyl.

Even more preferred compounds of formula X are where:

$R^3$ is $C_2$-$C_4$ alkyl; and

$R^5$ and $R^6$ are methyl or methoxy, provided that one of $R^5$ or $R^6$ is methyl.

Representative preferred compounds of formula X are:

N'-[3-(1-methylcyclohexyl)-5-isothiazolyl]-N,N-dimethylurea;

N'-(3-cyclohexyl-5-isothiazolyl)-N-methoxy-N-methylurea;

N'-[3-(1,1-dimethylethyl)-5-isothiazolyl]-N-methoxy-N-methylurea;

N'-[3-(1-methylpropyl)-5-isothiazolyl]-N-methyl-N-(1-methylethyl)urea;

N'-[3-(1-methylpropyl)-5-isothiazolyl]-N-methoxy-N-methylurea;

N'-[3-(1-ethyl-1-methylpropyl)-5-isothiazolyl]-N-ethyl-N-methylurea; and

N'-[3-(1-methylethyl)-5-isothiazolyl]-N-methoxy-N-methylurea.

The compounds of formula X for the aquatic herbicide tests were formulated in the following manner. Twenty milligrams of compound was weighed into a 12 ml disposable vial. To the vial containing the compound were added 1 ml of acetone and 9 ml of aqueous 0.1 percent polyoxyethylene sorbitan monooleate (Tween 80). This solution was then diluted with appropriate volumes of water to obtain solutions containing 10, 4, 2, 1, and 0.5 ppm (parts per million) of the test compound. The

herbicidal activity was determined by visual observations based upon non-treated controls. Activity ratings were made on a scale of 1 to 5 as follows:

1= no observable effect
2= slight plant injury
3= moderate plant injury
4= 50-90% plant injury
5 = plants completely killed

The results of this test are reported in Tables IX and X which follow.

Table IX

Aquatic Herbicide Activity 1-week post-treatment

| Compound of Example No. | Concentration ppm | Hydrilla | Coontail | Duckweed |
|---|---|---|---|---|
| 1 | 2 | 5 | 3 | 4 |
| | 4 | 5 | 3 | 5 |
| | 10 | 5 | 5 | 5 |
| 2 | 2 | 5 | 4 | 3 |
| | 4 | 5 | 4 | 4 |
| 3 | 2 | 4 | 2 | 4 |
| | 4 | 5 | 3 | 1 |
| 4 | 10 | 5 | 5 | 5 |
| 5 | 2 | 5 | 4 | 4 |
| | 4 | 5 | 4 | 4 |
| 6 | 2 | 5 | 5 | 4 |
| | 4 | 5 | 5 | 4 |
| | 10 | 5 | 5 | 5 |

Table IX cont'd.

| Compound of Example No. | Concentration ppm | Aquatic Herbicide Activity 1-week post-treatment: Hydrilla | Coontail | Duckweed |
|---|---|---|---|---|
| 7 | 2 | 5 | - | 2 |
| | 4 | 5 | - | 4 |
| 8 | 2 | 5 | - | 4 |
| | 4 | 5 | - | 4 |
| 9 | 2 | 3 | 1 | 1 |
| | 4 | 2 | 3 | 1 |
| 10 | 2 | 2 | 1 | 3 |
| | 2 | 5 | 4 | 2 |
| | 4 | 5 | 4 | 2 |
| | 4 | 5 | 5 | 4 |
| | 10 | 5 | 5 | 4 |
| | 10 | 5 | 5 | 5 |
| 14 | 2 | 5 | - | 1 |
| | 4 | 5 | - | 1 |
| 15 | 2 | 5 | - | 4 |
| | 4 | 5 | - | 4 |
| 16 | 2 | 5 | - | 4 |
| | 4 | 5 | - | 4 |
| | 10 | 5 | 5 | 5 |
| 17 | 2 | 5 | 3 | 3 |
| | 4 | 5 | 5 | 4 |
| 18 | 2 | 3 | 1 | 3 |
| | 4 | 5 | 4 | 4 |
| 20 | 2 | 5 | 3 | 4 |
| | 4 | 5 | 4 | 5 |
| | 10 | 5 | 4 | 4 |
| 22 | 2 | 3 | 1 | 4 |
| | 4 | 5 | 5 | 5 |

Table IX cont'd.

| Compound of Example No. | Concentration ppm | Hydrilla | Coontail | Duckweed |
|---|---|---|---|---|
| | | Aquatic Herbicide Activity 1-week post-treatment | | |
| 23 | 2 | 4 | 3 | 4 |
| | 4 | 5 | 3 | 4 |
| | 10 | 5 | 5 | 5 |
| 24 | 2 | 5 | 1 | 5 |
| | 4 | 5 | 5 | 5 |
| 26 | 2 | 5 | 4 | 4 |
| | 4 | 5 | 4 | 5 |
| | 10 | 5 | 5 | 5 |
| 28 | 2 | 4 | 4 | 4 |
| | 4 | 3 | 3 | 4 |
| 29 | 2 | 3 | 2 | 3 |
| | 4 | 5 | 3 | 5 |
| | 10 | 5 | 3 | 4 |
| 30 | 2 | 3 | 3 | 4 |
| | 4 | 3 | 4 | 4 |
| 31 | 2 | 4 | 3 | 5 |
| | 4 | 5 | 5 | 4 |
| 34 | 2 | 5 | 5 | 4 |
| | 4 | 5 | 5 | 5 |
| | 10 | 5 | 5 | 5 |
| 35 | 2 | 5 | 2 | 4 |
| | 4 | 5 | 3 | 2 |
| | 10 | 5 | 5 | 4 |
| 36 | 2 | 2 | 3 | 2 |
| | 4 | 5 | 4 | 2 |
| | 10 | 5 | 2 | 1 |

Table IX cont'd.

Aquatic Herbicide Activity 1-week post-treatment

| Compound of Example No. | Concentration ppm | Hydrilla | Coontail | Duckweed |
|---|---|---|---|---|
| 37 | 2 | 5 | 2 | 4 |
| | 4 | 5 | 5 | 5 |
| | 10 | 5 | 4 | 4 |
| 38 | 2 | 5 | 4 | 4 |
| | 4 | 5 | 4 | 4 |
| | 10 | 5 | 5 | 5 |
| 39 | 2 | 5 | 4 | 5 |
| | 4 | 5 | 5 | 5 |
| | 10 | 5 | 5 | 5 |
| 40 | 2 | 5 | - | 4 |
| | 4 | 5 | - | 5 |
| 41 | 2 | 2 | 3 | 5 |
| | 4 | 5 | 3 | 5 |
| | 10 | 5 | 4 | 5 |
| 42 | 2 | 5 | 3 | 2 |
| | 4 | 5 | 3 | 3 |
| 44 | 2 | 5 | 5 | 4 |
| | 4 | 5 | 1 | 3 |
| 55 | 2 | 5 | 5 | 4 |
| | 4 | 5 | 4 | 4 |

Table X

Aquatic Herbicide Activity

| Compound of Example No. | Concentration ppm | 1-week post treatment: Hydrilla | Coontail | Duckweed | Naiad | Milfoil | Cabomba | Sago Pond Weed | 2-week post treatment: Hydrilla | Coontail | Duckweed | Naiad | Milfoil | Cabomba | Sago Pond Weed | 3-week post treatment: Hydrilla | Coontail | Duckweed | Naiad | Milfoil | Cabomba | Sago pond weed |
|---|---|---|---|---|---|---|---|---|---|---|---|---|---|---|---|---|---|---|---|---|---|---|
| 2 | 0.5 | 4 | 2 | 1 | 1 | 2 | 2 | 1 | 5 | 2 | 1 | 3 | 3 | 3 | 1 | 5 | 2 | 2 | 5 | 5 | 4 | 2 |
| | 1 | 2 | 2 | 1 | 1 | 2 | 1 | 1 | 3 | 2 | 2 | 4 | 4 | 3 | 1 | 5 | 2 | 2 | 5 | 5 | 4 | 1 |
| | 2 | 4 | 2 | 1 | 4 | 3 | 2 | 4 | 5 | 5 | 3 | 5 | 5 | 4 | 4 | 5 | 5 | 3 | 5 | 5 | 5 | 5 |
| 4 | 0.5 | | | | | | | | 1 | 2 | 2 | 4 | 5 | 4 | 1 | 1 | 2 | 2 | 5 | 5 | 5 | 4 |
| | 1 | | | | | | | | 5 | 2 | 2 | 4 | 5 | 4 | 1 | 5 | 3 | 3 | 5 | 5 | 5 | 1 |
| | 2 | | | | | | | | 5 | 4 | 4 | 5 | 5 | 5 | 4 | 5 | 4 | 4 | 5 | 5 | 5 | 3 |
| 5 | 0.5 | 2 | 2 | 1 | 1 | - | 1 | 1 | 1 | 3 | 1 | 1 | - | 1 | 1 | 2 | 3 | 2 | 1 | - | 1 | 1 |
| | 1 | 2 | 1 | 1 | 3 | - | 2 | 4 | 3 | 2 | 1 | 4 | - | 2 | 4 | 5 | 2 | 2 | 5 | - | 5 | 4 |
| | 2 | 1 | 2 | 2 | 3 | - | 3 | 2 | 5 | 5 | 3 | 5 | - | 5 | 4 | 5 | 5 | 3 | 5 | - | 5 | 3 |
| 7 | 0.5 | 1 | 1 | 1 | 1 | 2 | 1 | 1 | 2 | 1 | 1 | 2 | 2 | 2 | 1 | 3 | 1 | 1 | 1 | 2 | 3 | 1 |
| | 1 | 1 | 2 | 1 | 1 | 2 | 1 | 2 | 3 | 3 | 2 | 3 | 5 | 4 | 4 | 4 | 1 | 2 | 5 | 5 | 5 | 4 |
| | 2 | 2 | 1 | 2 | 2 | 4 | 2 | 2 | 5 | 3 | 4 | 5 | 5 | 4 | 3 | 5 | 2 | 2 | 5 | 5 | 5 | 5 |
| 8 | 0.5 | 1 | 1 | 3 | - | 3 | 1 | 2 | 1 | 1 | 4 | - | 3 | 1 | 3 | 1 | 3 | 4 | - | 3 | 3 | 3 |
| | 1 | 1 | 1 | 3 | - | 3 | 1 | 3 | 5 | 1 | 4 | - | 4 | 1 | 4 | 5 | 4 | 5 | - | 4 | 5 | 5 |
| | 2 | 1 | 1 | 3 | - | 3 | 1 | 1 | 3 | 1 | 4 | - | 4 | 3 | 3 | 5 | 4 | 5 | - | 5 | 5 | 5 |
| 17 | 0.5 | 1 | 3 | 1 | 1 | 1 | 1 | 1 | 1 | 4 | 1 | 1 | 1 | 3 | 1 | 2 | 4 | 2 | 2 | 4 | 4 | 1 |
| | 1 | 1 | 2 | 2 | 1 | 1 | 1 | 1 | 1 | 2 | 2 | 2 | 1 | 3 | 2 | 2 | 4 | 4 | 2 | 4 | 4 | 1 |
| | 2 | 2 | 1 | 2 | 2 | 2 | 1 | 1 | 2 | 2 | 4 | 4 | 4 | 3 | 2 | 4 | 2 | 4 | 5 | 5 | 4 | 4 |

Table X cont'd.

Aquatic Herbicide Activity

| Compound of Example No. | Concentration ppm | 1-week post treatment: Hydrilla | Coontail | Duckweed | Naiad | Milfoil | Cabomba | Sago Pond Weed | 2-week post treatment: Hydrilla | Coontail | Duckweed | Naiad | Milfoil | Cabomba | Sago Pond Weed | 3-week post treatment: Hydrilla | Coontail | Duckweed | Naiad | Milfoil | Cabomba | Sago pond weed |
|---|---|---|---|---|---|---|---|---|---|---|---|---|---|---|---|---|---|---|---|---|---|---|
| 22 | 0.5 | 1 | 1 | 1 | 1 | 1 | 1 | 1 | 2 | 1 | 1 | 1 | 2 | 2 | 2 | 3 | 1 | 2 | 3 | 4 | 3 | 2 |
| | 1 | 1 | 1 | 2 | 1 | 1 | 1 | 1 | 5 | 1 | 3 | 5 | 4 | 3 | 2 | 5 | 3 | 3 | 5 | 5 | 5 | 3 |
| | 2 | 1 | 1 | 2 | 1 | 1 | 2 | 1 | 5 | 3 | 4 | 5 | 5 | 5 | 4 | 5 | 4 | 4 | 5 | 5 | 5 | 5 |
| 24 | 0.5 | 1 | 1 | 1 | 1 | 2 | 1 | 5 | 3 | 1 | 2 | 3 | 3 | 3 | 5 | 4 | 2 | 3 | 5 | 5 | 5 | 5 |
| | 1 | 5 | 1 | 1 | 4 | 2 | 3 | 5 | 5 | 3 | 3 | 5 | 5 | 5 | 5 | 5 | 5 | 4 | 5 | 5 | 5 | 5 |
| | 2 | 4 | 3 | 2 | 4 | 3 | 2 | 4 | 5 | 3 | 3 | 5 | 5 | 5 | 5 | 5 | 4 | 4 | 5 | 5 | 5 | 5 |
| 28 | 0.5 | 1 | 1 | 1 | 4 | 1 | 1 | 1 | 1 | 1 | 1 | 4 | 3 | 1 | 3 | 1 | 3 | 1 | 5 | 3 | 3 | 1 |
| | 1 | 1 | 1 | 1 | 1 | 1 | 1 | 1 | 1 | 2 | 3 | 1 | 5 | 1 | 3 | 3 | 3 | 2 | 1 | 5 | 3 | 5 |
| | 2 | 3 | 3 | 2 | 1 | 2 | 3 | 1 | 5 | 3 | 4 | 5 | 5 | 5 | 3 | 5 | 5 | 4 | 5 | 5 | 5 | 3 |
| 31 | 0.5 | 1 | 1 | 1 | 1 | 1 | 1 | 1 | 2 | 1 | 1 | 1 | 2 | 2 | 2 | 3 | 2 | 1 | 5 | 4 | 3 | 3 |
| | 1 | 2 | 1 | 1 | 1 | 2 | 2 | 5 | 3 | 3 | 1 | 1 | 2 | 1 | 5 | 4 | 1 | 2 | 1 | 3 | 3 | 5 |
| | 2 | 3 | 2 | 1 | 1 | 3 | 2 | 3 | 4 | 3 | 2 | 5 | 4 | 5 | 3 | 5 | 5 | 3 | 5 | 5 | 5 | 3 |
| 35 | 0.5 | 1 | 1 | 2 | 1 | 2 | 1 | 1 | 1 | 2 | 2 | 4 | 3 | 1 | 1 | 1 | 2 | 2 | 5 | 4 | 5 | 1 |
| | 1 | 1 | 1 | 2 | 2 | 2 | 1 | 1 | 3 | 1 | 2 | 5 | 4 | 2 | 1 | 4 | 2 | 3 | 5 | 5 | 5 | 3 |
| | 2 | 3 | 1 | 2 | 2 | 3 | 2 | 2 | 5 | 3 | 3 | 5 | 5 | 5 | 3 | 5 | 3 | 3 | 5 | 5 | 5 | 3 |
| 39 | 0.5 | | | | | | | | 1 | 2 | 2 | 2 | 3 | 2 | 1 | 1 | 3 | 3 | 4 | 5 | 5 | 2 |
| | 1 | | | | | | | | 2 | 3 | 3 | 5 | 5 | 4 | 1 | 5 | 5 | 4 | 5 | 5 | 5 | 1 |
| | 2 | | | | | | | | 5 | 2 | 4 | 5 | 4 | 5 | 1 | 5 | 5 | 5 | 5 | 5 | 5 | 3 |

Table X cont'd.

Aquatic Herbicide Activity

| Compound of Example No. | Concentration ppm | 1-week post treatment: Hydrilla | Coontail | Duckweed | Naiad | Milfoil | Cabomba | Sago Pond Weed | 2-week post treatment: Hydrilla | Coontail | Duckweed | Naiad | Milfoil | Cabomba | Sago Pond Weed | 3-week post treatment: Hydrilla | Coontail | Duckweed | Naiad | Milfoil | Cabomba | Sago pond weed |
|---|---|---|---|---|---|---|---|---|---|---|---|---|---|---|---|---|---|---|---|---|---|---|
| 47 | 0.5 | 1 | 1 | 1 | 1 | 1 | 1 | 1 | 2 | 2 | 1 | 3 | 3 | 2 | 1 | 3 | 2 | 1 | 1 | 4 | 3 | 3 |
| | 1 | 1 | 1 | 1 | 1 | 1 | 1 | 1 | 3 | 2 | 1 | 4 | 4 | 3 | 2 | 3 | 2 | 1 | 3 | 5 | 4 | 3 |
| | 2 | 1 | 1 | 1 | 3 | 1 | 1 | 1 | 3 | 2 | 2 | 5 | 4 | 3 | 2 | 3 | 3 | 2 | 5 | 5 | 4 | 3 |
| 55 | 0.5 | 1 | 1 | 1 | 1 | 2 | 2 | 2 | 2 | 1 | 2 | 1 | 4 | 2 | 3 | 3 | 3 | 2 | 2 | 5 | 3 | 4 |
| | 1 | 2 | 2 | 1 | 3 | 3 | 1 | 2 | 4 | 2 | 2 | 4 | 5 | 1 | 3 | 4 | 3 | 3 | 4 | 5 | 3 | 4 |
| | 2 | 4 | 2 | 2 | 4 | 2 | 1 | 2 | 5 | 3 | 3 | 5 | 5 | 4 | 5 | 5 | 4 | 4 | 5 | 5 | 5 | 5 |

Various compounds of formula X were tested as aquatic herbicides under field conditions in artificial ponds maintained outdoors. The artificial ponds were plastic-lined cylindrical vessels approximately 1 m in diameter and 0.5 m deep and contained a layer of earth at the bottom. The vessels were filled with water, and then the weeds or plants were added and allowed to become rooted before treatment.

The compounds of formula X were formulated as wettable powders (50W) described in the Formulations section hereinafter, and diluted with appropriate volumes of water to obtain solutions containing 0.1 to 1.0 ppm of the active compound of formula X. The activity was determined by visual observation based upon non-treated controls. Plant injury ratings were made visually on a scale of 0-10 with 0 being no injury and 10 being plant death. The injury rating was multiplied by 10 to obtain a percent inhibition. The results of these tests are reported in Table XI.

TABLE XI

PERCENT INHIBITION--DAYS AFTER TREATMENT

| Compound of Example No. | Concentration ppm | Hydrilla | | | Watermilfoil | | | Naiad | | |
|---|---|---|---|---|---|---|---|---|---|---|
| | | 21 | 35 | 63 | 21 | 35 | 63 | 21 | 35 | 63 |
| 5 | 0.1 | 30.0 | 50.0 | 0.0 | 95.0 | 99.5 | 100.0 | 75.0 | 94.0 | 90.0 |
| | 0.3 | 80.0 | 94.5 | 99.0 | 95.0 | 99.5 | 100.0 | 100.0 | 100.0 | 100.0 |
| | 1.0 | 90.0 | 99.5 | 100.0 | 90.0 | 99.5 | 100.0 | 100.0 | 100.0 | 100.0 |
| 8 | 0.1 | 0.0 | 0.0 | 0.0 | 0.0 | 0.0 | 0.0 | 0.0 | 0.0 | 0.0 |
| | 0.3 | 0.0 | 10.0 | 10.0 | 55.0 | 84.5 | 90.0 | 45.0 | 50.0 | 50.0 |
| | 1.0 | 70.0 | 79.5 | 89.0 | 90.0 | 94.5 | 100.0 | 100.0 | 100.0 | 100.0 |
| 15 | 0.1 | 10.0 | 0.0 | 0.0 | 70.0 | 80.0 | 0.0 | 85.0 | 50.0 | 0.0 |
| | 0.3 | 80.0 | 94.5 | 95.0 | 90.0 | 100.0 | 100.0 | 100.0 | 100.0 | 100.0 |
| | 1.0 | 90.0 | 99.5 | 100.0 | 95.0 | 100.0 | 100.0 | 100.0 | 100.0 | 100.0 |
| 17 | 0.1 | 0.0 | 0.0 | 0.0 | 0.0 | 0.0 | 0.0 | 0.0 | 0.0 | 0.0 |
| | 0.3 | 0.0 | 0.0 | 0.0 | 25.0 | 0.0 | 15.0 | 50.0 | 0.0 | 0.0 |
| | 1.0 | 85.0 | 89.5 | 94.0 | 95.0 | 99.5 | 100.0 | 100.0 | 100.0 | 100.0 |
| 24 | 0.1 | 0.0 | 0.0 | 0.0 | 0.0 | 0.0 | 0.0 | 0.0 | 0.0 | 0.0 |
| | 0.3 | 0.0 | 10.0 | 0.0 | 70.0 | 85.0 | 94.0 | 0.0 | 0.0 | 0.0 |
| | 1.0 | 85.0 | 97.0 | 99.5 | 90.0 | 99.5 | 100.0 | 95.0 | 100.0 | 100.0 |
| Control | 0 | -- | 0.0 | 0.0 | -- | 0.0 | 0.0 | -- | 0.0 | 0.0 |

Formulations

Also provided is a formulation comprising a compound of the formula IX, VII, or X together with an agriculturally- or aquatically acceptable carrier.

The aquatic herbicidal method and the algicidal method are practiced by adding the active isothiazolylureas of formula VII or X to the water containing the submerged, emergent, ditchbank, or floating aquatic plants or algae or otherwise contacting the plants with the active compounds, for example, by applying the compounds to the sub-aqueous soil in which the aquatic plants are rooted. The formulations suitably contain from about 5 to about 90 percent by weight of the active ingredient. The compounds may also be mixed with surface-active dispersing agents to form concentrates to facilitate dispersion in water and to improve the wetting properties when used as sprays. If desired, the compounds may be mixed with a powdered solid carrier, such as bentonite, Fuller's earth, diatomaceous earth, or various mineral silicates, and clays together with surface-active wetting and dispersing agents, so that a wettable powder may be obtained which may be applied directly, or which may be mixed with water to make an aqueous dispersion for application in that form. These wettable powder formulations suitably contain from about 25 to about 85 percent by weight of the active ingredient. The compounds may be dissolved in an oil, such as a hydrocarbon or chlorinated hydrocarbon oil, and the

oil solution of the compound dispersed in water with the aid of a surface-active emulsifying agent to give a sprayable aqueous emulsion. These surface-active emulsifying agents may be anionic, nonionic, or cationic surface-active agents. Such surface-active agents are well-known, and reference is made to Hoffman et al., U.S. Patent No. 2,614,916, columns 2-4, for detailed examples of the same.

Further, the compounds can also be applied in an invert emulsion formulation. An invert emulsion formulation is prepared by first making a solution of the compound in heavy oils, such as diesel fuel, inverting oil, and the like, and combining the thus-obtained solution with water under high shear stirring. Densifying agents, such as powered limestone or iron oxide may be needed to increase the density of the invert. The thick emulsion is placed in the water and sinks to the bottom of the lake, river, pond, or the like, and the aquatic herbicide or algicide is gradually released. The following is an example of an invert emulsion formulation.

Invert Emulsion

| Ingredient | Amount |
|---|---|
| Compound of Example No. 17 | 12.5 g |
| Diesel fuel | 333 ml |
| Inverting oil* | 333 ml |

*Visko-Rhap Inverting Oil (Rhodia, Inc.)

Two-hundred fifty milliliters of this solution is combined with 3750 ml of water under high shear stirring to give a thick invert emulsion.

The compounds can also be applied as pellets, which are prepared from a mixture of about 5% of the active ingredient, about 87% balling clay, and about 10% water, all percentages being by weight. The mixture is then extruded through a pellet mill using a suitably sized die, e.g., about 1/8 in. diameter. The extruded pellets are about 1/8 in. by 1 1/2 in., and are then dried to about 8% moisture content.

The method of controlling aquatic weeds or algae is practiced by adding to the water containing the submerged, emergent, ditchbank, or floating plants or algae, a herbicidal or algicidal amount of one of the herein-disclosed compounds of formula VII or X such that a concentration of from about 0.01 to about 10 ppm of the active compound is attained.

The optimum concentration of active compound of formula VII or X for any specific aquatic control problem varies with the temperature, the species to be controlled and the shape of the body of water to be treated. At higher water temperatures, less compound is generally required for a given degree of control than is needed at lower temperatures. When used to control algae or aquatic weeds, the compounds will usually be employed at concentrations of about 0.1 to about 10 ppm. In terms of pounds of compound per acre of water one foot deep 0.1 to 10 ppm is equal to about 0.3 to about 30 pounds per acre of water one foot deep.

In considering the treatment of moving streams for the purpose of controlling vegetation fixed therein, special account must be taken of the fact that the compounds will pass over the area to be treated and that the concentration during the contact period is dependent upon the water flow rate, the rate of chemical addition, and the time period of addition.

The isothiazolylurea herbicides of formula IX to be employed according to the terrestrial method can be formulated for convenient application as aqueous sprays, solid pellets or granules, and the like. Herbicidal formulations containing an isothiazolylurea of formula IX will contain from about 1.0 to about 90 percent by weight of active isothiazolylurea, and can be prepared by combining the isothiazolylurea with any number of common herbicidal carriers, diluents, adjuvants, and the like. The formulations can contain liquid carriers and adjuvants such as organic solvents, including benzene, xylene, toluene, a halotoluene such as _ortho_-chlorotoluene, n-hexane, n-pentane, kerosene, and similar organic solvents. The formulations can additionally contain art-known emulsifiers, stabilizers, dispersants, suspending agents, spreaders, penetrants, wetting agents, and the like.

Typical carriers utilized in the dry formulations include clay, diatomaceous earth, silica, pyrophyllite, and the like. Herbicidal powders are prepared by grinding and blending such solid carriers with a compound of formula IX. Granular formulations provided herein are prepared by impregnating an isothiazolylurea onto or into a granulated carrier such as a vermiculite.

Such granules typically have a particle size of about 0.2 to about 2.0 mm.

Preferred formulations are in the form of aqueous suspension concentrates (flowables). Such formulations employ any number of commercially available wetting agents, and dispersing agents, such as ethoxylated nonyl phenols, ethoxylated octyl phenols, polyoxyethylene stearyl ethers, lignin sulfonates, blends of such agents, and the like. Such formulations can optionally contain adjuvants: such as thickening agents, for instance heteropolysaccharide gums and the like; such as xanthan gum; as well as antibacterial agents, such as aqueous formaldehyde.

The following detailed examples of such formulations are given as illustrations.

Granule

| Ingredient | Percent by weight |
|---|---|
| Compound of Example 17 | 5.0 |
| Clay Granule | 95.0 |

The compound is substantially dissolved in acetone or similar solvent, and the organic solution is sprayed onto the clay, which is in the form of chips. The mixture is then thoroughly blended and dried.

Wettable Powder (75 W)

| Ingredient | Percent by Weight |
|---|---|
| Compound of Example 17 | 75.0 |
| Fuller's earth | 19.0 |
| Sulfonated lignin | 3.5 |
| Sodium lauryl sulfate | 2.5 |

Wettable Powder (50 W)

| Ingredient | Percent by Weight |
|---|---|
| Compound of Example 8 | 51.5 |
| Wetting agent | 5.0 |
| Dispersing agent | 5.0 |
| Anticaking agent | 5.0 |
| Barden clay | 33.5 |

Wettable Powder (50 W)

| Ingredient | Percent by Weight |
|---|---|
| Compound of Example 17 | 51.5 |
| Wetting and dispersing agent | 8.0 |
| Anticaking agent | 3.0 |
| Barden clay | 37.5 |

Examples of the various classes of ingredients are: wetting agent--naphthalene sulfonate (Sellogen HR by Westvaco Chemical); dispersing agent--sulfonated lignin (Polyfon O by Westvaco Chemical); anticaking agent--$SiO_2 \cdot H_2O$--(Zeolex 7 by Hubler Chemical); wetting and dispersing agent--(Reax 45L by Westvaco Chemical); carrier--(Barden clay by Hubler Chemical). Other

suitable wetting agents, dispersing agents, anticaking agents or carriers discussed above can also be employed.

The above ingredients for each formulation are blended to uniformity and are ground in a hammer mill or air mill. The product is then reblended to a homogenous free flowing powder. The powder is dispersed in water and sprayed onto the weed-infested area.

## CLAIMS

1. A urea compound of the formula

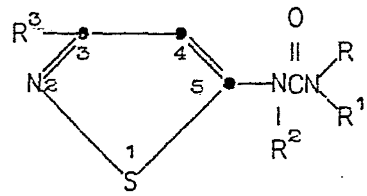

IX

wherein

R and $R^1$ are hydrogen, $C_1$-$C_3$ alkyl, n-butyl, sec-butyl, $C_2$-$C_6$ alkenyl, or $C_1$-$C_2$ alkoxy;

provided that only one of R or $R^1$ may be hydrogen;

provided that only one of R or $R^1$ may be $C_1$-$C_2$ alkoxy;

provided that at least one of R or $R^1$ is less than three carbon atoms; or

R and $R^1$ together with the nitrogen atom form a pyrrolidyl, piperidyl, morpholinyl, or piperazinyl ring;

$R^2$ is hydrogen or $C_1$-$C_2$ alkyl; and

$R^3$ is $C_2$-$C_{10}$ alkyl, benzyl, $C_3$-$C_6$ cycloalkyl, ($C_1$-$C_4$ alkyl)$C_3$-$C_6$ cycloalkyl, ($C_2$-$C_4$ alkenyl)$C_3$-$C_6$ cycloalkyl, ($C_1$-$C_4$ haloalkyl)$C_3$-$C_6$ cycloalkyl, or ($C_3$-$C_6$ cycloalkyl)$C_1$-$C_4$ alkyl;

or an agriculturally- or aquatically-acceptable acid-addition salt thereof.

2. A compound of claim 1 wherein

R and $R^1$ are $C_1$-$C_3$ alkyl or $C_1$-$C_2$ alkoxy;

$R^2$ is hydrogen; and

$R^3$ is $C_3$-$C_6$ alkyl.

3. A compound of claim 2 wherein

R is methyl; $R^1$ is methoxy or methyl; and

$R^3$ is $C_3$-$C_4$ alkyl.

4. Any one of the following compounds or an acid-addition salt thereof:

N'-(3-cyclohexyl-5-isothiazolyl)-N,N-dimethylurea

N'-(3-cyclohexyl-5-isothiazolyl)-N-methoxy-N-methylurea

N'-[3-(1-methylethyl)-5-isothiazolyl]-N,N-dimethylurea

N'-[3-(1-methylethyl)-5-isothiazolyl]-N-methoxy-N-methylurea

N'-(3-cyclohexylmethyl-5-isothiazolyl)-N,N-dimethylurea

N'-[3-(1,1-dimethylethyl)-5-isothiazolyl]-N-methoxy-N-methylurea

N'-[3-(1-methylpropyl)-5-isothiazolyl]-N-methoxy-N-methylurea

N'-[3-(2-methylpropyl)-5-isothiazolyl]-N-methoxy-N-methylurea

N'-[3-(1,1-dimethylethyl)-5-isothiazolyl]-N,N-dimethylurea.

5. A process for preparing a urea compound of formula IX as defined in claim 1, which comprises reacting a 5-aminothiazole of the formula

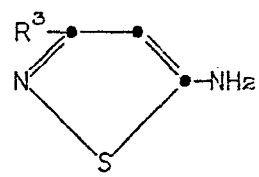

V

wherein $R^3$ is defined as in claim 1, with a ureido forming reagent, to provide the compounds of formula IX wherein $R^2$ is hydrogen;

followed by alkylation with

$R^{2'}Y$

wherein $R^{2'}$ is $C_1$-$C_2$ alkyl and Y is bromo, chloro, fluoro, or iodo, to provide the compounds of formula IX wherein $R^2$ is $C_1$-$C_2$ alkyl; and

optionally followed by salification to form the corresponding acid-addition salt.

6. The use of a urea derivative of the formula

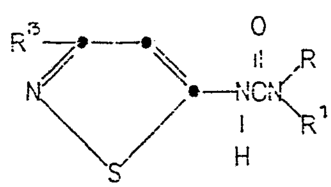

VII

or an acid-addition salt thereof, wherein R, $R^1$ and $R^3$ are defined as in claim 1, as an aquatic algicide

7. The use of a urea derivative of formula IX, or an acid-addition salt thereof, as claimed in any one of claims 1 to 4 as a terrestrial herbicide

8. The use of a urea derivative of the formula

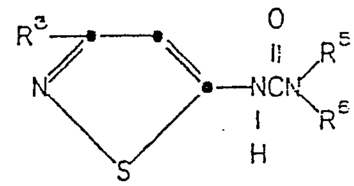

X

or an acid-addition salt thereof, wherein $R^3$ is defined as in claim 1, $R^5$ and $R^6$ are $C_1$-$C_3$ alkyl, n-butyl, sec-butyl, $C_2$-$C_6$ alkenyl, or $C_1$-$C_2$ alkoxy; provided that one of $R^5$ or $R^6$ is methyl, as an aquatic herbicide.

9. An aquatic algicide formulation comprising as an active ingredient a compound of formula VII, as defined in claim 6, or an acid-addition salt thereof, associated with one or more aquatically-acceptable carriers or diluents therefor.

10. A herbicidal formulation comprising as an active ingredient a compound of formula IX, or an acid-addition salt thereof, as claimed in any one of claims 1 to 4, associated with one or more herbicidally-acceptable carriers or diluents therefor.